# EUROPEAN PATENT SPECIFICATION

(11) **EP 4 007 778 B1**
(45) Date of publication and mention of the grant of the patent: **25.11.2026**
(21) Application number: 20754675.5
(22) Date of filing: 31.07.2020
(51) Int. Cl.: C07K 16/28, C07K 16/30, C07K 16/46, A61K 39/00

(54) **MODIFIED BI SPECIFIC ANTI CD3 ANTIBODIES**
MODIFIZIERTE BISPEZIFISCHE ANTI CD3-ANTIKÖRPER
ANTICORPS ANTI-CD3 BISPÉCIFIQUES MODIFIÉS

(30) Priority: 02.08.2019 DE 102019121022; 02.08.2019 US 201962882364 P
(43) Date of publication of application: 08.06.2022
(73) Proprietor: Immatics Biotechnologies GmbH, 72076 Tübingen (DE)
(72) Inventor: PSZOLLA, Gabriele, 72076 Tübingen (DE); HOFMANN, Martin, 72076 Tübingen (DE); UNVERDORBEN, Felix, 70199 Stuttgart (DE); HUTT, Meike, 70565 Stuttgart (DE); MAURER, Dominik, 72116 Mössingen (DE); BUNK, Sebastian, 72074 Tübingen (DE)
(74) Representative: Forresters IP LLP
(86) International application number: PCT/EP2020/071655
(87) International publication number: WO 2021/023657

(56) References cited:
- EP-A1- 3 428 194
- MICHAEL DURBEN ET AL: "Characterization of a Bispecific FLT3 X CD3 Antibody in an Improved, Recombinant Format for the Treatment of Leukemia", MOLECULAR THERAPY, vol. 23, no. 4, April 2015 (2015-04-01), US, pages 648 - 655, XP055554970, ISSN: 1525-0016, DOI: 10.1038/mt.2015.2
- Z. WU ET AL: "T cell engaging bispecific antibody (T-BsAb): From technology to therapeutics", PHARMACOLOGY AND THERAPEUTICS., vol. 182, February 2018 (2018-02-01), GB, pages 161 - 175, XP055636083, ISSN: 0163-7258, DOI: 10.1016/j.pharmthera.2017.08.005

## Description

The present disclosure concerns bispecific antigen binding proteins directed against a major histocompatibility (MHC) presented target antigens (TA). The disclosure in particular concerns bispecific antigen binding proteins comprising at least two antigen binding sites (A and B), wherein the antigen binding site A binds to CD3 and the antigen binding site B binds to a target antigenic (TA) peptide/MHC complex. The bispecific antigen binding proteins comprise, in particular, the complementary determining regions (CDRs) of the VL and VH domains of novel engineered anti-CD3 antibodies having a reduced affinity. The bispecific antigen binding proteins are of use for the diagnosis, treatment and prevention of TA associated diseases, such as tumor-associated antigen (TAA) expressing cancerous diseases. Further provided are nucleic acids encoding the bispecific antigen binding proteins, vectors comprising these nucleic acids, recombinant cells expressing the antigen binding proteins and pharmaceutical compositions comprising the bispecific antigen binding proteins.

### Background of the Invention

T cells recognize (virus-) infected cells and tumor cells by detecting the presence of disease-specific peptides that are presented on the cells surface by the major histocompatibility (MHC) complex with their clone-specific T cell receptor (TCR). TCR based molecules are therefore of high interest for the development of disease or tumor specific immunotherapeutics (see e.g. the bispecific format of EP 3 428 194 A1). While advances have been made in the development of molecular-targeting drugs for cancer therapy, there remains a need in the art to develop new anti-cancer agents that specifically target molecules highly specific to cancer cells but not to normal cells. In analogy, target molecules highly specific to the diseased cells but not to normal cells are also of high importance for the development of drugs targeting infectious diseases such as HIV.

In the context of the present invention, the proteins from which the target antigenic (TA) peptides are derived are degraded by the proteasome into short peptides, transported into the endoplasmic reticulum, packaged in the groove of newly synthesized MHC molecules, and delivered as peptide-MHC (pMHC) complexes to the cell membrane (TA peptide/MHC). The recognition pattern induced by the TA allows the immune system to distinguish the diseased cell, such as transformed neoplastic cells in case of a TAA antigenic peptide, from surrounding normal tissue cells and triggers the immune cascade against them.

For the development of such TA-targeting drugs, TCRs specifically targeting TAs have been identified and the V_{α} and v_{β} domains were used for engineering new TA targeting molecules, in particular TAA targeting molecules.

With regards to TAA targeting molecules it should be noted, that native T cell receptors (TCRs) specifically binding to MHC presented cancer antigens are often of lower affinity (K*_{D}* = 1-300 µM) when compared to TCRs specifically binding to MHC presented viral antigens. Part of the explanation for this phenomenon seems to be that T cells that develop in the thymus are negatively selected (tolerance induction) on self-peptide-MHC ligands, such that T cells with too high affinity to such self-peptide MHCs are deleted. This low affinity may be one possible explanation for tumor immune escape (Aleksic et al. 2012, Eur J Immunol. 2012 Dec;42(12):3174-9). Therefore, it appears desirable to design TCR variants that bind with higher affinity to cancer antigens for use as antigen recognizing constructs in an adoptive cell therapy (ACT), or as recognition module of a soluble approach, i.e. using bispecific molecules (Hickman et al. 2016, J Biomol Screen. 2016 Sep;21(8):769-85).

However, simply increasing the affinity of TCRs may also increase the risk of side effects. As mentioned above, in nature high affinity TCRs directed against tumor-associated antigens, which are self-proteins, are precluded by thymic selection, to avoid recognition of self-peptides present on normal tissue through cross-reactivity. Accordingly, simply increasing the TCRs affinity for its target sequence might also increase the affinity to similar non cancer-specific peptides and therefore increase the risk of cross-reactivity and unwanted cytotoxic effects on normal tissue. This is not just a theoretic risk, since that has been painfully discovered for engineered TCRs targeting melanoma associated antigen A3 (MAGE-A3). In particular, previously published results have shown lethal toxicities in two patients, who were infused with T cells engineered to express a TCR targeting MAGE-A3 cross-reacting with a peptide from the muscle protein Titin, even though no cross-reactivities had been predicted in the pre-clinical studies (Linette GP et al. Blood 2013;122:863-71, Cameron BJ, et al. Sci. Transl. Med. 2013; 5: 197-103). These patients demonstrated that TCR-engineered T cells can have serious and unpredictable off-target and organ-specific toxicities.

Therefore, despite the advancements in TCR technology, there remains a need for additional therapeutics, in particular cancer therapeutics, particularly those that efficiently target and kill diseased cells, in particular cancer cells, while a high safety profile is maintained.

As indicated herein above, native TCRs have often of a quite low affinity for their target TAA/MHC complex, the low affinity avoids recognition of self-peptides present on normal tissue through cross-reactivity. However, an increased affinity for their target TAA/MHC complex is preferable to create efficient anti-cancer drugs.

To address this problematic, the inventors of the present invention combine in one molecule, affinity maturated TCR variable domains that bind a TA/MHC of interest with variable light and heavy chain domains targeting CD3 with a lower affinity than prior art anti-CD3 heavy and light chain variable domains. The resulting molecules have the advantage that they recognize diseased cells, such as cancer cells, even if the TA, such as the TAA, is presented on the cell surface only in small amounts while a high safety profile is maintained. In particular, thanks to the quite low affinity of the CD3 binding domain, the resulting molecule mimics a natural T cell/molecule (TCR)/TA relationship, because, in case of the bispecific antigen binding proteins of the invention, the low affinity binding occurs at the interface between the T cell and the CD3 binding domain of the bispecific antigen binding protein, instead of the interface between the TCR and the TA/MHC complex as it is the case in a native TCR expressing T cell that binds to a TA/MHC.

One of the technical advantageous effects of the use of low affinity CD3 binding domains is thus, that the resulting bispecific antigen binding proteins are specific to a TA of interest due to the use of high affinity TCR variable domains, while having a high safety profile, i.e. a safety window according to which around more than 1000-fold the dose used to treat a TA presenting cell, such as a cancer cell, would be necessary in order to kill cells of normal or healthy tissues, such as cells of normal tissues expressing off-target peptides. The combination of a high affinity antigen binding protein and a low affinity CD3 binding domain thus, leads to specific binding to the target peptide with reduced or no cross-recognition of off-target peptides on healthy tissues, for example to off target peptides and, thus, provides a surpisingly large safety window.

Accordingly, bispecific antigen binding proteins of the invention that combine low affinity CD3 binding domains with affinity maturated TA/MHC binding domains have the advantage that the resulting bispecific antigen binding proteins efficiently target the diseased cell but not healthy cells and also have a beneficial safety profile or even an improved safety profile. Advantageously, the resulting bispecific antigen molecules furthermore have an increased stability, and/or an increased solubility as compared to bispecific molecules using anti-CD3 domains already known in the art, providing promising bispecific molecules suitable for a medical use.

In summary, the described CD3 binding domains if used in conjunction, e.g. in a bispecific format, with TCRs or MHC-peptide complex binding fragments thereof or antigen binding proteins provide *inter alia* the following advantages over the art: (i) reduction of cross-reactivity of given TCRs or antigen binding proteins with similar peptides on healthy tissues while maintaining high tumor selectivity and/or specificity (ii) increased safety profile of TCRs or MHC-peptide complex binding fragments thereof, or antigen binding proteins,; (iii) reduction of off target and off-tumor cytotoxicity of TCRs or MHC-peptide complex binding fragments thereof, or antigen binding proteins; and (iv) the provision of improved specific, selective and safe TCRs or MHC-peptide complex binding fragments thereof, or antigen binding proteins.

### SUMMARY OF THE INVENTION

The invention is as defined in the claims.

The invention provides a bispecific antigen binding protein comprising at least two antigen binding sites B and D, wherein the antigen binding site D binds to T cell receptor (TCR) alpha/beta and wherein the antigen binding site B binds to a target antigenic (TA) peptide/MHC complex, wherein the antigen binding site D comprises a heavy chain variable domain (V_{H}) and a light chain variable domain (V_{L}), and wherein
(i) said V_{L} comprises or consists of the amino acid sequence of SEQ ID NO: 42, and
(ii) said V_{H} comprises or consists of the amino acid sequence of SEQ ID NO:

As set out in the claims, the invention further provides a nucleic acid coding the antigen binding protein, vectors comprising the coding nucleic acid, compositions comprising the protein or the coding nucleic acid or vector, host cells, and a method of producing the antigen binding protein.

Also provided, as set out in the claims, are the aforementioned protein, coding nucleic acid, vector or composition for use in medicine, and in particular for use in a method of treating a proliferative disease like cancer.

### DETAILED DESCRIPTION

The technical disclosure set out below may in some respects go beyond the scope of the claims. Elements of the disclosure which do not fall within the scope of the claims are provided for information.

The term "antigen binding protein" herein refers to polypeptides or binding proteins that are able to bind to at least one antigen.

The term "antigen" as used herein refers to a molecule or a portion of a molecule or complex that is capable of being bound by at least one antigen binding site, wherein said one antigen binding site is, for example, present in a conventional antibody, a conventional TCR and/or in the bispecific antigen binding proteins of the present invention.

The "bispecific antigen binding protein" of the present invention has at least two valences and binding specificities for at least two different antigens, in a bispecific antigen binding protein disclosed for reference antigen binding site A binds to CD3 and antigen binding site B binds to a target antigen (TA) peptide/MHC complex.

The antigen binding-site A specific for CD3 is derived from a newly humanized version of the mouse monoclonal antibody UCHT1, more particularly, from an improved humanized version of the mouse monoclonal antibody UCHT1 and, it is preferred, that the antigen binding site B is derived from a TCR.

The "bispecific antigen binding proteins" of the present invention are also herein referred to as the "antigen binding proteins of the invention" and they comprise at least 6 CDRs as defined in the context of the present invention. The antigen binding site B in the context of the present invention binds to a target antigenic (TA) peptide/MHC complex, in particular to a tumor associated antigen (TAA) peptide/MHC complex, and may be derived from an antibody or a TCR, preferably from a TCR. Accordingly, the antigen binding proteins of the invention comprises a bispecific antigen binding site B that binds to a target antigen (TA) peptide/MHC complex, wherein said antigen binding site B preferably comprises at least one variable alpha domain (v_{α}) and at least one variable beta domain (v_{β}) which derived from a TCR.

The bispecific antigen binding protein may also herein be referred to as "bispecific molecule".

The "target antigenic (TA) peptide" as used in the context of the present invention refers to peptides which have been isolated and identified from infected or tumorous material, such as material isolated from individuals suffering from tuberculosis, or from an infection of the Epstein-Barr virus or from cancer. The protein from which the TA peptide is derived is subject to antigen processing in an infected cell or a tumor cell, ten presented at the cell surface by the MHC molecule and the cell, in particular the TA peptide/MHC complex can thus be recognized by immune effector cells of the host, such as T cells or NKT cells. The TA peptide in the context of the present invention comprises or consists of 10, 12 or 14, such as 8 to 14, 8 to 12, for example 9 to 11 amino acids. In the context of the present invention, when it is referred to a specific TA peptide, it is referred to TA-C. Examples of TA antigenic peptides, such as TA-C peptides are viral antigenic peptides, bacterial antigenic peptides or tumour associated antigen (TAA) antigenic peptides, preferably TAA antigenic peptides. Accordingly, in one embodiment, the TA antigenic peptide, in particular the TA-C, is a viral peptide, a bacterial peptide or a tumour associated antigen (TAA) antigenic peptide, preferably a TAA antigenic peptide.

A "viral antigenic peptide" in the context of the present invention is an antigenic peptide that is presented by the MHC molecule on the surface of a diseased cell and is of a viral origin, i.e. the cell is typically infected by said virus. Such viral antigenic peptides have been discovered in the context of infections from, for example, human immunodeficiency viruses (HIV), Humane Cytomegalovirus (HCMV), cytomegalovirus (CMV), human papillomavirus (HPV), Hepatitis B virus (HBV), Hepatitis C virus (HCV), Epstein-Barr virus (EBV), Influenza virus. Accordingly, the viral antigenic peptide in the context of the present invention may be an antigenic peptide selected from the group consisting of HIV antigenic peptides, HCMV antigenic peptide, CMV antigenic peptides, HPV antigenic peptides, HBV antigenic peptides; HCV antigenic peptides; EBV antigenic peptides, Influenza antigenic peptides, preferably HIV, HBV, Influenza and HCMV antigenic peptides.

Viral antigenic peptides that are capable of use with methods and embodiments described herein include, for example, those viral antigenic peptides described in in the table herein below. In an aspect, viral antigenic peptides that are capable of use with the methods and embodiments described herein include at least one viral antigenic peptide comprising or consisting of an amino acid sequence selected from the amino acid sequences of SEQ ID NO: 146 to SEQ ID NO: 148, as depicted herein below in table 1.

**Table 1: List of viral antigenic peptides**

| SEQ ID NO: | Peptide | Virus | MHC |
|---|---|---|---|
| 146 | SLYNTVATL | HIV | HLA-A*02:01 |
| 147 | GILGFVFTL | Influenza A | HLA-A*02:01 |
| 148 | NLVPMVATV | HCMV | HLA-A*02:01 |

A "bacterial antigenic peptide" in the context of the present invention is an antigenic peptide that is presented by the MHC molecule on the surface of a diseased cell and is of a bacterial origin, i.e. the cell is typically infected by said bacteria. Such bacterial antigenic peptides have been discovered in the context of infections from, for example, *Mycobacterium tuberculosis.* Accordingly, the bacterial antigenic peptide in the context of the present invention may be a *Mycobacterium tuberculosis* antigenic peptide.

"Tumor-associated antigens (TAA) peptides" also referred to as "TAA peptides" herein denotes peptides which have been isolated and identified from tumorous material and which underwent antigen processing in a tumor cell and can thus be recognized by immune effector cells of the host. The TAA peptides comprises or consists of 10, 12 or 14, such as 8 to 14, 8 to 12, for example 9 to 11 amino acids. The TAA peptides in the context of the present invention may be for example a cancer/testis (CT) antigenic peptide. Examples of cancer/testis (CT) antigenic peptides are the MAGE-A antigenic peptide of the amino acid sequence of SEQ ID NO: 10 and the PRAME antigenic peptide of the amino acid sequence of SEQ ID NO: 9. The TAA peptide in the context of the present invention comprises a T cell epitope and may also be referred to as TAA peptide, in a general context, and as TAA peptide C in the context of the present invention when it is referred to one specific TAA peptide.

In an aspect, tumor associated antigen (TAA) peptides that are capable of use with methods and embodiments described herein include, for example, those TAA peptides described in U.S. Publication 20160187351, U.S. Publication 20170165335, U.S. Publication 20170035807, U.S. Publication 20160280759, U.S. Publication 20160287687, U.S. Publication 20160346371, U.S. Publication 20160368965, U.S. Publication 20170022251, U.S. Publication 20170002055, U.S. Publication 20170029486, U.S. Publication 20170037089, U.S. Publication 20170136108, U.S. Publication 20170101473, U.S. Publication 20170096461, U.S. Publication 20170165337, U.S. Publication 20170189505, U.S. Publication 20170173132, U.S. Publication 20170296640, U.S. Publication 20170253633, U.S. Publication 20170260249, U.S. Publication 20180051080, and U.S. Publication No. 20180164315.

In an aspect, the bispecific antigen binding proteins described herein, in particular the antigen binding site B in the context of the present invention, selectively recognize cells which present a TAA peptide described in one of more of the patents and publications described above. In another aspect, TAA that are capable of use with the methods and embodiments described herein include at least one TAA consisting of an amino acid sequence selected from the amino acid sequences of SEQ ID NO: 52 to 65, 67 to 96, 98 to 110, SEQ ID NO: 172 to 182, 184 to 268, SEQ ID NO: 9 and 10, preferably SEQ ID NO: 9 and 10. In an aspect, the bispecific antigen binding proteins, in particular the antigen binding site B of the bispecific antigen binding proteins, selectively recognize cells which present a TAA peptide/MHC complex, wherein the TAA peptide comprises or consist of an amino acid sequence of SEQ ID NO: 52 to 65, 67 to 96, 98, SEQ ID NO: 172 to 182, 184 to 268, SEQ ID NO: 9 and 10, or any of the amino acid sequences described in the patents or applications described herein, preferably SEQ ID NO: 9 and 10.

**Table 2: List of TAAs**

| SEQ ID NO: | Amino Acid Sequence | SEQ ID NO: | Amino Acid Sequence | SEQ ID NO: | Amino Acid Sequence |
|---|---|---|---|---|---|
| 52 | YLYDSETKNA | 105 | VLDGKVAVV | 219 | KMSELQTYV |
| 53 | HLMDQPLSV | 106 | GLLGKVTSV | 220 | ALLEQTGDMSL |
| 54 | GLLKKINSV | 107 | KMISAIPTL | 221 | VIIKGLEEITV |
| 55 | FLVDGSSAL | 108 | GLLETTGLLAT | 222 | KQFEGTVEI |
| 56 | FLFDGSANLV | 109 | TLNTLDINL | 223 | KLQEEIPVL |
| 57 | FLYKIIDEL | 110 | VIIKGLEEI | 224 | GLAEFQENV |
| 58 | FILDSAETTTL | 172 | YLEDGFAYV | 225 | NVAEIVIHI |
| 59 | SVDVSPPKV | 173 | KIWEELSVLEV | 226 | ALAGIVTNV |
| 60 | VADKIHSV | 174 | LLIPFTIFM | 227 | NLLIDDKGTIKL |
| 61 | IVDDLTINL | 175 | ISLDEVAVSL | 228 | VLMQDSRLYL |
| 62 | GLLEELVTV | 176 | KISDFGLATV | 10 | KVLEHVVRV |
| 63 | TLDGAAVNQV | 177 | KLIGNIHGNEV | 229 | LLWGNLPEI |
| 64 | SVLEKEIYSI | 178 | ILLSVLHQL | 230 | SLMEKNQSL |
| 65 | LLDPKTIFL | 179 | LDSEALLTL | 231 | KLLAVIHEL |
| 67 | YLMDDFSSL | 180 | VLQENSSDYQSNL | 232 | ALGDKFLLRV |
| 68 | KVWSDVTPL | 181 | HLLGEGAFAQV | 233 | FLMKNSDLYGA |
| 69 | LLWGHPRVALA | 182 | SLVENIHVL | 234 | KLIDHQGLYL |
| 70 | KIWEELSVLEV | 184 | SLSEKSPEV | 235 | GPGIFPPPPPQP |
| 71 | LLIPFTIFM | 185 | AMFPDTIPRV | 236 | ALNESLVEC |
| 72 | FLIENLLAA | 186 | FLIENLLAA | 237 | GLAALAVHL |
| 73 | LLWGHPRVALA | 187 | FTAEFLEKV | 238 | LLLEAVWHL |
| 74 | FLLEREQLL | 188 | ALYGNVQQV | 239 | SIIEYLPTL |
| 75 | SLAETIFIV | 189 | LFQSRIAGV | 240 | TLHDQVHLL |
| 76 | TLLEGISRA | 190 | ILAEEPIYIRV | 241 | SLLMWITQC |
| 77 | ILQDGQFLV | 191 | FLLEREQLL | 242 | FLLDKPQDLSI |
| 78 | VIFEGEPMYL | 192 | LLLPLELSLA | 243 | YLLDMPLWYL |
| 79 | SLFESLEYL | 193 | SLAETIFIV | 244 | GLLDCPIFL |
| 80 | SLLNQPKAV | 194 | AILNVDEKNQV | 245 | VLIEYNFSI |
| 81 | GLAEFQENV | 195 | RLFEEVLGV | 246 | TLYNPERTITV |
| 82 | KLLAVIHEL | 196 | YLDEVAFML | 247 | AVPPPPSSV |
| 83 | TLHDQVHLL | 197 | KLIDEDEPLFL | 248 | KLQEELNKV |
| 84 | TLYNPERTITV | 198 | KLFEKSTGL | 249 | KLMDPGSLPPL |
| 85 | KLQEKIQEL | 199 | SLLEVNEASSV | 250 | ALIVSLPYL |
| 86 | SVLEKEIYSI | 200 | GVYDGREHTV | 251 | FLLDGSANV |
| 87 | RVIDDSLVVGV | 201 | GLYPVTLVGV | 252 | ALDPSGNQLI |
| 88 | VLFGELPAL | 202 | ALLSSVAEA | 253 | ILIKHLVKV |
| 89 | GLVDIMVHL | 203 | TLLEGISRA | 254 | VLLDTILQL |
| 90 | FLNAIETAL | 204 | SLIEESEEL | 255 | HLIAEIHTA |
| 91 | ALLQALMEL | 205 | ALYVQAPTV | 256 | SMNGGVFAV |
| 92 | ALSSSQAEV | 206 | KLIYKDLVSV | 257 | MLAEKLLQA |
| 93 | SLITGQDLLSV | 207 | ILQDGQFLV | 258 | YMLDIFHEV |
| 94 | QLIEKNWLL | 208 | SLLDYEVSI | 259 | ALWLPTDSATV |
| 95 | LLDPKTIFL | 209 | LLGDSSFFL | 260 | GLASRILDA |
| 96 | RLHDENILL | 210 | VIFEGEPMYL | 261 | ALSVLRLAL |
| 98 | GLPSATTTV | 211 | ALSYILPYL | 262 | SYVKVLHHL |
| 99 | GLLPSAESIKL | 212 | FLFVDPELV | 263 | VYLPKIPSW |
| 100 | KTASINQNV | 213 | SEWGSPHAAVP | 264 | NYEDHFPLL |
| 9 | SLLQHLIGL | 214 | ALSELERVL | 265 | VYIAELEKI |
| 101 | YLMDDFSSL | 215 | SLFESLEYL | 266 | VHFEDTGKTLLF |
| 102 | LMYPYIYHV | 216 | KVLEYVIKV | 267 | VLSPFILTL |
| 103 | KVWSDVTPL | 217 | VLLNEILEQV | 268 | HLLEGSVGV |
| 104 | LLWGHPRVALA | 218 | SLLNQPKAV | | |

Furthermore, the TA antigenic peptide in the context of the present invention is a specific ligand of MHC-class-I-molecules or MHC-class-II-molecules, preferably MHC-class-I-molecules.

In the context of the present invention, the TAA antigenic peptide C is preferably selected from the group of TAA antigenic peptides consisting of the amino acids sequence of SEQ ID NO: 52 to 65, 67 to 96, 98 to 110, SEQ ID NO: 172 to 182, 184 to 268,, SEQ ID NO: 9 and SEQ ID NO: 10, preferably the PRAME antigenic peptide comprising or consisting of the amino acid sequence 'SLLQHLIGL' of SEQ ID NO: 9 or the MAGE-A antigenic peptide comprising or consisting of the amino acid sequence 'KVLEHVVRV' of SEQ ID NO: 10, more preferably SEQ ID NO: 10, wherein the MHC is preferably a HLA-A*02.

"PRAME" or "Preferentially Expressed Antigen In Melanoma" was first identified as an antigen that is over expressed in melanoma (Ikeda et al Immunity. 1997 Feb;6(2): 199-208); it is also known as CT130, MAPE, OIP-4 and has the Uniprot accession number P78395 (as available on January 11, 2019). The protein functions as a repressor of retinoic acid receptor signaling (Epping et al., Cell. 2005 Sep 23; 122(6):835-47). PRAME belongs to the family of germline-encoded antigens known as cancer testis antigens. Cancer testis antigens are attractive targets for immunotherapeutic intervention since they typically have limited or no expression in normal adult tissues. PRAME is expressed in a number of solid tumors as well as in leukemias and lymphomas (Doolan et al Breast Cancer Res Treat. 2008 May; 109(2):359-65; Epping et al Cancer Res. 2006 Nov 15;66(22): 10639-42; Ercolak et al Breast Cancer Res Treat. 2008 May; 109(2):359-65; Matsushita et al Leuk Lymphoma. 2003 Mar;44(3):439-44; Mitsuhashi et al Int. J Hematol. 2014; 100(1 ):88-95; Proto-Sequeire et al Leuk Res. 2006 Nov;30(11): 1333-9; Szczepanski et al Oral Oncol. 2013 Feb;49(2): 144-51; Van Baren et al Br J Haematol. 1998 Sep; 102(5): 1376-9). PRAME targeting therapies of the inventions may be particularly suitable for treatment cancers including, but not limited to, lung cancer, such as non-small cell lung cancer, small cell lung cancer, liver cancer, head and neck cancer, skin cancer, renal cell cancer, brain cancer, gastric cancer, colorectal cancer, hepatocellular cancer, pancreatic cancer, prostate cancer, leukemia, breast cancer, Merkel cell carcinoma, melanoma, ovarian cancer, urinary bladder cancer, uterine cancer, gallbladder and bile duct cancer, and esophageal cancer.

The "PRAME derived peptide" in context of the present invention comprises or consist of the amino acid sequence SLLQHLIGL (SEQ ID NO: 9) which corresponds to amino acids 425-433 of the full length PRAME protein of the amino acid sequence of SEQ ID NO: 7 as accessible under the Uniprot accession number P78395 (as available on January 11, 2019).). The PRAME derived peptide which comprises or consist of the amino acid sequence SLLQHLIGL (SEQ ID NO: 9) is also herein referred to as PRAME-004. The PRAME-004 peptide is a peptide epitope derived from a tumor-associated or tumor-specific protein and is presented on the cell surface by molecules of the major histocompatibility complex (MHC).More particularly, the PRAME-004 derived peptide is presented on the cell surface in complex with HLA-A*02. Med. 2001 Jan 1;193(1 ):73-88). In the context of the invention, the "PRAME derived peptide" or "PRAME-004" are used interchangeably and thus refer to PRAME derived peptide comprising or consisting of the amino acid sequence SLLQHLIGL (SEQ ID NO: 9).

"MAGE-A" or "melanoma associated antigen A" subfamily proteins were the first tumor associated antigens identified at the molecular level (van der Bruggen P, et al. Science. 1991; 254:1643-47). MAGE-A is a sub-family of 12 genes (MAGE-A1 to -A12) located in the q28 region of the X chromosome. Members of the MAGE-A subfamily proteins are normally expressed only in testis or placenta and their restricted expression suggests that they may function in germ cell development. MAGE-A proteins were also detected in the early development of the central nervous system and the spinal cord and brainstem, revealing that MAGE-A proteins may also be involved in neuronal development. The members of this family encode proteins with 50 to 80% sequence identity to each other and all MAGE proteins share the common MAGE homology domain (MHD), a highly conserved domain consisting of approximately 170 amino acids. The biological functions and underlying regulatory mechanism of MAGE-A proteins expression in cancer is still not fully understood.

The "MAGE-A4" or "Melanoma-associated antigen 4" protein is a member of the MAGE-A gene family and has the Uniprot accession number P43358 of SEQ ID NO: 111 (as available on July 8, 2019). MAGE-A4 localization has been described as cytoplasmic. However, MAGE-A4 staining has also been detected in nuclei, with differential distribution between nucleus and cytoplasm in well-differentiated versus less differentiated cancers (Sarcevic B et. al., 2003, Oncology 64, 443-449). MAGE-A4 is used as a male germ cell marker. It is not expressed in gonocytes, but expressed in pre-spermatogonia and mature germ cells (Mitchell et al., 2014, Mod. Pathol. 27, 1255-1266). Expression of the MAGE-A4 protein and mRNA has been linked to the development and prognosis of various cancers.

The "MAGE-A8" or "Melanoma-associated antigen 8" protein is a member of the MAGE-A superfamily and has the Uniprot accession number P43361 of SEQ ID NO: 112 (as available on July 8, 2019).

The "MAGE-A4" and "MAGE-A8" proteins have a sequence identity of 72% as determined by a protein sequence alignment using the BLASTP 2.9.0 algorithm (Stephen F et al. (1997) Nucleic Acids Res. 25:3389-3402). Furthermore, "MAGE-A4" and "MAGE-A8" both comprise the MAG-003 peptide, *i.e.,* KVLEHVVRV (SEQ ID NO: 10).

The term "epitope" as used in the context of the present invention comprises the terms "structural epitope" and "functional epitope". The "structural epitope" are those amino acids of the antigen, e.g. peptide-MHC complex, that are covered by the antigen binding protein when bound to the antigen. Typically, all amino acids of the antigen are considered covered that are within 5 Å of any atom of an amino acid of the antigen binding protein. The structural epitope of an antigen may be determined by art known methods including X-ray crystallography or NMR analysis. The structural epitope of an antibody typically comprises 20 to 30 amino acids. The structural epitope of a TCR typically comprises 20 to 30 amino acids. The "functional epitope" is a subset of those amino acids forming the structural epitope and comprises the amino acids of the antigen that are critical for formation of the interface with the antigen binding protein of the invention, either by directly forming non-covalent interactions such as H-bonds, salt bridges, aromatic stacking or hydrophobic interactions or by indirectly stabilizing the binding conformation of the antigen and is, for instance, determined by mutational scanning. Typically, the functional epitope of an antigen bound by an antibody comprises between 4 to 6 amino acids. Typically, the functional epitope of a peptide-MHC complex comprises between 2 to 6 amino acids of the peptide and 2 to 7 amino acids of the MHC molecule. Since MHC I presented peptides typically have a length between 8 to 10 amino acids only a subset of amino acids of each given peptide is part of the functional epitope of a peptide-MHC complex. In the context of the present invention, the epitope, in particular the functional epitope, bound by the bispecific antigen binding proteins of the present invention comprises or consists of the amino acids of the antigen that are required for formation of the binding interface, therefore the functional epitope comprises at least 3, preferably at least 4 amino acids of the MAGE-A antigenic peptide of SEQ ID NO: 10.

"CD3" in the context of the present invention denotes an antigen that is expressed on T cells as part of the multimolecular T cell receptor complex and that consists of at least three different chains: CD3ε, CD3δ and CD3γ. CD3δ and CD3γ have a low sequence identity and/or similarity to human CD3ε (similarity and identity is less than 20%). "CD3ε/δ-complex" refers to the complex that is formed by CD3ε and CDR3δ. CD3ε also forms a complex with CDR3γ, the so-called "CD3ε/γ-complex". Clustering of CD3 on T cells, e.g., by immobilized anti-CD3-antibodies, leads to T cell activation similar to the engagement of the T cell receptor, but independent from its clone typical specificity. "CD3ε" comprises three domains, an intracellular domain, a transmembrane domain and an extracellular domain.

The "UCHT1" monoclonal antibody specifically binds to the complex of human CD3δ-chain and the CD3ε-chain, herein referred to as CD3ε/δ-complex, a 36-kDa subunit of the CD3/T cell antigen receptor complex. The mouse monoclonal antibody UCHT-1 comprises a VL domain comprising or consisting of the amino acid sequence of SEQ ID NO: 36 and a VH domain comprising or consisting of the amino acid sequence of SEQ ID NO: 37. Humanization of UCHT1 has been described, for example, by Shalaby et al. (J. Exp. Med. (1992); 175(1): 217-225), which was then further modified resulting in the humanized variant 9 of UCHT1, referred to as hUCHT1(V9), which was described by Zhu et al. (J Immunol, 1995, 155, 1903-1910). hUCHT1(V9) comprises a VL domain comprising or consisting of the amino acid sequence of SEQ ID NO: 38 and a VH domain comprising or consisting of the amino acid sequence of SEQ ID NO: 39. However, humanized UCHT prior art variants suffer from low solubility and are difficult to use in the molecular context of soluble molecules. Furthermore, those prior art variants have a high affinity to CD3 which might be, as discovered in the context of the present invention, a disadvantage.

"BMA031" in the context of the present invention denotes the monoclonal antibody (mAb) WT31 which is specific for human alpha/beta TCRs. Different humanized variants have been disclosed in the art including, for example the alpha/beta TCR-specific humanized antibody BMA031 described in Shearman et al. (J Immunol, 1991, 147, 4366-73). The humanized antibody described in Sherman et al. (J Immunol, 1991, 147, 4366-73) comprises a V_{L} domain comprising or consisting of the amino acid sequence of SEQ ID NO: 40 and a V_{H} domain comprising or consisting of the amino acid sequence of SEQ ID NO: 41.

The "major histocompatibility complex" (MHC) in the context of the present invention is a set of cell surface proteins essential for the acquired immune system to recognize foreign molecules in vertebrates, which in turn determines histocompatibility. The main function of MHC molecules is to bind to antigens derived from pathogens and display them on the cell surface for recognition by the appropriate T cells. The human MHC is also called the HLA (human leukocyte antigen) complex (often just the HLA). The MHC gene family is divided into three subgroups: class I, class II, and class III. Complexes of peptide and MHC class I are recognized by CD8-positive T cells bearing the appropriate T cell receptor (TCR), whereas complexes of peptide and MHC class II molecules are recognized by CD4- positive-helper-T cells bearing the appropriate TCR. Since both types of response, CD8 and CD4 dependent, contribute jointly and synergistically to the anti-tumor effect, the identification and characterization of tumor-associated antigens and corresponding T cell receptors is important in the development of cancer immunotherapies such as vaccines and cell therapies. The HLA-A gene is located on the short arm of chromosome 6 and encodes the larger, α-chain, constituent of HLA-A. Variation of HLA-A α-chain is key to HLA function. This variation promotes genetic diversity in the population. Since each HLA has a different affinity for peptides of certain structures, greater variety of HLAs means greater variety of antigens to be 'presented' on the cell surface. Each individual can express up to two types of HLA-A, one from each of their parents. Some individuals will inherit the same HLA-A from both parents, decreasing their individual HLA diversity; however, the majority of individuals will receive two different copies of HLA-A. This same pattern follows for all HLA groups. In other words, every single person can only express either one or two of the 2432 known HLA-A alleles.

The MHC class I HLA protein in the context of the present invention may be an HLA-A, HLA-B or HLA-C protein, preferably HLA-A protein, more preferably HLA-A*02.

"HLA-A*02" signifies a specific HLA allele, wherein the letter A signifies the gene and the suffix "*02" indicates the A2 serotype.

In the MHC class I dependent immune reaction, peptides not only have to be able to bind to certain MHC class I molecules expressed by tumor cells, they subsequently also have to be recognized by T cells bearing specific T cell receptors (TCR).

A "TCR" in the context of the present invention is a heterodimeric cell surface protein of the immunoglobulin super-family, which is associated with invariant proteins of the CD3 complex involved in mediating signal transduction. TCRs exist in αβ and γδ forms, which are structurally similar but have quite distinct anatomical locations and probably functions. The extracellular portion of native heterodimeric αβ TCR and γδ TCR each contain two polypeptides, each of which has a membrane-proximal constant domain, and a membrane-distal variable domain. Each of the constant and variable domains include an intra-chain disulfide bond. The variable domains contain the highly polymorphic loops analogous to the complementarity determining regions (CDRs) of antibodies.

The term "TCR" herein denotes TCRs and fragments thereof, as well as single chain TCRs and fragments thereof, in particular variable alpha and beta domains of single domain TCRs, and chimeric, humanized, bispecific or multispecific TCRs.

"Fragments of a TCR" comprise a portion of an intact or native TCR, in particular the antigen binding region or variable region of the intact or native TCR. Examples of TCR fragments include fragments of the α, β, δ, γ chain, such as V_{α}- Cₐ or V_{β}- C_{β} or portions thereof, such fragments might also further comprise the corresponding hinge region or single variable domains, such as V_{α}, V_{β}, V_{δ}, V_{γ}, single chain VαVβ fragments or bispecific and multispecific TCRs formed from TCR fragments. Fragments of a TCR exert identical functions compared to the naturally occuring full-length TCR, i.e. fragments selectively and specifically bind to their target peptide.

"Single chain TCR (scTCR)" herein denotes a protein wherein the variable domains of the TCR, such as the V_{α} and V_{β} or V_{δ} and V_{γ} are located on one polypeptide. Typically, the variable domains are separated by a linker, wherein said linker typically comprises 5 to 20, such as 5 to 15 amino acids.

"Native" as used for example in the wording "native TCR" refers to a wildtype TCR. Native alpha-beta heterodimeric TCRs have an alpha chain and a beta chain. Each alpha chain comprises variable, joining and constant regions, and the beta chain also usually contains a short diversity region between the variable and joining regions, but this diversity region is often considered as part of the joining region. The constant, or C, regions of TCR alpha and beta chains are referred to as TRAC and TRBC respectively (Lefranc, (2001), Curr Protoc Immunol Appendix 1: Appendix 10). Each variable region, herein referred to as alpha variable domain and beta variable domain, comprises three Complementarity Determining Regions (CDRs) embedded in a framework sequence, one being the hypervariable region named CDR3. The alpha variable domain CDRs are herein referred to as CDRa1, CDRa2, CDRa3, and the beta

variable domain CDRs are herein referred to as CDRb1, CDRb2, CDRb3. There are several types of alpha chain variable (Valpha) regions and several types of beta chain variable (Vbeta) regions distinguished by their framework, CDR1 and CDR2 sequences, and by a partly defined CDR3 sequence. The Valpha types are referred to in IMGT nomenclature by a unique TRAV number, Vbeta types are referred in IMGT nomenclature to by a unique TRBV number (Folch and Lefranc, (2000), Exp Clin Immunogenet 17(1): 42-54; Scaviner and Lefranc, (2000), Exp Clin Immunogenet 17(2): 83-96; LeFranc and LeFranc, (2001), "T cell Receptor Factsbook", Academic Press). For more information on immunoglobulin antibody and TCR genes see the international ImMunoGeneTics information system^{®}, Lefranc M-P et al (Nucleic Acids Res. 2015 Jan;43 (Database issue):D413-22; and http://www.imgt.org/). A conventional TCR antigen-binding site, therefore, includes, usually, six CDRs, comprising the CDR set from each of an alpha and a beta chain variable region, wherein CDR1 and CDR3 sequences are relevant to the recognition and binding of the peptide antigen that is bound to the HLA protein and the CDR2 sequences are relevant to the recognition and binding of the HLA protein.

Analogous to antibodies, "TCR Framework Regions" (FRs) refer to amino acid sequences interposed between CDRs, i.e. to those portions of TCR alpha and beta chain variable regions that are to some extent conserved among different TCRs in a single species. The alpha and beta chains of a TCR each have four FRs, herein designated FR1-a, FR2-a, FR3-a, FR4-a, and FR1-b, FR2-b, FR3-b, FR4-b, respectively. Accordingly, the alpha chain variable domain may thus be designated as (FR1-a)-(CDRa1)-(FR2-a)-(CDRa2)-(FR3-a)-(CDRa3)-(FR4-a) and the beta chain variable domain may thus be designated as (FR1-b)-(CDRb1)-(FR2-b)-(CDRb2)-(FR3-b)-(CDRb3)-(FR4-b).

In the context of the invention, CDR/FR definition in an α or β chain or a γ or δ chain is to be determined based on IMGT definition (Lefranc et al. Dev. Comp. Immunol., 2003, 27(1):55-77; www.imgt.org). Accordingly, CDR/FR amino acid positions when related to TCR or TCR derived domains are indicated according to said IMGT definition. In one example, the IMGT position of the CDR/FR amino acid positions of the first variable domain is given in analogy to the IMGT numbering of TRAV5 and/or the IMGT position of the CDR/FR amino acid positions of the second variable domain is given in analogy to the IMGT numbering of TRBV12-4, this is for example the case for the variable domains of the antigen binding site B directed against MAGE-A antigenic peptide of SEQ ID NO: 10.

With respect to gamma/delta TCRs, the term "TCR gamma variable domain" as used herein refers to the concatenation of the TCR gamma V (TRGV) without leader region (L), and TCR gamma J (TRGJ) regions; and the term TCR gamma constant domain refers to the extracellular TRGC region, or to a C-terminal truncated TRGC sequence. Likewise the term "TCR delta variable domain" refers to the concatenation of the TCR delta V (TRDV) without leader region (L), and TCR delta D/J (TRDD/TRDJ) regions; and the term TCR delta constant domain refers to the extracellular TRDC region, or to a C-terminal truncated TRDC sequence.

In an "antibody" also called "immunoglobulin" two heavy chains are linked to each other by disulfide bonds and each heavy chain is linked to a light chain by a disulfide bond. There are two types of light chain, lambda (l) and kappa (k). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (V_{L}) and a constant domain (C_{L}). The heavy chain includes four domains, a variable domain (V_{H}) and three constant domains (C_{H1}, C_{H2} and C_{H3}, collectively referred to as C_{H}). The variable regions of both light (V_{L}) and heavy (V_{H}) chains determine binding recognition and specificity to the antigen. The constant region domains of the light (C_{L}) and heavy (C_{H}) chains confer important biological properties such as antibody chain association, secretion, trans-placental mobility, complement binding, and binding to F_{c} receptors (F_{c}R). The Fv fragment is the N-terminal part of the Fab fragment of an immunoglobulin and consists of the variable portions of one light chain and one heavy chain. The specificity of the antibody resides in the structural complementarity between the antibody combining site (synonym to antibody binding site) and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences that together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

In the context of the invention, the antibody or immunoglobulin is an IgM, IgD, IgG, IgA or IgE.

"Antibody Framework Regions" (FRs) refer to amino acid sequences interposed between CDRs, i.e. to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively. Accordingly, the light chain variable domain may thus be designated as (FR1-L)-(CDR1-L)-(FR2-L)-(CDR2-L)-(FR3-L)-(CDR3-L)-(FR4-L) and the heavy chain variable domain may thus be designated as (FR1-H)-(CDR1-H)-(FR2-H)-(CDR2-H)-(FR3-H)-(CDR3-H)-(FR4-H).

In the context of the invention, CDR/FR definition in an immunoglobulin light or heavy chain, in particular in the immunoglobulin light or heavy chain of the anti-CD3 antibody variants in the context of the present invention, is to be determined based on Kabat (Kabat EA, Te, Wu T, Foeller C, Perry HM, Gottesman KS. (1992) Sequences of Proteins of Immunological Interest.). However, positional numbering of CDR/FR amino acids of immunoglobulin light or heavy chains, in particular of the UCHT1 variants, is ordinal. Accordingly, CDRH1, for example, as determined according to Kabat ranges from amino acid position 31 to 35, CDRH2 as determined according to Kabat ranges from amino acid position 50 to 66 and CDRH3 as determined according to Kabat ranges from amino acid position 99 to 111. Accordingly, CDRL1, for example, as determined according to Kabat ranges from amino acid position 24 to 34, CDRL2 as determined according to Kabat ranges from amino acid position 50 to 56

As used herein, a "human framework region" is a framework region that is substantially identical (about 85%, or more, in particular 90%, 95%, 97%, 99% or 100%) to the framework region of a naturally occurring antigen binding protein, such as a naturally occurring human antibody or human TCR.

The term "antibody" denotes antibodies and fragments thereof, as well as single domain antibodies and fragments thereof, in particular a variable heavy chain of a single domain antibody, and chimeric, humanized, bispecific or multispecific antibodies.

A "conventional antibody" as herein referred to is an antibody that has the same domains as an antibody isolated from nature and comprises antibody derived CDRs and Framework regions. In analogy, a "conventional TCR" as herein referred to is a TCR that comprises the same domains as a native TCR and TCR derived CDRs and Framework regions.

The term "humanized antibody" refers to an antibody which is completely or partially of non-human origin and which has been modified to replace certain amino acids, in particular in the framework regions of the heavy and light chains of the non-human monoclonal antibody, in order to avoid or minimize an immune response in humans. The constant domains of a humanized antibody are mainly human C_{H} and C_{L} domains.

Numerous methods for humanization of an antibody sequence are known in the art; see e.g. the review by Almagro & Fransson (2008) Front Biosci. 13: 1619-1633. One commonly used method is CDR grafting, or antibody reshaping, which involves grafting of the CDR sequences of a donor antibody, generally a mouse antibody, into the framework scaffold of a human antibody of different specificity. Since CDR grafting may reduce the binding specificity and affinity, and, thus, the biological activity of a CDR grafted non-human antibody, back mutations may be introduced at selected positions of the CDR grafted antibody in order to retain the binding specificity and affinity of the parent antibody. Identification of positions for possible back mutations can be performed using information available in the literature and in antibody databases. An alternative humanization technique to CDR grafting and back mutation is resurfacing, in which non-surface exposed residues of non-human origin are retained, while surface residues are altered to human residues. Another alternative technique is known as "guided selection" (Jespers et al. (1994) Biotechnology 12, 899) and can be used to derive from for example a murine antibody a fully human antibody conserving the epitope and binding characteristics of the parental antibody. A further method of humanization is the so-called 4D humanization. The 4D humanization protocol is described in, for example in patent application US20110027266 A1 (WO2009032661A1). The monoclonal mouse antibody UCHT1 has been humanized as herein described in detail in Example 1. For chimeric antibodies, humanization typically involves modification of the framework regions of the variable region sequences.

"Vernier zone" in the context of the present invention refer to murine residues in framework regions that have been demonstrated to affect the conformation of CDR loops and affinity of the antibody. These residues, also called "Vernier residues" are located in the β-sheet framework regions closely underlying the CDRs and do not participate in a direct interaction with the antigen, i.e. these residues are retained in the "humanized" antibody (Foote & Winter, 1992).

Amino acid residues that are part of a CDR will typically not be altered in connection with humanization, although in certain cases it may be desirable to alter individual CDR amino acid residues, for example to remove a glycosylation site, a deamidation site, an isomerization site, or an undesired cysteine residue. N-linked glycosylation occurs by attachment of an oligosaccharide chain to an asparagine residue in the tripeptide sequence Asn-X-Ser or Asn-X-Thr, where X may be any amino acid except Pro. Removal of an N-glycosylation site may be achieved by mutating either the Asn or the Ser/Thr residue to a different residue, in particular by way of conservative substitution. Deamidation of asparagine and glutamine residues can occur depending on factors such as pH and surface exposure. Asparagine residues are particularly susceptible to deamidation, primarily when present in the sequence Asn-Gly, and to a lesser extent in other dipeptide sequences such as Asn-Ala. When such a deamidation site, in particular Asn-Gly, is present in a CDR sequence, it may therefore be desirable to remove the site, typically by conservative substitution to remove one of the implicated residues.

"Fragments of antibodies" in the context of the present invention comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of an antibody may also be a single domain antibody, such as a heavy chain antibody or VHH.

The term "Fab" denotes an antibody fragment having a molecular weight of about 50,000 Dalton and antigen binding activity, in which about a half of the N-terminal side of H chain and the entire L chain, among fragments obtained by treating IgG with a protease, e.g. papain, are bound together through a disulfide bond.

The term "format" in the context of the present invention refers to bispecific antigen binding proteins comprising a specific number and type of domains that are present in said bispecific antigen binding protein and the spatial organization thereof.

Many different formats, such as bispecific formats, are described in the art, typically, in the context of antibodies, such formats include the non-limiting examples, such as, diabodies, the Cross-Over-Dual-Variable-Domain (CODV) and/or in the Dual variable domain (DVD) proteins. An overview of these different bispecific antibodies and ways of making them is disclosed in, for example, Brinkmann U. and Kontermann R.E. MAbs. 2017 Feb-Mar; 9(2): 182-212. More particularly, the DVD format is, for example, disclosed in the following scientific articles (Wu C et al. Nat Biotechnol 2007; 25:1290-7; PMID:17934452; Wu C. et al. MAbs 2009; 1:339-47; Lacy SE et al. MAbs 2015; 7:605-19; PMID:25764208; Craig RB et al. PLoS One 2012; 7:e46778; PMID:23056448; Piccione EC et al. MAbs 2015). The CODV is for example disclosed in Onuoha SC et al. Arthritis Rheumatol. 2015 Oct; 67(10):2661-72 or for example in WO2012/135345, WO2016/116626). Bispecific diabodies are for example described in Holliger P et al. Protein Eng 1996; 9:299-305; PMID:8736497; Atwell JL et al. Mol Immunol 1996; 33:1301-12; PMID:9171890; Kontermann RE, Nat Biotechnol 1997; 15:629-31; PMID:9219263; Kontermann RE et al. Immunotechnology 1997; 3:137-44; PMID:9237098; Cochlovius B et al. Cancer Res 2000; 60:4336-41; PMID:10969772; and DeNardo DG et al. Cancer Biother Radiopharm 2001; 16:525-35; PMID:11789029).

"Diabodies" as used in the context of antibodies, typically refer to bivalent molecules composed of two chains, each comprising a VH and VL domain, either from the same or from different antibodies. The two chains typically have the configuration VHA-VLB and VHB-VLA (A and B representing two different specificities) or VLA-VHB and VLB-VHA.

In the context of the present invention, "diabodies (Db)" or the "diabody format" herein refers to bivalent molecules composed of two polypeptide chains, each comprising two variable domains connected by a linker (L_{Db1} and L_{Db2}), wherein two of the domains are first and second domains as defined in the context of the present invention (V₁ and V₂) and the other two domains may be TCR derived or antibody derived variable domains (V_{A},V_{B}). The V₁ and V₂ domains are located on two different polypeptides and the V_{A} and V_{B} domains are located on two different polypeptides and the domains dimerize in a head-to-tail orientation. Accordingly, the orientation may be V₁-L_{Db1}-V_{A} and V_{B}-L_{Db2}-V₂, V₂-L_{Db1}-V_{A} and V_{B}-L_{Db2}-V₁, V₁-L_{Db1}-V_{B} and V_{A}-L_{Db2}-V₂ or V₂-L_{Db1}-V_{B} and V_{A}-L_{Db2}-V₁. In order to allow the domains to dimerize head to-tail the linker, i.e. L_{Db1} and L_{Db1}, can be identical or different and are short linkers. A short linker is a linker that is typically between 2 to 12, 3 to 13, such as 3, 4, 5, 6, 7, 8, 9 amino acids long, for example 4, 5 (Brinkmann U. and Kontermann R.E. (MAbs. 2017 Feb-Mar; 9(2): 182-212) or 8 amino acids long, such as 'GGGS' of SEQ ID NO: 114, 'GGGGS' of SEQ ID NO: 115 or 'GGGSGGGG' of SEQ ID NO: 118.

The "dual-variable-domain immunoglobulin (DVD-Ig^{™})" format was initially described in 2007 by Wu C. et al. (Nat Biotechnol. 2007 Nov; 25(11):1290-7). In this format, the target-binding variable domains of a, typically, second monoclonal antibody (B) are typically fused to a conventional antibody (A) (comprising the domains V_{LA} and V_{HA}), wherein the light chain of the conventional antibody (A) thus comprises an additional light chain variable domain (V_{LB}) and the heavy chain of the conventional antibody (A) comprises an additional heavy chain variable domain (V_{HB}). The DVD-Ig^{™} as described in the art, is thus typically composed of two polypeptide chains, one heavy **chain comprising** V_{HB}-L-V_{HA}-C_{H1}-C_{H2}-C_{H3} and one light chain comprising V_{LB}-L-V_{LA}-C_{L}. The domain pairs V_{LA}/V_{HA} and V_{LA}/ V_{LA} are thus pairing in parallel.

In the context of the present invention, the "dual-variable-domain Ig format" refers to a protein comprising two polypeptide chains, each comprising two variable domains connected by a linker (L₁, L₃), wherein two of the domains are first and second domains as defined in the context of the present invention (V₁ and V₂) and the other two domains are antibody derived heavy and light chain variable domains (V_{HA} and V_{HB}). In the DVD-Ig format in the context of the present invention the polypeptide chains have, for example the organization V₁-L₁-V_{HA}-L₂-C_{H1}-C_{H2}-C_{H3} and V₂-L₃-V_{LA}-L₄-C_{L} or V₂-L₁-V_{HA}-L₂-C_{H1}-C_{H2}-C_{H3} and V₁-L₃-V_{LA}-L₄-C_{L}. The connecting linkers L₁ and L₃ are preferably between 5 to 20 amino acid residues long, such as 5 to 15 amino acid residues, and/or the connecting linkers L₂ and L₄ may be present or absent.

The "crossover dual-variable domain-lg-like proteins" as described in the art in the context of antibodies represent a format in which two V_{H} and two V_{L} domains are linked in a way that allows crossover pairing of the variable V_{H}-V_{L} domains, which are arranged either (from N- to C-terminus) in the order V_{HA}-V_{HB} and V_{LB}-V_{LA}, or in the order V_{HB}-V_{HA} and V_{LA}-V_{LB}.

In the context of the present invention, the "crossover dual-variable domain-Ig-like protein" refers to a protein comprising two polypeptide chains, each comprising two variable domains connected by a linker (L₁, L₂, L₃ and L₄), wherein two of the domains are first and second domains as defined in the context of the present invention (V₁ and V₂) and the other two domains are antibody derived heavy and light chain variable domains (V_{HA}, V_{HB}). In the CDVD-Ig format in the context of the present invention the polypeptide chains have, for example the organization V₁-L₁-V_{HA}-L₂-C_{H1}-C_{H2}-C_{H3} and V_{LA}-L₃-V₂-L₄-C_{L}, V₂-L₁-V_{HA}-L₂-C_{H1}-C_{H2}-C_{H3} and V_{LA}-L₃-V₁-L₄-C_{L}, V_{HA}-L₁-V₁-L₂-C_{H1}-C_{H2}-C_{H3} and V₂-L₃-V_{LA}-L_{DVD3}-C_{L} or V_{HA}-L₁-V₂-L₂-C_{H1}-C_{H2}-C_{H3} and V₁-L₃-V_{LA}-L₄-C_{L}. In this CDVD format, the linkers (L₁ to L₄) are typically of different length, including all-glycine linkers and linkers as described herein below in the section linkers. For example, L₁ is 3 to 12 amino acid residues in length, L₂ is 3 to 14 amino acid residues in length, L₃ is 1 to 8 amino acid residues in length, and L₄ is 1 to 3 amino acid residues in length, or L₁ is 5 to 10 amino acid residues in length, L₂ is 5 to 8 amino acid residues in length, L₃ is 1 to 5 amino acid residues in length, and L₄ is 1 to 2 amino acid residues in length or L₁ is 7 amino acid residues in length, L₂ is 5 amino acid residues in length, L₃ is 1 amino acid residues in length, and L₄ is 2 amino acid residues in length.

"At least one" herein refers to one or more of the specified objects such as 1, 2, 3, 4, 5 or 6 or more of the specified objects. For example, at least one binding site herein refers to 1, 2, 3, 4, 5 or 6 or more binding sites.

A sequence "at least 85% identical to a reference sequence" is a sequence having, on its entire length, 85%, or more, in particular 90%, 91%, 92%, 93%, 94%, 95%, 96%, 97%, 98% or 99% sequence identity with the entire length of a reference sequence.

In the context of the present application, the "percentage of identity" is calculated using a global pairwise alignment *(i.e.* the two sequences are compared over their entire length). Methods for comparing the identity of two or more sequences are well known in the art. The "needle" program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the World Wide Web site and is further described in the following publication *(*EMBOSS: The European Molecular Biology Open Software Suite (2000) Rice, P. Longden, I. and Bleasby, A. Trends in Genetics 16, (6) pp. 276-277). The percentage of identity between two polypeptides, in accordance with the invention, is calculated using the EMBOSS: needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95%, 96%, 97%, 98% or 99% identical" to a reference sequence may comprise amino acid mutations such as deletions, insertions and/or substitutions compared to the reference sequence. In case of substitutions, the protein consisting of an amino acid sequence at least 80%, 85%, 90%, 95%, 96%, 97%, 98% or 99% identical to a reference sequence may correspond to a homologous sequence derived from another species than the reference sequence.

"Amino acid substitutions" may be conservative or non-conservative. Preferably, substitutions are conservative substitutions, in which one amino acid is substituted for another amino acid with similar structural and/or chemical properties.

Conservative substitutions may include those, which are described by Dayhoff in "The Atlas of Protein Sequence and Structure. Vol. 5", Natl. Biomedical Research. For example, amino acids, which belong to one of the following groups, can be exchanged for one another, thus, constituting a conservative exchange: Group 1: alanine (A), proline (P), glycine (G), asparagine (N), serine (S), threonine (T); Group 2: cysteine (C), serine (S), tyrosine (Y), threonine (T); Group 3: valine (V), isoleucine (I), leucine (L), methionine (M), alanine (A), phenylalanine (F); Group 4: lysine (K), arginine (R), histidine (H); Group 5: phenylalanine (F), tyrosine (Y), tryptophan (W), histidine (H); and Group 6: aspartic acid (D), glutamic acid (E). In an aspect, a conservative amino acid substitution may be selected from the following of T→A, G→A, A→I, T→V, A→M, T→l, A→V, T→G, and/or T→S.

A conservative amino acid substitution may include the substitution of an amino acid by another amino acid of the same class, for example, (1) nonpolar: Ala, Val, Leu, Ile, Pro, Met, Phe, Trp; (2) uncharged polar: Gly, Ser, Thr, Cys, Tyr, Asn, Gln; (3) acidic: Asp, Glu; and (4) basic: Lys, Arg, His. Other conservative amino acid substitutions may also be made as follows: (1) aromatic: Phe, Tyr, His; (2) proton donor: Asn, Gln, Lys, Arg, His, Trp; and (3) proton acceptor: Glu, Asp, Thr, Ser, Tyr, Asn, Gln (see, for example, U.S. Patent No. 10,106,805,).

Conservative substitutions may be made in accordance with Table 3. Methods for predicting tolerance to protein modification may be found in, for example, Guo et al., Proc. Natl. Acad. Sci., USA, 101(25):9205-9210 (2004).

**Table 3: Conservative Amino Acid substitution**

| Conservative Amino Acid Substitutions | |
|---|---|
| Amino Acid | Substitutions (others are known in the art) |
| Ala | Ser, Gly, Cys |
| Arg | Lys, Gln, His |
| Asn | Gln, His, Glu, Asp |
| Asp | Glu, Asn, Gln |
| Cys | Ser, Met, Thr |
| Gln | Asn, Lys, Glu, Asp, Arg |
| Glu | Asp, Asn, Gln |
| Gly | Pro, Ala, Ser |
| His | Asn, Gln, Lys |
| Ile | Leu, Val, Met, Ala |
| Leu | Ile, Val, Met, Ala |
| Lys | Arg, Gln, His |
| Met | Leu, Ile, Val, Ala, Phe |
| Phe | Met, Leu, Tyr, Trp, His |
| Ser | Thr, Cys, Ala |
| Thr | Ser, Val, Ala |
| Trp | Tyr, Phe |
| Tyr | Trp, Phe, His |
| Val | Ile, Leu, Met, Ala, Thr |

Conservative substitutions may be those shown in Table 3 under the heading of "conservative substitutions." If such substitutions result in a change in biological activity, then more substantial changes, denominated "exemplary substitutions" in Table 4, may be introduced and the products screened if needed.

**Table 4: Amino Acid substitution**

| Amino Acid Substitutions | | |
|---|---|---|
| Original Residue (naturally occurring amino acid) | Conservative Substitutions | Exemplary Substitutions |
| Ala (A) | Val | Val; Leu; Ile |
| Arg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn; Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

In some embodiments disclosed for reference and not claimed, the bispecific antigen binding protein may include a variant antigen binding protein, wherein said variant bispecific antigen binding protein includes a first polypeptide chain (such as an α chain) and a second polypeptide chain (such as a β chain) comprising for up to 8, 9, 10, 11, 12, 13, 14, 15, or more amino acid substitutions, preferably in the CDR regions of the first variable domain (such as a Valpha domain) and the second variable domain (such as a Vbeta domain) in comparison to the bispecific antigen binding protein from which the variant is derived. In this regard, there may be 1, 2, 3, 4, 5, 6, 7, 8, 9, 10, 11, 12, 13, 14, 15, or more amino acid substitutions in each of the CDR regions of the bispecific antigen binding protein or in all of the CDR regions of the first and/or second variable domain. Substitutions may be in the CDRs either in the first and/or the second variable domain.

In one embodiment disclosed for reference and not claimed, the variant is a functional variant.

The term "functional variant" as used herein refers to an bispecific antigen binding protein having substantial or significant sequence identity or similarity to a parent bispecific antigen binding protein, such as those bispecific antigen binding proteins containing conservative amino acid substitutions, wherein said functional variant retains the biological activity of the parental bispecific antigen binding protein. Functional variants encompass, for example, those variants of bispecific antigen binding proteins described herein (the bispecific antigen binding proteins described herein are then themselves referred to as parent antigen binding proteins) that retain the ability to recognize target cells to a similar extent, the same extent, or to a higher extent, as the parent bispecific antigen binding proteins. In reference to the parent bispecific antigen binding protein the functional variant can, for instance, have an amino acid sequence that is sequence at least 80%, 85%, 90%, 95%, 96%, 97%, 98% or 99% identical to the amino acid sequence to the parent bispecific antigen binding protein.

The functional variant can, for example, comprise the amino acid sequence of the parent bispecific antigen binding protein with at least one conservative amino acid substitution. Alternatively, or additionally, the functional variants can comprise the amino acid sequence of the parent bispecific antigen binding protein with at least one non-conservative amino acid substitution. In this case, it is preferable for the non-conservative amino acid substitution to not interfere with or inhibit the biological activity of the functional variant. Preferably, the non-conservative amino acid substitution enhances the biological activity of the functional variant, such that the biological activity of the functional variant is increased as compared to the parental bispecific antigen binding protein.

The modified TCRs, polypeptides, and antigen binding proteins of the present invention (including functional portions, fragments and functional variants) can be of any length, i.e., can comprise any number of amino acids, provided that the modified TCRs, polypeptides, or proteins (or functional portions or functional variants thereof) retain their biological activity, e.g., the ability to specifically bind to antigen, detect diseased cells in a host, or treat or prevent disease in a host, etc.

The bispecific antigen binding proteins of the present invention (including functional portions, fragments and functional variants) can comprise synthetic amino acids in place of one or more naturally-occurring amino acids. Such synthetic amino acids are known in the art, and may include, for example, aminocyclohexane carboxylic acid, norleucine, α-amino n-decanoic acid, homoserine, S-acetylaminomethyl-cysteine, trans-3- and trans-4-hydroxyproline, 4-aminophenylalanine, 4-nitrophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, α-(2-amino-2-norbornane)-carboxylic acid, α,γ-diaminobutyric acid, α,β-diaminopropionic acid, homophenylalanine, and α-tert-butylglycine.

The bispecific antigen binding protein (including functional portions and functional variants) can be glycosylated, amidated, carboxylated, phosphorylated, esterified, N-acylated, cyclized via, e.g., a disulfide bridge, or converted into an acid addition salt and/or optionally dimerized or polymerized, or conjugated.

The bispecific antigen binding protein of the present disclosure can be synthetic, recombinant, isolated, and/or purified.

A "covalent link" herein refers for example to a disulfide bridge or a peptide link or a covalent link via a linker or linker sequence, such as a polypeptide linker.

The term "linker" as used herein refers to one or more amino acid residues inserted between two domains to provide sufficient mobility for the domains, for example in single chain constructs, between the first variable domain and the second variable domain of the bispecific antigen binding proteins of the invention and, optionally, between the variable domains of light and heavy chain variable domains, to fold correctly to form the antigen binding site or, in case of bispecific antigen binding proteins, to form the antigen binding site and the at least one further antigen binding site, either in a cross over pairing (in a CODV format or in some of the diabody formats) or in a parallel pairing configuration (for example, in a DVD format) of the bispecific antigen binding proteins of the invention.

In some embodiments, a linker consists of 0 amino acid meaning that the linker is absent. A linker is inserted at the transition between variable domains or between variable domains and constant domains, respectively, at the amino acid sequence level. The transition between domains can be identified because the approximate size of the immunoglobulin domains as well as of the TCR domains is well understood. It is known by the skilled in the art, that the precise location of a domain transition can be determined by locating peptide stretches that do not form secondary structural elements such as beta-sheets or alpha-helices as demonstrated by experimental data or can be identified or assumed using techniques of modeling or secondary structure prediction. The term linker used in the context of the present invention refers but is not limited to the linkers referred to as L₁, L₂, L₃, L₄, L₅ and L₆.

A linker, as long as it is not specified otherwise in the respective context, such as L₁, L₂, L₃, L₄, L₅ and L₆, can be from at least 1 to 30 amino acids in length. In some embodiments, a linker, such as L₁, L₂, L₃, L₄, L₅ and L₆, can be 2-25, 2-20, or 3-18 amino acids long. In some embodiments, a linker, such as L₁, L₂, L₃, L₅ and L₆, can be a peptide of a length of no more than 14, 13, 12, 11, 10, 9, 8, 7, 6, or 5 amino acids. In other embodiments, a linker, such as L₁, L₂, L₃, L₄, L₅ and L₆, can be 5-25, 5-15, 4-11, 10-20, or 20-30 amino acids long. In other embodiments, a linker, such as L₁, L₂, L₃, L₄, L₅ and L₆, can be about, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids long. In a particular embodiment linkers, such as L₁, L₂, L₃, L₄, L₅ and L₆, may be less than 24, less than 20, less than 16, is less than 12, less than 10, for example from 5 to 24, 10 to 24 or 5-10 amino acid residues in length. In some embodiments, said linker is equal to 1 or more amino acid residues in length, such as more than 1, more than 2, more than 5, more than 10, more than 20 amino acid residues in length, more than 22 amino acid residues in length. Exemplary linkers, such as L₁, L₂, L₃, L₄, L₅ and L₆, comprise or consist of the amino acid sequence selected from the group of amino acids consisting of the amino acid sequences TVAAP (SEQ ID NO: 113), GGGS (SEQ ID NO: 114), GGSGG (SEQ ID NO: 28), GGGGS (SEQ ID NO: 115), TVLRT (SEQ ID NO: 116), TVSSAS (SEQ ID NO: 117), GGGSGGGG (SEQ ID NO: 118), GGGGSGGGGS (SEQ ID NO: 119), GGGGSAAA (SEQ ID NO: 120), GGSGGGGSGG (SEQ ID NO: 29), GGSGGGGSGGGGSGG (SEQ ID NO: 32), GGGGSGGGGSGGGGS (SEQ ID NO: 121) GGGGSGGGGSGGGGSGGGGSGGGGSGS (SEQ ID NO: 122), GGSGGGGSGGGGSGGGGSGG (SEQ ID NO: 33), GGGGSGGGGSGGGGSGGGGSGGGGS (SEQ ID NO: 123), GGSGGGGSGGGGSGGGGSGGGGSGG (SEQ ID NO: 66), GSADDAKKDAAKKDGKS (SEQ ID NO: 97), GGQGSGGTGSGGQGSGGTGSGGQGS (SEQ ID NO: 143), TVLSSAS (SEQ ID NO: 124), GGGGSGT (SEQ ID NO: 183) and GGGGSGGGGSGGGGSGGGGS (SEQ ID NO: 125), in particular GGGSGGGG (SEQ ID NO:118), GGGGSGGGGSGGGGSGGGGS (SEQ ID NO: 125) and GGGGSGGGGSGGGGSGGGGSGGGGS (SEQ ID NO: 123).

The term "F_{c} domain" as used in the context of the present invention encompasses native F_{c} domains and F_{c} domain variants and sequences as further defined herein below. As with F_{c} variants and native F_{c} molecules, the term "F_{c} domain" includes molecules in monomeric or multimeric form, whether digested from whole antibody or produced by other means.

The term "native F_{c}" as used herein refers to a molecule comprising the sequence of a non-antigen-binding fragment resulting from digestion of an antibody or produced by other means, whether in monomeric or multimeric form, and may contain the hinge region. The original immunoglobulin source of the native F_{c} is, in particular, of human origin and can be any of the immunoglobulins, preferably IgG1 or IgG2, most preferably IgG1. Native Fc molecules are made up of monomeric polypeptides that can be linked into dimeric or multimeric forms by covalent (i.e., disulfide bonds) and non-covalent association. The number of intermolecular disulfide bonds between monomeric subunits of native F_{c} molecules ranges from 1 to 4 depending on class (e.g., IgG, IgA, and IgE) or subclass (e.g., IgG1, IgG2, IgG3, IgA1, and IgGA2). One example of a native F_{c} is a disulfide-bonded dimer resulting from papain digestion of an IgG. The term "native F_{c}" as used herein is generic to the monomeric, dimeric, and multimeric forms. One example of a native F_{c} amino acid sequence is the amino acid sequence of SEQ ID NO: 126, which is the native Fc amino acid sequence of IGHG1*01.

The "hinge" or "hinge region" or "hinge domain" refers typically to the flexible portion of a heavy chain located between the C_{H1} domain and the C_{H2} domain. It is approximately 25 amino acids long, and is divided into an "upper hinge," a "middle hinge" or "core hinge," and a "lower hinge." A "hinge subdomain" refers to the upper hinge, middle (or core) hinge or the lower hinge. The amino acids sequences of the hinges of an IgG1, IgG2, IgG3 and IgG4 molecule are indicated herein below:
IgG1: E₂₁₆PKSCDKTHTCPPCPAPELLG (SEQ ID No. 127)
IgG2: E₂₁₆RKCCVECPPCPAPPVAGP (SEQ ID No. 128)
IgG3:ELKTPLGDTTHTCPRCPEPKSCDTPPPCPRCPE₂₁₆PKSCDTPPPCPRCPAPELL
G (SEQ ID No. 129)
IgG4: E₂₁₆SKYGPPCPSCPAPEFLG (SEQ ID No. 130).

In the context of the present invention it is referred to amino acid positions in the Fc domain, these amino acid positions or residues are indicated according to the EU numbering system as described, for example in Edelman, G.M. et al., Proc. Natl. Acad. USA, 63, 78-85 (1969).

The term "F_{c} variant" as used herein refers to a molecule or sequence that is modified from a native F_{c} but still comprises a binding site for the salvage receptor, F_{c}Rn (neonatal F_{c} receptor). Exemplary F_{c} variants, and their interaction with the salvage receptor, are known in the art. Thus, the term "F_{c} variant" can comprise a molecule or sequence that is humanized from a non-human native F_{c}. Furthermore, a native F_{c} comprises regions that can be removed because they provide structural features or biological activity that are not required for the bispecific antigen binding proteins of the invention. Thus, the term "F_{c} variant" comprises a molecule or sequence that lacks one or more native F_{c} sites or residues, or in which one or more F_{c} sites or residues has be modified, that affect or are involved in: (1) disulfide bond formation, (2) incompatibility with a selected host cell, (3) N-terminal heterogeneity upon expression in a selected host cell, (4) glycosylation, (5) interaction with complement, (6) binding to an F_{c} receptor other than a salvage receptor, or (7) antibody-dependent cellular cytotoxicity (ADCC).

Accordingly, in one embodiment the Fc-domain, such as F_{c1} and/or F_{c2}, comprises a hinge domain.

In one embodiment, the Fc-domain is a human IgG Fc domain, preferably derived from human IgG1, IgG2, IgG3 or IgG4, preferably IgG1 or IgG2, more preferably IgG1.

In some embodiments, in particular, when the bispecific antigen binding protein comprises two Fc domains, i.e. in the TCER^{®} format described herein below (such as F_{c1} and F_{c2}), the two Fc domains may be of the same immunoglobulin isotype or isotype subclass or of different immunoglobulin isotype or isotype subclass, preferably of the same. Accordingly, in some embodiments F_{c1} and F_{c2}, are of the IgG1 subclass, or of the IgG2 subclass, or of the IgG3 subclass, or of the IgG4 subclass, preferably of the IgG1 subclass, or of the IgG2 subclass, more preferably of the IgG1 subclass.

In some embodiments, the Fc domain is a variant Fc domain and thus comprises one or more of the amino acid substitutions described herein below.

In some embodiments, the F_{c} domain comprises or further comprise the "RF" and/or "Knob-into-hole" mutation, preferably the "Knob-into-hole".

The "RF mutation" typically refers to the amino acid substitutions of the amino acids HY into RF in the C_{H3} domain of F_{c} domains, such as the amino acid substitution H435R and Y436F in C_{H3} domain as described by Jendeberg, L. et al. (1997, J. Immunological Meth., 201: 25-34) and is described as advantageous for purification purposes as it abolishes binding to protein A. In case the bispecific antigen binding protein comprises two F_{C}-domains, the RF mutation may be in one or both, preferably in one Fc-domain.

The "Knob-into-Hole" or also called "Knob-into-Hole" technology refers to amino acid substitutions T366S, L368A and Y407V (Hole) and T366W (Knob) both in the C_{H3}-C_{H3} interface to promote heteromultimer formation. Those knob-into-hole mutation can be further stabilized by the introduction of additional cysteine amino acid substitutions Y349C and S354C. The "Knob-into-Hole" technology together with the stabilizing cysteine amino acid substitutions has been described in patents US5731168 and US8216805.

In the context of the present invention, the "Knob" mutation together with the cysteine amino acid substitution S354C is, for example, present in the Fc domain comprising or consisting of the amino acid sequence of SEQ ID NO: 131 and the "Hole" mutation together with the cysteine amino acid substitutions Y349C is present in the Fc domain comprising or consisting amino acid sequence of SEQ ID NO: 132.

In some embodiments, the Fc domain of one of the polypeptides, for example F_{c1}, comprises the amino acid substitution T366W (Knob) in its C_{H3} domain and the Fc domain of the other polypeptide, for example F_{c2}, comprises the amino acid substitution T366S, L368A and Y407V (Hole) in its C_{H3} domain, or *vice versa.*

In some embodiments, the Fc domain of one of the polypeptides, for example F_{c1}, comprises or further comprises the amino acid substitution S354C in its C_{H3} domain and the F_{c} domain of the other polypeptide, for example F_{c2}, comprises or further comprises the amino acid substitution Y349C in its C_{H3} domain, or *vice versa.*

Accordingly, in some embodiments, the Fc domain of one of the polypeptides, for example F_{c1}, comprises the amino acid substitutions S354C and T366W (Knob) in its C_{H3} domain and the Fc domain of the other polypeptide, for example F_{c2}, comprises the amino acid substitution Y349C, T366S, L368A and Y407V (Hole) in its C_{H3} domain, or *vice versa.*

This set of amino acid substitutions can be further extended by inclusion of the amino acid substitutions K409A on one polypeptide and F405K in the other polypeptide as described by Wei et al. (Structural basis of a novel heterodimeric Fc for bispecific antibody production, Oncotarget. 2017). Accordingly, in some embodiments, the F_{c} domain of one of the polypeptides, for example F_{c1}, comprises or further comprises the amino acid substitution K409A in its C_{H3} domain and the F_{c} domain of the other polypeptide, for example F_{c2}, comprises or further the amino acid substitution F405K in its C_{H3} domain, or *vice versa.*

In some cases artificially introduced cysteine bridges may improve the stability of the bispecific antigen binding proteins, optimally without interfering with the binding characteristics of the bispecific antigen binding proteins. Such cysteine bridges can further improve heterodimerization.

Further amino acid substitutions, such as charged pair substitutions, have been described in the art, for example in EP 2 970 484 to improve the heterodimerization of the resulting proteins.

Accordingly in one embodiment, the Fc domain of one of the polypeptides, for example F_{c1}, comprises or further comprises the charge pair substitutions E356K, E356R, D356R, or D356K and D399K or D399R, and the F_{c} domain of the other polypeptide, for example F_{c2}, comprises or further comprises the charge pair substitutions R409D, R409E, K409E, or K409D and N392D, N392E, K392E, or K392D, or *vice versa.*

In a further embodiment, the F_{c} domain on one or both, preferably both polypeptide chains can comprise one or more alterations that inhibit F_{c} gamma receptor (FcyR) binding. Such alterations can include L234A, L235A.

With the inclusion of Fc-parts consisting of Hinges, C_{H2} and C_{H3} domains, or parts thereof, into antigen binding proteins, more particularly into bispecific antigen binding proteins the problem of unspecific immobilization of these molecules, induced by Fc:Fc-gamma receptor (FcgR) interactions arose. FcgRs are composed of different cell surface molecules (FcgRI, FcgRlla, FcgRIIb, FcgRIII) binding with differing affinities to epitopes displayed by Fc-parts of IgG-molecules. As such an unspecific (i.e. not induced by either of the two binding domains of a bispecific molecule) immobilization is unfavorable due to i) influence on pharmacokinetics of a molecule and ii) off-target activation of immune effector cells various Fc-variants and mutations to ablate FcgR-binding have been identified. In this context, Morgan et al. 1995, Immunology (The N-terminal end of the C_{H2} domain of chimeric human IgG1 anti-HLA-DR is necessary for C1q, FcyRI and FcyRIll binding) disclose the exchange of the residues 233-236 of human IgG1 with the corresponding sequence derived from human IgG2, i.e. the residues 233P, 234V and 235A and wherein no amino acid is present at position 236, resulting in abolished FcgRI binding, abolished C1q binding and diminished FcgRIll binding. EP1075496 discloses antibodies and other Fc-containing molecules with variations in the Fc region (such as one or more of 233P, 234V, 235A and no residue or G in position 236 and 327G, 330S and 331S) wherein the recombinant antibody is capable of binding the target molecule without triggering significant complement dependent lysis, or cell mediated destruction of the target.

Accordingly, in some embodiments, the Fc region comprises or further comprise one or more of the amino acids or deletions selected from the group consisting of 233P, 234V, 235A, 236 (No residue) or G, 327G, 330S, 331S, preferably, the Fc region comprises or further comprises the amino acids 233P, 234V, 235A, 236 (No residue) or G and one or more amino acids selected from the group consisting of 327G, 330S, 331S, most preferably, the Fc region comprises or further comprises the amino acids 233P, 234V, 235A, 236 (No residue) and 331S.

In one further embodiment, the Fc domain comprises or further comprises the amino acid substitution N297Q, N297G or N297A, preferably N297Q.

The amino acid substitution "N297Q", "N297G" or "N297A" refer to amino acid substitutions at position 297 that abrogate the native N-Glycosylation site within the Fc-domain. This amino acid substitution further prevents Fc-gamma-receptor interaction and decreases the variability of the final protein products, i.e. the bispecific antigen binding proteins of the present invention, due to sugar residues as described for example in Tao, MH and Morrison, SL (J Immunol. 1989 Oct 15;143(8):2595-601.).

In one further embodiment, in particular when no light chain, the Fc domain comprises or further comprises the amino acid substitution C220S. The amino acid substitution "C220S" deletes the cysteine forming the C_{H1}-CL disulfide-bridge.

In some embodiments, the F_{c} domain comprises or further comprises at least two additional cysteine residues, for example S354C and Y349C or L242C and K334C, wherein S354C is in the Fc-domain of one polypeptide, such as F_{c1}, and Y349C is in the Fc domain of the other polypeptide, such as F_{c2}, to form a heterodimer and/or wherein L242C and K334C are located in the same Fc-domain, either in the F_{c1} or F_{c2} of one or both polypeptides to form a intradomain C-C bridge.

By "purified" and "isolated" it is meant, when referring to a polypeptide (i.e. the bispecific antigen binding protein of the invention) or a nucleotide sequence, that the indicated molecule is present in the substantial absence of other biological macromolecules of the same type. The term "purified" as used herein in particular means at least 75%, 85%, 95%, or 98% by weight, of biological macromolecules of the same type are present.

An "isolated" nucleic acid molecule that encodes a particular polypeptide refers to a nucleic acid molecule that is substantially free of other nucleic acid molecules that do not encode the subject polypeptide; however, the molecule may include some additional bases or moieties, which do not deleteriously affect the basic characteristics of the composition.

A "domain" may be any region of a protein, generally defined on the basis of sequence homologies and often related to a specific structural or functional entity.

A "recombinant" molecule is one that has been prepared, expressed, created, or isolated by recombinant means.

The term "gene" means a DNA sequence that codes for, or corresponds to, a particular sequence of amino acids which comprises all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

"Affinity" is defined, in theory, by the equilibrium binding between the bispecific antigen binding protein and the antigen, in the context of the present invention by the equilibrium binding between the bispecific antigen binding protein and its antigen TA/MHC, or TA-C/MHC or CD3. Affinity may be expressed for example in half-maximal effective concentration (EC₅₀) (sometimes also referred to as half-maximal binding concentration (EC₅₀)) or the equilibrium dissociation constant (K_{D}).

"K_{D}" is the equilibrium dissociation constant, a ratio of k_{off}/kₒₙ, between the bispecific antigen binding protein and its antigen. K_{D} and affinity are inversely related. The K_{D} value relates to the concentration of the bispecific antigen binding protein and the lower the K_{D} value, the higher the affinity of the bispecific antigen binding protein. Affinity, i.e. the K_{D} value, can be experimentally assessed by a variety of known methods, such as measuring association and dissociation rates with surface plasmon resonance (SPR) or biolayer interferometry (BLI), as described in more detail herein below in the section 'Bispecific antigen binding proteins'.

"Half maximal effective concentration" also called "EC₅₀" typically refers to the concentration of a molecule which induces a response halfway between the baseline and maximum after a specified exposure time. EC₅₀ and affinity are inversely related, the lower the EC₅₀ value the higher the affinity of the molecule. In one example, the "EC₅₀" refers to the concentration of the bispecific antigen binding protein of the invention which induces a response halfway between the baseline and maximum after a specified exposure time, more particularly, refers to the concentration of the bispecific antigen binding protein of the invention which induces a response halfway between the baseline and maximum after a specified exposure time. EC₅₀ values can be experimentally assessed by a variety of known methods, using for example a IFN-gamma release assay or a LDH release assay as described in more detail in the experimental section in example 2 and 5. In the context of the present invention the EC₅₀ value is preferably determined by LDH release assay and thus, refers to induced cytotoxicity.

A "diagnostic agent" herein refers to a detectable molecule or substance, such as a fluorescent molecule, a radioactive molecule or any other labels known in the art that provide (either directly or indirectly) a signal.

"Fluorescent molecules" are known in the art include fluorescein isothiocyanate (FITC), phycoerythrin (PE), fluorophores for use in the blue laser (e.g. PerCP, PE-Cy7, PE-Cy5, FL3 and APC or Cy5, FL4), fluorophores for use in the red, violet or uv laser (e.g. Pacific blue, pacific orange).

"Radioactive molecules" include but are not limited radioactive atom for scintigraphic studies such as I¹²³, I¹²⁴, In¹¹¹, Re¹⁸⁶, Re¹⁸⁸, Tc⁹⁹. Bispecific antigen binding proteins of the invention may also comprise a spin label for nuclear magnetic resonance (NMR) imaging (also known as magnetic resonance imaging, MRI), such as iodine-123, indium-111, fluorine-19, carbon-13, nitrogen-15, oxygen-17, gadolinium, manganese or iron.

Such diagnostic agents are may be either directly coupled (i.e., physically linked) to the bispecific antigen binding protein or may be indirectly linked.

A "therapeutic agent" herein refers to an agent that has a therapeutic effect. In one embodiment, such a therapeutic agent may be a growth inhibitory agent, such as a cytotoxic agent or a radioactive isotope.

A "growth inhibitory agent", or "anti-proliferative agent", which can be used indifferently, refers to a compound or composition which inhibits growth of a cell, especially a tumour cell, either *in vitro* or *in vivo.*

The term "cytotoxic agent" as used herein refers to a substance that inhibits or prevents the function of cells and/or causes destruction of cells. The term "cytotoxic agent" is intended to include chemotherapeutic agents, enzymes, antibiotics, and toxins such as small molecule toxins or enzymatically active toxins of bacterial, fungal, plant or animal origin, including fragments and/or variants thereof, and the various antitumor or anticancer agents disclosed below. In some embodiments, the cytotoxic agent is a taxoid, vincas, taxanes, a maytansinoid or maytansinoid analog such as DM1 or DM4, a small drug, a tomaymycin or pyrrolobenzodiazepine derivative, a cryptophycin derivative, a leptomycin derivative, an auristatin or dolastatin analog, a prodrug, topoisomerase II inhibitors, a DNA alkylating agent, an anti-tubulin agent, a CC-1065 or CC-1065 analog.

The term "radioactive isotope" is intended to include radioactive isotopes suitable for treating cancer, such as At²¹¹, Bi²¹², Er¹⁶⁹, I¹³¹, I¹²⁵, Y⁹⁰, In¹¹¹, P³², Re¹⁸⁶, Re¹⁸⁸, Sm¹⁵³, Sr⁸⁹, and radioactive isotopes of Lu. Such radioisotopes generally emit mainly beta-radiation. In an embodiment the radioactive isotope is alpha-emitter isotope, more precisely Thorium 227 which emits alpha-radiation.

A "PK modifying moiety" herein refers to a moiety that modifies the pharmacokinetics (PK) of the bispecific antigen binding protein of the invention. Accordingly, the moiety modifies in particular the in vivo half-life and distribution of the bispecific antigen binding protein of the invention. In a preferred embodiment, the PK modifying moiety increases the half-life of the bispecific antigen binding protein. Examples of PK modifying moieties include, but are not limited to, PEG (Dozier et al., (2015) Int J Mol Sci. Oct 28;16(10):25831-64 and Jevsevar et al., (2010) Biotechnol J.Jan;5(1):113-28), PASylation (Schlapschy et al., (2013) Protein Eng Des Sel. Aug;26(8):489-501), albumin (Dennis et al., (2002) J Biol Chem. Sep 20;277(38):35035-43), the F_{c}-part of an antibody and/or unstructured polypeptides (Schellenberger et al., (2009) Nat Biotechnol. Dec; 27(12):1186-90).

### Bispecific antigen binding proteins

The inventors of the present invention humanized the mouse monoclonal antibody anti-CD3 antibody UCHT1 as disclosed in example 1 and obtained the humanized monoclonal antibody UCHT1 (V17), disclosed for reference. The resulting humanized monoclonal antibody UCHT1 (V17) has an increased stability, and/or an increased solubility as compared to UCHT1 (V9) already known in the art.

The inventors then demonstrated in example 2 in a proof of principle experiment, that when using variable domains of a T Cell-recruiting antibody having a moderate affinity for its target (such as BMA31 targeting TCRαβ) in combination with maturated TCR variable domains, then the resulting bispecific antigen binding protein has a much wider safety window than an antigen binding protein using a high affinity anti-CD3 antibody (such as UCHT1(V17)) in connection with the same TCR variable domains.

These new antigen binding proteins, in particular, in the form of TCER^{®} molecules, show a high cytotoxicity against tumor cells. For example, for new antigen binding proteins in the form of TCER^{®} molecules, the half maximal effective concentration (EC₅₀) against NCI-H1755, Hs695T cells and U2OS is in the range of 1 pM to 100 pM, more particularly, between 1 pM and 20 pM for cells and the EC₅₀ of the antigen binding proteins of the present invention for diseased cells is thus more than 1000-fold lower in comparison to the EC₅₀ obtained for cells of normal tissue cells, such as tumor cell line Hs695T versus primary cells, demonstrating high safety.

The inventors therefore created moderate affinity variants (V20, V21, V23, V17opt, V20opt, V21opt and V23opt) of the high affinity anti-CD3 antibody UCHT1(V17) thus, creating a variety of T cell-recruiting variable domains suitable to be used in combination with TCR variable domains to obtain bispecific antibodies with a beneficial safety window.

Accordingly, disclosed for reference is a bispecific antigen binding protein comprising at least two antigen binding sites (A and B), wherein the antigen binding site A binds to CD3, preferably the CD3ε/δ-complex, and wherein the antigen binding site B binds to a target antigenic (TA) peptide/MHC complex, preferably TAA antigenic peptide/MHC complex, and wherein the antigen binding site A comprises a heavy chain variable domain (V_{H}) and a light chain variable domain (V_{L}), and
a) wherein said V_{L} comprises three complementary determining regions (CDRs) CDRL1, CDRL2 and CDRL3, wherein
   - CDRL1 comprises or consists of the amino acid sequence 'RASQDIRNYLN' of SEQ ID NO: 1,
   - CDRL2 comprises or consists of the amino acid sequence 'YTSRLHS' of SEQ ID NO: 2, and
   - CDRL3 comprises or consists of the amino acid sequence 'QQGQTLPWT' of SEQ ID NO: 3, and
b) wherein said V_{H} comprises three complementary determining regions (CDRs) CDRH1, CDRH2 and CDRH3, wherein
   - CDRH1 comprises or consists of the amino acid sequence 'X₁YTMN' of SEQ ID NO: 4, wherein X₁ is G or E, preferably G,
   - CDRH2 comprises or consists of the amino acid sequence of 'LINPX₂X₃GVX₄TYAQKX₅QX₆' SEQ ID NO: 5, wherein X₂ is any amino acid, preferably Q, Y or E, more preferably Q or Y, such as Q, and X₃ is any amino acid, preferably R, K or E, more preferably R or K, such as K, and X₄ is any amino acid, preferably S or T, more preferably S, and X₅ is any amino acid, preferably F or V, more preferably F and X₆ is any amino acid, preferably G or D, more preferably D and
   - CDRH3 comprises or consists of the amino acid sequence 'SGYYGX₇SWYFDV' of SEQ ID NO: 6, wherein X₇ is any amino acid, preferably E or D, more preferably D.

For reference, X₁ of CDRH1 may be E, when CDRH2 comprises or consists of the amino acid sequence 'LINPYKGVSTYAQKFQD' of SEQ ID NO: 7 and CDRH3 comprises or consists of the amino acid sequence 'SGYYGDSDWYFDV' of SEQ ID NO: 8.

For reference, said V_{H} may comprise CDRH1 according to SEQ ID NO: 4, CDRH2 according to SEQ ID NO: 5 and CDRH3 according to SEQ ID NO: 7 with the proviso that CDRH1 does not comprise or consist of SEQ ID NO: 133, CDRH2 does not comprise or consist of SEQ ID NO: 7 and CDRH3 does not comprise or consist of of SEQ ID NO: 8.

Disclosed for reference is a bispecific antigen binding protein comprising at least two antigen binding sites (A and B), wherein the antigen binding site A binds to CD3, preferably the CD3ε/δ-complex, and wherein the antigen binding site B binds to a target antigenic (TA) peptide/MHC complex, preferably TAA antigenic peptide/MHC complex, and wherein the antigen binding site A comprises a heavy chain variable domain (V_{H}) and a light chain variable domain (V_{L}), and
a) wherein said V_{L} comprises three complementary determining regions (CDRs) CDRL1, CDRL2 and CDRL3, wherein
   - CDRL1 comprises or consists of the amino acid sequence 'RASQDIRNYLN' of SEQ ID NO: 1,
   - CDRL2 comprises or consists of the amino acid sequence 'YTSRLHS' of SEQ ID NO: 2, and
   - CDRL3 comprises or consists of the amino acid sequence 'QQGQTLPWT' of SEQ ID NO: 3, and
b) wherein said V_{H} comprises three complementary determining regions (CDRs) CDRH1, CDRH2 and CDRH3, wherein
   - CDRH1 comprises or consists of the amino acid sequence 'GYTMN' of SEQ ID NO: 133 or 'EYTMN' of SEQ ID NO: 134, preferably GYTMN of SEQ ID NO: 133, or optionally an amino acid sequence differing from SEQ ID NO: 133 or 134 by at least one amino acid substitution, preferably by one or two amino acid substitutions, or only one amino acid substitution, wherein preferably an amino acid sequence differing from SEQ ID NO: 133 or 134 comprises 31G or 31E,
   - CDRH2 comprises or consists of the amino acid sequence selected from the group consisting of the amino acid sequence of SEQ ID NO: 135 to 142, or optionally an amino acid sequence differing from SEQ ID NO: 133 or 134 by at least one amino acid substitution, preferably by one, two, three or four amino acid substitutions, preferably by one or two amino acid substitutions, or only one amino acid substitution, wherein preferably an amino acid sequence differing from SEQ ID NO: 133 or 134 comprises the amino acid 61A, and optionally at least one of the amino acids 54Q, 54E or 54Y, 55R or 55E, 58S or 58T, 64F or 64V, 65Q, 66D or 66G, preferably 66D, and
   - CDRH3 comprises or consists of the amino acid sequence of SEQ ID NO: 8 or 144, or optionally an amino acid sequence differing from SEQ ID NO: 8 or 144 by at least one amino acid substitution, preferably by one, two, three or four amino acid substitutions, preferably by one or two amino acid substitutions, or only one amino acid substitution, wherein preferably an amino acid sequence differing from SEQ ID NO: 8 or 134 comprises the amino acid 104E

Antigen binding proteins are disclosed for reference comprising variants of the CDR amino acid sequences that are disclosed herein, typically variants of CDRL1, CDRL2, CDRL3, CDRH1, CDRH2 and/or CDRH3, such variants may comprise least one, such as four, three, two, or one, preferably one, two or three amino acid substitutions wherein the preferred number of amino acid substitutions preferably depends on the length of the respective CDR.

For reference, the CDRL1 may comprise or consist of an amino acid sequence differing from the herein disclosed CDRL1 amino acid sequences by at least one amino acid substitution, preferably by one, two, three or four amino acid substitutions, preferably by one, two or three amino acid substitutions, preferably by one or two amino acid substitutions, such as one amino acid substitution, wherein said amino acid substitution is preferably at position 27, 28, 30 and 31.

For reference, the CDRL2 may comprise or consist of an amino acid sequence differing from the herein disclosed CDRL2 amino acid sequences by at least one amino acid substitution, preferably by one, two or three amino acid substitutions, preferably by one or two amino acid substitutions, such as one amino acid substitution, wherein said amino acid substitution is preferably at position 51, 52 and 53.

For reference, the CDRL3 may comprise or consist of an amino acid sequence differing from the herein disclosed CDRL3 amino acid sequences by at least one amino acid substitution, preferably by one, two, three or four amino acid substitutions, preferably by one or two amino acid substitutions, such as one amino acid substitution, wherein said amino acid substitution is preferably at any of the amino acid positions 93, 94 and 95.

Mutations may occur in the CDRs of the heavy chain variable domain of the antigen binding site A.

Accordingly, for reference, the CDRH1 may comprise or consist of an amino acid sequence differing from the herein disclosed CDRH1 amino acid sequences by at least one amino acid substitution, preferably by one or two or three amino acid substitutions, preferably by one amino acid substitutions, wherein preferably said amino acid substitution is at any of the amino acids positions 31 to 35.

For reference, the CDRH2 may comprise or consist of an amino acid sequence differing from the herein disclosed CDRH2 amino acid sequences by at least one amino acid substitution, preferably by one, two, three or four amino acid substitution, preferably by one, two or three amino acid substitutions, preferably by one or two amino acid substitutions, such as one amino acid substitution, wherein said amino acid substitution is preferably at any of the amino acids positions 54, 55 and 57 to 59.

For reference, the CDRH3 may comprise or consist of an amino acid sequence differing from the herein disclosed CDRH3 amino acid sequences by at least one amino acid substitution, preferably by one, two, three or four amino acid substitution, preferably by one or two amino acid substitutions, such as one amino acid substitution, wherein said amino acid substitution is preferably at any of the amino acid positions 105, 107 and 110.

The light chain variable domain and the heavy chain variable domain may further comprise the light and heavy chain framework regions.

Said light chain variable domain may further comprise one or more framework regions, preferably FR1-L, FR2-L, FR3-L and FR4-L, selected from the group consisting of FR1-L, FR2-L, FR3-L and FR4-L, wherein
- FR1-L comprises or consists of the amino acid sequence of 'DIQMTQSPSSLSASVGDRVTITC' of SEQ ID NO: 11 or an amino acid sequence at least 85% identical to SEQ ID NO: 11, wherein an amino acid sequence at least 85% identical to SEQ ID NO: 11 preferably comprises the amino acids 6Q and/or 23C,
- FR2-L comprises or consists of the amino acid sequence of 'WYQQKPGKAPKLLIY' of SEQ ID NO: 12 or 'WYQQKPGKAVKLLIY' of SEQ ID NO: 13, preferably SEQ ID NO: 12 or an amino acid sequence at least 85% identical to SEQ ID NO: 12 or 13, wherein an amino acid sequence at least 85% identical to SEQ ID NO: 12 or 13 preferably comprises the amino acids 35W, 36Y, 38Q, 44P, 46L and/or 49Y,
- FR3-L comprises or consists of the amino acid sequence of 'GVPSRFSGSGSGTDYTLTISSLQPEDIATYFC' of SEQ ID NO: 14 or an amino acid sequence at least 85% identical to SEQ ID NO: 14, wherein an amino acid sequence at least 85% identical to SEQ ID NO: 14 preferably comprises the amino acids 57G, 59P, 62F, 64G, 66G, 71Y, 82D, 86Y, 87F, 88C,
- FR4-L comprises or consists of the amino acid sequence of 'FGQGTKVEIKR' of SEQ ID NO: 15 or an amino acid sequence at least 85% identical to SEQ ID NO: 15, wherein an amino acid sequence at least 85% identical to SEQ ID NO: 15 preferably comprises the amino acid 98F and/or 101G.
wherein VH may further comprise one or more framework regions selected from the group consisting of FR1-H, FR2-H, FR3-H and FR4-H, and wherein
- FR1-H comprises or consists of the amino acid sequence of 'EVQLVQSGAEVKKPGASVKVSCKASGYSFT' of SEQ ID NO: 16 or an amino acid sequence at least 85% identical to SEQ ID NO: 16, wherein an amino acid sequence at least 85% identical to SEQ ID NO: 16 preferably comprises the amino acid 6Q, 14P, 22C, 24A, 26G, 27Y, 28S, 29F and/or 30T and optionally, comprises at least one of the amino acid substitution Q5V, P9A, L11V, V12K, M18V and/or I20V,
- FR2-H comprises or consists of the amino acid sequence of 'WVRQAPGQGLEWMG' of SEQ ID NO: 17 or an amino acid sequence at least 85% identical to SEQ ID NO: 17, wherein an amino acid sequence at least 85% identical to SEQ ID NO: 17 preferably comprises 36W, 37V, 39Q, 45L, 46E and/or 47W optionally, comprises at least one of the amino acid substitution K38R, S40A, H41P, K43Q, N44G,
- FR3-H comprises or consists of the amino acid sequence of 'RVTLTVDKSTSTAYMELSSLRSEDTAVYYCAR' of SEQ ID NO: 18 or an amino acid sequence at least 85% identical to SEQ ID NO: 18, wherein an amino acid sequence at least 85% identical to SEQ ID NO: 18 preferably comprises 70L, 72V, 79A, 90D, 94Y, 95Y, 96C, 97A, and/or 98R, and optionally, comprises at least one of the amino acid substitution K67R, A68V, K74T, S76T, L84S, T87R and/or S91T, and
- FR4-H comprises or consists of the amino acid sequence of 'WGQGTLVTVSS' of SEQ ID NO: 19 or an amino acid sequence at least 85% identical to SEQ ID NO: 19, wherein an amino acid sequence at least 85% identical to SEQ ID NO: 19 preferably comprises 112W, 113G, 115G and optionally, comprises at least one of the amino acid substitution A114Q and/or T117L.

Said light chain variable domain may further comprise one or more framework regions, preferably FR1-L, FR2-L, FR3-L and FR4-L, selected from the group consisting of FR1-L, FR2-L, FR3-L and FR4-L, wherein
- FR1-L comprises or consists of the amino acid sequence of 'DIQMTQSPSSLSASVGDRVTITC' of SEQ ID NO: 11 or an amino acid sequence at least 85% identical to SEQ ID NO: 11, wherein an amino acid sequence at least 85% identical to SEQ ID NO: 11 preferably comprises the amino acids 6Q and/or 23C,
- FR2-L comprises or consists of the amino acid sequence of 'WYQQKPGKAPKLLIY' of SEQ ID NO: 12 or 'WYQQKPGKAVKLLIY' of SEQ ID NO: 13, preferably SEQ ID NO: 12 or an amino acid sequence at least 85% identical to SEQ ID NO: 12 or 13, wherein an amino acid sequence at least 85% identical to SEQ ID NO: 12 or 13 preferably comprises the amino acids 35W, 36Y, 38Q, 44P, 46L and/or 49Y,
- FR3-L comprises or consists of the amino acid sequence of 'GVPSRFSGSGSGTDYTLTISSLQPEDIATYFC' of SEQ ID NO: 14 or an amino acid sequence at least 85% identical to SEQ ID NO: 14, wherein an amino acid sequence at least 85% identical to SEQ ID NO: 14 preferably comprises the amino acids 57G, 59P, 62F, 64G, 66G, 71Y, 82D, 86Y, 87F, 88C,
- FR4-L comprises or consists of the amino acid sequence of 'FGQGTKVEIK' of SEQ ID NO: 285 or an amino acid sequence at least 85% identical to SEQ ID NO: 15, wherein an amino acid sequence at least 85% identical to SEQ ID NO: 15 preferably comprises the amino acid 98F and/or 101G.
wherein VH further comprises one or more framework regions selected from the group consisting of FR1-H, FR2-H, FR3-H and FR4-H, and wherein
- FR1-H comprises or consists of the amino acid sequence of 'EVQLVQSGAEVKKPGASVKVSCKASGYSFT' of SEQ ID NO: 16 or an amino acid sequence at least 85% identical to SEQ ID NO: 16, wherein an amino acid sequence at least 85% identical to SEQ ID NO: 16 preferably comprises the amino acid 6Q, 14P, 22C, 24A, 26G, 27Y, 28S, 29F and/or 30T and optionally, comprises at least one of the amino acid substitution Q5V, P9A, L11V, V12K, M18V and/or I20V,
- FR2-H comprises or consists of the amino acid sequence of 'WVRQAPGQGLEWMG' of SEQ ID NO: 17 or an amino acid sequence at least 85% identical to SEQ ID NO: 17, wherein an amino acid sequence at least 85% identical to SEQ ID NO: 17 preferably comprises 36W, 37V, 39Q, 45L, 46E and/or 47W optionally, comprises at least one of the amino acid substitution K38R, S40A, H41P, K43Q, N44G,
- FR3-H comprises or consists of the amino acid sequence of 'RVTLTVDKSTSTAYMELSSLRSEDTAVYYCAR' of SEQ ID NO: 18 or an amino acid sequence at least 85% identical to SEQ ID NO: 18, wherein an amino acid sequence at least 85% identical to SEQ ID NO: 18 preferably comprises 70L, 72V, 79A, 90D, 94Y, 95Y, 96C, 97A, and/or 98R, and optionally, comprises at least one of the amino acid substitution K67R, A68V, K74T, S76T, L84S, T87R and/or S91T, and
- FR4-H comprises or consists of the amino acid sequence of 'WGQGTLVTVSS' of SEQ ID NO: 19 or an amino acid sequence at least 85% identical to SEQ ID NO: 19, wherein an amino acid sequence at least 85% identical to SEQ ID NO: 19 preferably comprises 112W, 113G, 115G and optionally, comprises at least one of the amino acid substitution A114Q and/or T117L.

Said light chain variable domain may further comprise one or more framework regions, preferably FR1-L, FR2-L, FR3-L and FR4-L, selected from the group consisting of FR1-L, FR2-L, FR3-L and FR4-L, wherein
- FR1-L comprises or consists of the amino acid sequence of 'DIQMTQSPSSLSASVGDRVTITC' of SEQ ID NO: 11 or an amino acid sequence at least 90% identical to SEQ ID NO: 11, wherein an amino acid sequence at least 90% identical to SEQ ID NO: 11 preferably comprises the amino acids 6Q and/or 23C,
- FR2-L comprises or consists of the amino acid sequence of 'WYQQKPGKAPKLLIY' of SEQ ID NO: 12 or 'WYQQKPGKAVKLLIY' of SEQ ID NO: 13, preferably SEQ ID NO: 12 or an amino acid sequence at least 90% identical to SEQ ID NO: 12 or 13, wherein an amino acid sequence at least 90% identical to SEQ ID NO: 12 or 13 preferably comprises the amino acids 35W, 36Y, 38Q, 44P, 46L and/or 49Y,
- FR3-L comprises or consists of the amino acid sequence of 'GVPSRFSGSGSGTDYTLTISSLQPEDIATYFC' of SEQ ID NO: 14 or an amino acid sequence at least 90% identical to SEQ ID NO: 14, wherein an amino acid sequence at least 90% identical to SEQ ID NO: 14 preferably comprises the amino acids 57G, 59P, 62F, 64G, 66G, 71Y, 82D, 86Y, 87F, 88C,
- FR4-L comprises or consists of the amino acid sequence of 'FGQGTKVEIK' of SEQ ID NO: 285 or an amino acid sequence at least 90% identical to SEQ ID NO: 15, wherein an amino acid sequence at least 90% identical to SEQ ID NO: 15 preferably comprises the amino acid 98F and/or 101G.
wherein VH further comprises one or more framework regions selected from the group consisting of FR1-H, FR2-H, FR3-H and FR4-H, and wherein
- FR1-H comprises or consists of the amino acid sequence of 'EVQLVQSGAEVKKPGASVKVSCKASGYSFT' of SEQ ID NO: 16 or an amino acid sequence at least 90% identical to SEQ ID NO: 16, wherein an amino acid sequence at least 90% identical to SEQ ID NO: 16 preferably comprises the amino acid 6Q, 14P, 22C, 24A, 26G, 27Y, 28S, 29F and/or 30T and optionally, comprises at least one of the amino acid substitution Q5V, P9A, L11V, V12K, M18V and/or I20V,
- FR2-H comprises or consists of the amino acid sequence of 'WVRQAPGQGLEWMG' of SEQ ID NO: 17 or an amino acid sequence at least 90% identical to SEQ ID NO: 17, wherein an amino acid sequence at least 90% identical to SEQ ID NO: 17 preferably comprises 36W, 37V, 39Q, 45L, 46E and/or 47W optionally, comprises at least one of the amino acid substitution K38R, S40A, H41P, K43Q, N44G,
- FR3-H comprises or consists of the amino acid sequence of 'RVTLTVDKSTSTAYMELSSLRSEDTAVYYCAR' of SEQ ID NO: 18 or an amino acid sequence at least 90% identical to SEQ ID NO: 18, wherein an amino acid sequence at least 90% identical to SEQ ID NO: 18 preferably comprises 70L, 72V, 79A, 90D, 94Y, 95Y, 96C, 97A, and/or 98R, and optionally, comprises at least one of the amino acid substitution K67R, A68V, K74T, S76T, L84S, T87R and/or S91T, and
- FR4-H comprises or consists of the amino acid sequence of 'WGQGTLVTVSS' of SEQ ID NO: 19 or an amino acid sequence at least 90% identical to SEQ ID NO: 19, wherein an amino acid sequence at least 90% identical to SEQ ID NO: 19 preferably comprises 112W, 113G, 115G and optionally, comprises at least one of the amino acid substitution A114Q and/or T117L.

Said light chain variable domain may further comprise one or more framework regions, preferably FR1-L, FR2-L, FR3-L and FR4-L, selected from the group consisting of FR1-L, FR2-L, FR3-L and FR4-L, wherein
- FR1-L comprises or consists of the amino acid sequence of 'DIQMTQSPSSLSASVGDRVTITC' of SEQ ID NO: 11 or an amino acid sequence at least 95% identical to SEQ ID NO: 11, wherein an amino acid sequence at least 95% identical to SEQ ID NO: 11 preferably comprises the amino acids 6Q and/or 23C,
- FR2-L comprises or consists of the amino acid sequence of 'WYQQKPGKAPKLLIY' of SEQ ID NO: 12 or 'WYQQKPGKAVKLLIY' of SEQ ID NO: 13, preferably SEQ ID NO: 12 or an amino acid sequence at least 95% identical to SEQ ID NO: 12 or 13, wherein an amino acid sequence at least 95% identical to SEQ ID NO: 12 or 13 preferably comprises the amino acids 35W, 36Y, 38Q, 44P, 46L and/or 49Y,
- FR3-L comprises or consists of the amino acid sequence of 'GVPSRFSGSGSGTDYTLTISSLQPEDIATYFC' of SEQ ID NO: 14 or an amino acid sequence at least 95% identical to SEQ ID NO: 14, wherein an amino acid sequence at least 95% identical to SEQ ID NO: 14 preferably comprises the amino acids 57G, 59P, 62F, 64G, 66G, 71Y, 82D, 86Y, 87F, 88C,
- FR4-L comprises or consists of the amino acid sequence of 'FGQGTKVEIK' of SEQ ID NO: 285 or an amino acid sequence at least 95% identical to SEQ ID NO: 15, wherein an amino acid sequence at least 95% identical to SEQ ID NO: 15 preferably comprises the amino acid 98F and/or 101G.
wherein VH further comprises one or more framework regions selected from the group consisting of FR1-H, FR2-H, FR3-H and FR4-H, and wherein
- FR1-H comprises or consists of the amino acid sequence of 'EVQLVQSGAEVKKPGASVKVSCKASGYSFT' of SEQ ID NO: 16 or an amino acid sequence at least 95% identical to SEQ ID NO: 16, wherein an amino acid sequence at least 95% identical to SEQ ID NO: 16 preferably comprises the amino acid 6Q, 14P, 22C, 24A, 26G, 27Y, 28S, 29F and/or 30T and optionally, comprises at least one of the amino acid substitution Q5V, P9A, L11V, V12K, M18V and/or I20V,
- FR2-H comprises or consists of the amino acid sequence of 'WVRQAPGQGLEWMG' of SEQ ID NO: 17 or an amino acid sequence at least 95% identical to SEQ ID NO: 17, wherein an amino acid sequence at least 95% identical to SEQ ID NO: 17 preferably comprises 36W, 37V, 39Q, 45L, 46E and/or 47W optionally, comprises at least one of the amino acid substitution K38R, S40A, H41P, K43Q, N44G,
- FR3-H comprises or consists of the amino acid sequence of 'RVTLTVDKSTSTAYMELSSLRSEDTAVYYCAR' of SEQ ID NO: 18 or an amino acid sequence at least 95% identical to SEQ ID NO: 18, wherein an amino acid sequence at least 95% identical to SEQ ID NO: 18 preferably comprises 70L, 72V, 79A, 90D, 94Y, 95Y, 96C, 97A, and/or 98R, and optionally, comprises at least one of the amino acid substitution K67R, A68V, K74T, S76T, L84S, T87R and/or S91T, and
- FR4-H comprises or consists of the amino acid sequence of 'WGQGTLVTVSS' of SEQ ID NO: 19 or an amino acid sequence at least 95% identical to SEQ ID NO: 19, wherein an amino acid sequence at least 95% identical to SEQ ID NO: 19 preferably comprises 112W, 113G, 115G and optionally, comprises at least one of the amino acid substitution A114Q and/or T117L.

The amino acids 35W, 36Y, 46L and 49Y of FR2-L and the amino acid 64G, 71Y of FR3-L were identified by the inventors to be located in the Vernier Zone and are preferably not substituted.

The amino acids 27Y, 28S, 29F and 30T of FR-1H, the amino acid 47W of FR2-H, the amino acid 70L, 72V, 79A and 97A and 98R of FR3-H and 112W of FR4-H were identified by the inventors to be located in the Vernier Zone and are preferably not substituted.

Variants of the antigen binding proteins as described herein are contemplated for reference and explicitly referred to using the wording "at least 85% identical to a reference sequence" as defined herein above in the section definitions. For instance, the sequence FR1-L, FR2-L, FR3-L and FR4-L and FR1-H, FR2-H, FR3-H and FR4-H may differ from the reference sequences SEQ ID NO:11 to SEQ ID NO: 19, as appropriate, by at least one amino acid substitution(s), in particular by at least one conservative amino acid substitution(s) and/or substitution(s) with canonical residues. In particular, the sequences FR1-L, FR2-L, FR3-L and FR4-L and FR1-H, FR2-H, FR3-H and FR4-H of the light and heavy chain variable domain may differ from the reference sequences SEQ ID NO:11 to SEQ ID NO: 19 by conservative amino acid substitution(s), only.

For reference, modifications and changes may be made in the amino acid sequence of the bispecific antigen binding protein, and in the corresponding DNA sequences, respectively, and still result in a functional antigen binding protein or polypeptide with desirable characteristics. Modifications may be made in the heavy and light chain variable domain of antigen binding site A or in the alpha and beta or gamma and delta variable domains of the antigen binding site B, in particular in the framework regions or in the each of the CDRs or in all CDRs comprised in the heavy and light chain variable domains of antigen binding site A or in the framework regions or in the each of the CDRs or in all CDRs comprised in the alpha and beta or gamma and delta variable domains.

The bispecific antigen binding protein may comprise a light chain variable region comprising an FR2-L wherein the amino acid sequence of this FR2-L is at least 85% identical to SEQ ID NO: 12 or 13 and comprises the amino acid 44P. This amino acid 44P (which is found in the human germline sequence Vk1-018) has the advantage of deimmunising the humanized variable domains, since proline is common at this position in the 10 most similar human germlines.

"Deimmunising" herein refers to the reduction of immunogenicity, i.e. the capacity to induce in a subject an immune response. This is performed by substitution of amino acids by the amino acids that are most common in the human germlines and therefore are not recognized as foreign by the immune system.

Accordingly, the antigen binding site A in the context of the bispecific antigen binding proteins disclosed for reference may comprise a heavy chain variable domain (V_{H}) and a light chain variable domain (V_{L}),
wherein said V_{L} comprises or consists of the amino acid sequence of SEQ ID NO: 145 or an amino acid sequence at least 85% identical to the amino acid sequence of SEQ ID NO 145 and wherein said amino acid sequence at least 85% identical to the amino acid sequence of SEQ ID NO 145 comprises preferably the amino acid sequence of CDRL1 of SEQ ID NO: 1, CDRL2 of SEQ ID NO: 2 and CDRL3 of SEQ ID NO: 3, and
wherein said V_{H} comprises or consists of an amino acid sequence selected from the group of amino acid sequences selected from the amino acid sequences of SEQ ID NO: 149 to SEQ ID NO: 160 or an amino acid sequence at least 85% identical to the amino acid sequence selected from the group of amino acid sequences consisting of the amino acid sequences of SEQ ID NO: 149 to SEQ ID NO: 160 and wherein, preferably, said amino acid sequence at least 85% identical to the amino acid sequence of SEQ ID NO: 149 comprises the amino acid sequence of CDRH1 of SEQ ID NO: 133, CDRH2 of SEQ ID NO: 138 and CDRH3 of SEQ ID NO: 8, and
wherein, preferably, said amino acid sequence at least 85% identical to the amino acid sequence of SEQ ID NO: 150 comprises the amino acid sequence of CDRH1 of SEQ ID NO: 133, CDRH2 of SEQ ID NO: 139 and CDRH3 of SEQ ID NO: 8, and
wherein, preferably, said amino acid sequence at least 85% identical to the amino acid sequence of SEQ ID NO: 151 comprises the amino acid sequence of CDRH1 of SEQ ID NO: 134, CDRH2 of SEQ ID NO: 138 and CDRH3 of SEQ ID NO: 8, and
wherein, preferably, said amino acid sequence at least 85% identical to the amino acid sequence of SEQ ID NO: 152 comprises the amino acid sequence of CDRH1 of SEQ ID NO: 133, CDRH2 of SEQ ID NO: 140 and CDRH3 of SEQ ID NO: 8, and
wherein, preferably, said amino acid sequence at least 85% identical to the amino acid sequence of SEQ ID NO: 153 comprises the amino acid sequence of CDRH1 of SEQ ID NO: 133, CDRH2 of SEQ ID NO: 141 and CDRH3 of SEQ ID NO: 8, and
wherein, preferably, said amino acid sequence at least 85% identical to the amino acid sequence of SEQ ID NO: 154 comprises the amino acid sequence of CDRH1 of SEQ ID NO: 133, CDRH2 of SEQ ID NO: 142 and CDRH3 of SEQ ID NO: 8, and
wherein, preferably, said amino acid sequence at least 85% identical to the amino acid sequence of SEQ ID NO: 155 comprises the amino acid sequence of CDRH1 of SEQ ID NO: 134, CDRH2 of SEQ ID NO: 142 and CDRH3 of SEQ ID NO: 8, and
wherein, preferably, said amino acid sequence at least 85% identical to the amino acid sequence of SEQ ID NO: 156 comprises the amino acid sequence of CDRH1 of SEQ ID NO: 133, CDRH2 of SEQ ID NO: 7 and CDRH3 of SEQ ID NO: 144, and
wherein, preferably, said amino acid sequence at least 85% identical to the amino acid sequence of SEQ ID NO: 157 comprises the amino acid sequence of CDRH1 of SEQ ID NO: 133, CDRH2 of SEQ ID NO: 138 and CDRH3 of SEQ ID NO: 144, and
wherein, preferably, said amino acid sequence at least 85% identical to the amino acid sequence of SEQ ID NO: 158 comprises the amino acid sequence of CDRH1 of SEQ ID NO: 134, CDRH2 of SEQ ID NO: 7 and CDRH3 of SEQ ID NO: 8, and
wherein, preferably, said amino acid sequence at least 85% identical to the amino acid sequence of SEQ ID NO: 159 comprises the amino acid sequence of CDRH1 of SEQ ID NO: 134, CDRH2 of SEQ ID NO: 7 and CDRH3 of SEQ ID NO: 144, and
wherein, preferably, said amino acid sequence at least 85% identical to the amino acid sequence of SEQ ID NO: 160 comprises the amino acid sequence of CDRH1 of SEQ ID NO: 134, CDRH2 of SEQ ID NO: 138 and CDRH3 of SEQ ID NO: 144.

Accordingly, the antigen binding site A in the context of the bispecific antigen binding proteins disclosed for reference may comprise a heavy chain variable domain (V_{H}) and a light chain variable domain (V_{L}),
wherein said V_{L} comprises or consists of the amino acid sequence of SEQ ID NO: 286 or an amino acid sequence at least 85% identical to the amino acid sequence of SEQ ID NO 286 and wherein said amino acid sequence at least 85% identical to the amino acid sequence of SEQ ID NO 286 comprises preferably the amino acid sequence of CDRL1 of SEQ ID NO: 1, CDRL2 of SEQ ID NO: 2 and CDRL3 of SEQ ID NO: 3, and
wherein said V_{H} comprises or consists of an amino acid sequence selected from the group of amino acid sequences selected from the amino acid sequences of SEQ ID NO: 149 to SEQ ID NO: 160 or an amino acid sequence at least 85% identical to the amino acid sequence selected from the group of amino acid sequences consisting of the amino acid sequences of SEQ ID NO: 149 to SEQ ID NO: 160 and wherein, preferably, said amino acid sequence at least 85% identical to the amino acid sequence of SEQ ID NO: 149 comprises the amino acid sequence of CDRH1 of SEQ ID NO: 133, CDRH2 of SEQ ID NO: 138 and CDRH3 of SEQ ID NO: 8, and
wherein, preferably, said amino acid sequence at least 85% identical to the amino acid sequence of SEQ ID NO: 150 comprises the amino acid sequence of CDRH1 of SEQ ID NO: 133, CDRH2 of SEQ ID NO: 139 and CDRH3 of SEQ ID NO: 8, and
wherein, preferably, said amino acid sequence at least 85% identical to the amino acid sequence of SEQ ID NO: 151 comprises the amino acid sequence of CDRH1 of SEQ ID NO: 134, CDRH2 of SEQ ID NO: 138 and CDRH3 of SEQ ID NO: 8, and
wherein, preferably, said amino acid sequence at least 85% identical to the amino acid sequence of SEQ ID NO: 152 comprises the amino acid sequence of CDRH1 of SEQ ID NO: 133, CDRH2 of SEQ ID NO: 140 and CDRH3 of SEQ ID NO: 8, and
wherein, preferably, said amino acid sequence at least 85% identical to the amino acid sequence of SEQ ID NO: 153 comprises the amino acid sequence of CDRH1 of SEQ ID NO: 133, CDRH2 of SEQ ID NO: 141 and CDRH3 of SEQ ID NO: 8, and
wherein, preferably, said amino acid sequence at least 85% identical to the amino acid sequence of SEQ ID NO: 154 comprises the amino acid sequence of CDRH1 of SEQ ID NO: 133, CDRH2 of SEQ ID NO: 142 and CDRH3 of SEQ ID NO: 8, and
wherein, preferably, said amino acid sequence at least 85% identical to the amino acid sequence of SEQ ID NO: 155 comprises the amino acid sequence of CDRH1 of SEQ ID NO: 134, CDRH2 of SEQ ID NO: 142 and CDRH3 of SEQ ID NO: 8, and
wherein, preferably, said amino acid sequence at least 85% identical to the amino acid sequence of SEQ ID NO: 156 comprises the amino acid sequence of CDRH1 of SEQ ID NO: 133, CDRH2 of SEQ ID NO: 7 and CDRH3 of SEQ ID NO: 144, and
wherein, preferably, said amino acid sequence at least 85% identical to the amino acid sequence of SEQ ID NO: 157 comprises the amino acid sequence of CDRH1 of SEQ ID NO: 133, CDRH2 of SEQ ID NO: 138 and CDRH3 of SEQ ID NO: 144, and
wherein, preferably, said amino acid sequence at least 85% identical to the amino acid sequence of SEQ ID NO: 158 comprises the amino acid sequence of CDRH1 of SEQ ID NO: 134, CDRH2 of SEQ ID NO: 7 and CDRH3 of SEQ ID NO: 8, and
wherein, preferably, said amino acid sequence at least 85% identical to the amino acid sequence of SEQ ID NO: 159 comprises the amino acid sequence of CDRH1 of SEQ ID NO: 134, CDRH2 of SEQ ID NO: 7 and CDRH3 of SEQ ID NO: 144, and
wherein, preferably, said amino acid sequence at least 85% identical to the amino acid sequence of SEQ ID NO: 160 comprises the amino acid sequence of CDRH1 of SEQ ID NO: 134, CDRH2 of SEQ ID NO: 138 and CDRH3 of SEQ ID NO: 144.

Said V_{L} may comprise or consist of the amino acid sequence of SEQ ID NO: 286 or an amino acid sequence at least 85% identical to the amino acid sequence of SEQ ID NO 286 and wherein said amino acid sequence at least 85% identical to the amino acid sequence of SEQ ID NO 286 comprises preferably the amino acid sequence of CDRL1 of SEQ ID NO: 1, CDRL2 of SEQ ID NO: 2 and CDRL3 of SEQ ID NO: 3, and
wherein said V_{H} comprises or consists of the amino acid sequences of SEQ ID NO: 156 or an amino acid sequence at least 85% identical to the amino acid sequence of SEQ ID NO: 156 and wherein, preferably, said amino acid sequence at least 85% identical to the amino acid sequence of SEQ ID NO: 156 comprises the amino acid sequence of SEQ ID NO: 156 comprises the amino acid sequence of CDRH1 of SEQ ID NO: 133, CDRH2 of SEQ ID NO: 7 and CDRH3 of SEQ ID NO: 144, or
wherein said V_{H} comprises or consists of the amino acid sequences of SEQ ID NO: 149 or an amino acid sequence at least 85% identical to the amino acid sequence of SEQ ID NO: 149 and wherein, preferably, said amino acid sequence at least 85% identical to the amino acid sequence of SEQ ID NO: 149 comprises the amino acid sequence of CDRH1 of SEQ ID NO: 133, CDRH2 of SEQ ID NO: 138 and CDRH3 of SEQ ID NO: 8, or
wherein said V_{H} comprises or consists of the amino acid sequences of SEQ ID NO: 151 or an amino acid sequence at least 85% identical to the amino acid sequence of SEQ ID NO: 151 and wherein, preferably, said amino acid sequence at least 85% identical to the amino acid sequence of SEQ ID NO: 151 comprises the amino acid sequence of CDRH1 of SEQ ID NO: 134, CDRH2 of SEQ ID NO: 138 and CDRH3 of SEQ ID NO: 8.

Said V_{L} may comprise or consist of the amino acid sequence of SEQ ID NO: 286 or an amino acid sequence at least 90% identical to the amino acid sequence of SEQ ID NO 286 and wherein said amino acid sequence at least 90% identical to the amino acid sequence of SEQ ID NO 286 comprises preferably the amino acid sequence of CDRL1 of SEQ ID NO: 1, CDRL2 of SEQ ID NO: 2 and CDRL3 of SEQ ID NO: 3, and
wherein said V_{H} comprises or consists of the amino acid sequences of SEQ ID NO: 156 or an amino acid sequence at least 90% identical to the amino acid sequence of SEQ ID NO: 156 and wherein, preferably, said amino acid sequence at least 90% identical to the amino acid sequence of SEQ ID NO: 156 comprises the amino acid sequence of SEQ ID NO: 156 comprises the amino acid sequence of CDRH1 of SEQ ID NO: 133, CDRH2 of SEQ ID NO: 7 and CDRH3 of SEQ ID NO: 144, or
wherein said V_{H} comprises or consists of the amino acid sequences of SEQ ID NO: 149 or an amino acid sequence at least 90% identical to the amino acid sequence of SEQ ID NO: 149 and wherein, preferably, said amino acid sequence at least 90% identical to the amino acid sequence of SEQ ID NO: 149 comprises the amino acid sequence of CDRH1 of SEQ ID NO: 133, CDRH2 of SEQ ID NO: 138 and CDRH3 of SEQ ID NO: 8, or
wherein said V_{H} comprises or consists of the amino acid sequences of SEQ ID NO: 151 or an amino acid sequence at least 90% identical to the amino acid sequence of SEQ ID NO: 151 and wherein, preferably, said amino acid sequence at least 90% identical to the amino acid sequence of SEQ ID NO: 151 comprises the amino acid sequence of CDRH1 of SEQ ID NO: 134, CDRH2 of SEQ ID NO: 138 and CDRH3 of SEQ ID NO: 8.

Said V_{L} may comprise or consist of the amino acid sequence of SEQ ID NO: 286 or an amino acid sequence at least 95% identical to the amino acid sequence of SEQ ID NO 286 and wherein said amino acid sequence at least 85% identical to the amino acid sequence of SEQ ID NO 286 comprises preferably the amino acid sequence of CDRL1 of SEQ ID NO: 1, CDRL2 of SEQ ID NO: 2 and CDRL3 of SEQ ID NO: 3, and
wherein said V_{H} comprises or consists of the amino acid sequences of SEQ ID NO: 156 or an amino acid sequence at least 95% identical to the amino acid sequence of SEQ ID NO: 156 and wherein, preferably, said amino acid sequence at least95% identical to the amino acid sequence of SEQ ID NO: 156 comprises the amino acid sequence of SEQ ID NO: 156 comprises the amino acid sequence of CDRH1 of SEQ ID NO: 133, CDRH2 of SEQ ID NO: 7 and CDRH3 of SEQ ID NO: 144, or
wherein said V_{H} comprises or consists of the amino acid sequences of SEQ ID NO: 149 or an amino acid sequence at least 95% identical to the amino acid sequence of SEQ ID NO: 149 and wherein, preferably, said amino acid sequence at least 95% identical to the amino acid sequence of SEQ ID NO: 149 comprises the amino acid sequence of CDRH1 of SEQ ID NO: 133, CDRH2 of SEQ ID NO: 138 and CDRH3 of SEQ ID NO: 8, or
wherein said V_{H} comprises or consists of the amino acid sequences of SEQ ID NO: 151 or an amino acid sequence at least 85% identical to the amino acid sequence of SEQ ID NO: 151 and wherein, preferably, said amino acid sequence at least 95% identical to the amino acid sequence of SEQ ID NO: 151 comprises the amino acid sequence of CDRH1 of SEQ ID NO: 134, CDRH2 of SEQ ID NO: 138 and CDRH3 of SEQ ID NO: 8.

In some embodiments, the antigen binding site B of the bispecific antigen binding proteins of the invention comprises or consists of an antibody or a fragment thereof or an alpha chain variable domain (v_{α}) and a beta chain variable domain (v_{β}) or a gamma chain variable domain (v_{γ}) or a delta chain variable domain (v_{δ}). Antibodies and Fragments thereof are as defined herein above in the section "Definitions".

In some embodiments, the antigen binding site B of the bispecific antigen binding proteins of the invention comprises an alpha chain variable domain (v_{α}) and a beta chain variable domain (v_{β}) or a gamma chain variable domain (v_{γ}) or a delta chain variable domain (v_{δ}), preferably a v_{α} and v_{β} domain. Alpha chain variable domain (v_{α}) and a beta chain variable domain (v_{β}) or a gamma chain variable domain (v_{γ}) or a delta chain variable domain (v_{δ}) in the context of the specific TA to which they binds that might be used in the context of the present invention are described in detail in, for example, WO2018172533, WO2018033291, WO2017158103, WO2018104438, WO2018104478, WO2019002444, WO2017158116.

Accordingly, in one embodiment, the v_{α} and v_{β} or v_{γ} and v_{δ} comprise or consist of the amino acid sequence disclosed in WO2018172533, WO2018033291, WO2017158103, WO2018104438, WO2018104478, WO2019002444, WO2017158116 and the v_{α} and v_{β} or v_{γ} and v_{δ} described in the cited prior art bind to the TA peptide, in particular TAA peptide, that is disclosed in the same patent application as cited.

In one embodiment, the bispecific antigen binding protein of the invention comprises a v_{α} and v_{β} domain or v_{γ} and v_{δ} domain, wherein
i) the v_{α} or v_{γ} comprises or consists of the amino acid sequence selected from the group consisting of 
‘EDVEQSLFLSVREGDSVVINCTYTDSSSTYLYWYKQEPGKGLQLLTYIYSSQDSKQD
QRLTVLLNKKDKHLSLRIADTQTGDSAIYFCAEMTSESKIIFGSGTRLSIRP’ SEQ ID NO:
20,
 
‘EDVEQSLFLSVREGDSVVINCTYTDSSSTYLYWYKQEPGKGLQLLTYIYSSQDQKQD
QRLTVLLNKKDKHLSLRIADTQTGDSAIYFCAEMTSESKIIFGSGTRLSIRP’ ‘SEQ ID
NO: 21,
 'EDVEQSLFLSVREGDSVVINCTYTESSSTYLYWYKQEPGKGLQLLTYIYSSQDQKQD QRLTVLLNKKOKHLSLRIAOTQTGOSAIYFCAEMTSESKIIFGSGTRLSIRP' SEQ ID NO: 22 or an amino acid sequence at least 85% identical to the amino acid sequence selected from the group consisting of SEQ ID NO: 20, 21 and 22 and wherein preferably the amino acid sequence at least 85% identical to the amino acid sequence of SEQ ID NO: 20 preferably comprises the amino acid sequence of CDRa1 of SEQ ID NO: 23, CDRa2 of SEQ ID NO: 24 and CDRa3 of SEQ ID NO: 25, and wherein preferably the amino acid sequence at least 85% identical to the amino acid sequence of SEQ ID NO: 21 preferably comprises the amino acid sequence of CDRa1 of SEQ ID NO: 23, CDRa2 of SEQ ID NO: 26 and CDRa3 of SEQ ID NO: 25, and wherein preferably the amino acid sequence at least 85% identical to the amino acid sequence of SEQ ID NO: 22 preferably comprises the amino acid sequence of CDRa1 of SEQ ID NO: 27, CDRa2 of SEQ ID NO: 26 and CDRa3 of SEQ ID NO: 25, and wherein the amino acid of said first variable domain preferably comprises the amino acids 19V and/or 48K, and the v_{β} or v_{δ} comprises or consists of the amino acid sequence 'DAGVIQSPRHEVTEMGQEVTLRCKPIPGHDYLFWYRQTMMRGLELLFYFCYGTPCD DSGMPEDRFSAKMPNASFSTLKIQPSEPRDSAVYFCASRADTGELFFGEGSRLTVL' SEQ ID NO: 30 or an amino acid sequence at least 85% identical to the amino acid sequence consisting of SEQ ID NO 30, and wherein preferably the amino acid sequence at least 85% identical to the amino acid sequence of SEQ ID NO: 30 preferably comprises the amino acid sequence of CDRb1 of SEQ ID NO: 31, CDRb2 of SEQ ID NO: 34 and CDRb3 of SEQ ID NO: 35, respectively, and optionally comprises the amino acid 54F and/or 66C, or
(ii) the v_{α} or v_{γ} comprises or consists of the amino acid sequence SEQ ID NO: 48 or an amino acid sequence at least 85% identical to the amino acid sequence of SEQ ID NO: 48, wherein preferably an amino acid sequence at least 85% identical to the amino acid sequence of SEQ ID NO: 48 comprises the amino acid sequence of CDRa1 of SEQ ID NO: 49, CDRa2 of SEQ ID NO: 50 and CDRa3 of SEQ ID NO: 51 and the v_{β} or v_{δ} comprises or consists of the amino acid sequence of SEQ ID NO: 44 or an amino acid sequence at least 85% identical to SEQ ID NO: 44 wherein preferably said amino acid sequence at least 85% identical to the amino acid sequence of SEQ ID NO: 44 comprises the amino acid sequence of CDRb1 of SEQ ID NO: 45, CDRb2 of SEQ ID NO: 46 and CDRb3 of SEQ ID NO: 47.

In one embodiment, the first variable domain and the second variable domain as herein defined in the context of the antigen binding proteins of the invention may comprise an amino acid substitution at position 44 according to the IMGT numbering. In a preferred embodiment, said amino acid at position 44 is substituted with another suitable amino acid, in order to improve pairing. In particular embodiments, preferably in which said antigen binding protein is a TCR, said amino acid substitution improves for example the pairing of the chains (i.e. pairing of α and β chains or pairing of γ and δ. In a preferred embodiment, one of or both of the amino acids present at position 44 in the first variable domain (v₁44) and the amino acid present at position 44 in the second variable domain (v₂44) are substituted into the amino acid pairs v₁44/v₂44 selected from the group of amino acid pairs consisting of v₁44D/v₂44R, ᵥ₁44R/v₂44D, ᵥ₁44E/v₂44K, v₁44K/v₂44E, v₁44D/v₂44K, v₁44K/v₂44D, v₁44R/v₂44E; v₁44E/v₂44R, v₁44L/v₂44W, v₁44W/v₂44L, v₁44V/v₂44W, v₁44W/v₂44V.

Accordingly, in a further embodiment, the antigen binding protein may further include one of the preferred substitution pairs (v₁44/v₂44) selected from the group consisting of: v₁Q44D/v₂Q44R; v₁Q44R/v₂Q44D; v₁Q44E/v₂Q44K; v₁Q44K/v₂Q44E; v₁Q44D/v₂Q44K; v₁Q44K/v₂Q44D; v₁Q44E/v₂Q44R; v₁Q44R/v₂Q44E; v₁Q44L/v₂Q44W; v₁Q44W/v₂Q44L; v₁Q44V/v₂Q44W; and v₁Q44W/v₂Q44V; v₁W44D/v₂Q44R; v₁W44R/v₂Q44D; v₁W44E/v₂Q44K; v₁W44K/v₂Q44E; v₁W44D/v₂Q44K; v₁W44K/v₂Q44D; v₁W44E/v₂Q44R; v₁W44R/v₂Q44E; v₁W44L/v₂Q44W; v₁W44/v₂Q44L; v₁W44V/v₂Q44W; and v₁W44/v₂Q44V; v₁H44D/v₂Q44R; v₁H44R/v₂Q44D; v₁H44E/v₂Q44K; v₁H44K/v₂Q44E; v₁H44D/v₂Q44K; v₁H44K/v₂Q44D; v₁H44E/v₂Q44R; v₁H44R/v₂Q44E; v₁H44L/v₂Q44W; v₁H44W/v₂Q44L; v₁H44V/v₂Q44W; and v₁H44W/v₂Q44V; v₁K44D/v₂Q44R; v₁K44R/v₂Q44D; v₁K44E/v₂Q44K; v₁K44/v₂Q44E; v₁K44D/v₂Q44K; v₁K44/v₂Q44D; v₁K44E/v₂Q44R; v₁K44R/v₂Q44E; v₁K44L/v₂Q44W; v₁K44W/v₂Q44L; v₁K44V/v₂Q44W; and v₁K44W/v₂Q44V; v₁E44D/v₂Q44R; v₁E44R/v₂Q44D; v₁E44/v₂Q44K; v₁E44K/v₂Q44E; v₁E44D/v₂Q44K; v₁E44K/v₂Q44D; v₁E44/v₂Q44R; v₁E44R/v₂Q44E; v₁E44L/v₂Q44W; v₁E44W/v₂Q44L; v₁E44V/v₂Q44W; and v₁E44W/v₂Q44V; v₁Q44D/v₂R44; v₁Q44R/v₂R44D; v₁Q44E/v₂R44K; v₁Q44K/v₂R44E; v₁Q44D/v₂R44K; v₁Q44K/v₂R44D; v₁Q44E/v₂R44; v₁Q44R/v₂R44E; v₁Q44L/v₂R44W; v₁Q44W/v₂R44L; v₁Q44V/v₂R44W; and v₁Q44W/v₂R44V; v₁W44D/v₂R44; v₁W44R/v₂R44D; v₁W44E/v₂R44K; v₁W44K/v₂R44E; v₁W44D/v₂R44K; v₁W44K/v₂R44D; v₁W44E/v₂R44; v₁W44R/v₂R44E; v₁W44L/v₂R44W; v₁W44/v₂R44L; v₁W44V/v₂R44W; and v₁W44/v₂R44V; v₁H44D/v₂R44; v₁H44R/v₂R44D; v1H44E/v₂R44K; v₁H44K/v₂R44E; v₁H44D/v₂R44K; v₁H44K/v₂R44D; v₁H44E/v₂R44; v₁H44R/v₂R44E; v₁H44L/v₂R44W; v₁H44W/v₂R44L; v₁H44V/v₂R44W; and v₁H44W/v₂R44V; v₁K44D/v₂R44; v₁K44R/v₂R44D; v₁K44E/v₂R44K; v₁K44/v₂R44E; v₁K44D/v₂R44K; v1K44/v₂R44D; v₁K44E/v₂R44; v₁K44R/v₂R44E; v₁K44L/v₂R44W; v₁K44W/v₂R44L; v1K44V/v2R44W; and v₁K44W/v₂R44V; v₁E44D/v₂R44; v₁E44R/v₂R44D; v₁E44/v₂R44K; v₁E44K/v₂R44E; v₁E44D/v₂R44K; v₁E44K/v₂R44D; v₁E44R/v₂R44E; v₁E44L/v₂R44W; v₁E44W/v₂R44L; v₁E44V/v₂R44W; and v₁E44W/v₂R44V; v₁Q44D/v₂K44R; v₁Q44R/v₂K44D; v₁Q44E/v₂44K; v₁Q44K/v₂K44E; v₁Q44D/v₂44K; v₁Q44K/v₂K44D; v₁Q44E/v₂K44R; v₁Q44R/v₂K44E; v₁Q44L/v₂K44W; v₁Q44W/v₂K44L; v₁Q44V/v₂K44W; and v₁Q44W/v₂K44V; v₁W44D/v₂K44R; v₁W44R/v₂K44D; v₁W44E/v₂44K; v₁W44K/v₂K44E; v₁W44D/v₂44K; v₁W44K/v₂K44D; v₁W44E/v₂K44R; v₁W44R/v₂K44E; v₁W44L/v₂K44W; v₁W44/v₂K44L; v₁W44V/v₂K44W; and v₁W44/v₂K44V; v₁H44D/v₂K44R; v₁H44R/v₂K44D; v₁H44E/v₂44K; v₁H44K/v₂K44E; v₁H44D/v₂44K; v₁H44K/v₂K44D; v₁H44E/v₂K44R; v₁H44R/v₂K44E; v₁H44L/v₂K44W; v₁H44W/v₂K44L; v₁H44V/v₂K44W; and v₁H44W/v₂K44V; v₁K44D/v₂K44R; v₁K44R/v₂K44D; v₁K44E/v₂44K; v₁K44/v₂K44E; v₁K44D/v₂44K; v₁K44/v₂K44D; v₁K44E/v₂K44R; v₁K44R/v₂K44E; v₁K44L/v₂K44W; v₁K44W/v₂K44L; v₁K44V/v₂K44W; and v₁K44W/v₂K44V; v₁E44D/v₂K44R; v₁E44R/v₂K44D; v₁E44/v₂44K; v₁E44K/v₂K44E; v₁E44D/v₂44K; v₁E44K/v₂K44D; v₁E44/v₂K44R; v₁E44R/v₂K44E; v₁E44L/v₂K44W; v₁E44W/v₂K44L; v₁E44V/v₂K44W; and v₁E44W/v₂Q44V.

In the above, e.g., "v₁Q44R/v₂Q44D" shall mean, for example, that, in the first variable domain Q44 is substituted by R, while in the second variable domain, Q44 is substituted by D. Additional substitutions and description may be found in U.S. Patent Application No. 2018-0162922.

A bispecific antigen binding protein disclosed for reference may be a bispecific antibody or fragment thereof, a bispecific T cell receptor (TCR) or fragment thereof or a bispecific single chain TCR (scTCR) or a bispecific single-chain antibody.

The bispecific antibody and TCR and the respective fragments are as defined herein above in the section 'Definitions'.

In one embodiment, the antigen binding protein is of human origin, which is understood as being generated from a human antigen locus and therefore comprising human sequences, in particular, human TCR or antibody sequences.

In one embodiment, the light chain variable domain and the heavy chain variable domain are linked together and/or the v_{α} and the v_{β} or the v_{γ} and the v_{δ} domain are linked together, preferably, via a covalent link.

In one embodiment, the bispecific antigen binding protein comprises at least two polypeptides.

In a related embodiment, the light chain variable domain and the heavy chain variable domain are located on the same or on different polypeptides, preferably different polypeptides.

In the same embodiment, the alpha chain variable domain (v_{α}) and the beta chain variable domain (v_{β}) or the gamma chain variable domain (v_{γ}) and a delta chain variable domain (v_{δ}), preferably the v_{α} and v_{β}, are located on the same or different polypeptides.

In one preferred embodiment, the antigen binding protein is a soluble protein.

A "covalent link", "linker sequence" or "polypeptide linker" is as defined herein above in the section definitions under "linker".

In one embodiment the bispecific antigen binding protein of the invention further comprises one or more of the following:
(i) a diagnostic agent;
(ii) a therapeutic agent; or
(iii) PK modifying moiety.

"Diagnostic agent", "therapeutic agent" and "PK modifying moiety" are defined herein above in the section definitions.

In some embodiments, the antigen binding proteins of the present invention are covalently attached, directly or via a cleavable or non-cleavable linker, to the at least one growth inhibitory agent. An antigen binding protein to which such the at least one growth inhibitory agent is attached may also be referred to as a conjugate.

The preparation of such conjugates, for example immunoconjugates, is described in the application WO2004/091668 or Hudecz, F., Methods Mol. Biol. 298: 209-223 (2005) and Kirin et al., Inorg Chem. 44(15): 5405-5415 (2005), and may by the skilled in the art be transferred to the preparation of antigen binding proteins of the present invention to which such a at least one growth inhibitory agent is attached.

"Linker" in the context of the attachment of at least one growth inhibitory agent, means a chemical moiety comprising a covalent bond or a chain of atoms that covalently attaches a polypeptide to a drug moiety.

The conjugates may be prepared by *in vitro* methods. In order to link a drug or prodrug to the antibody, a linking group is used. Suitable linking groups are well known in the art and include disulfide groups, thioether groups, acid labile groups, photolabile groups, peptidase labile groups and esterase labile groups. Conjugation of an antigen binding protein of the invention with cytotoxic agents or growth inhibitory agents may be made using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1-pyrrolidinyl ester (nitro-SPDB), 4-(Pyridin-2-yldisulfanyl)-2-sulfo-butyric acid (sulfo-SPDB), N-succinimidyl (2-pyridyldithio) propionate (SPDP), succinimidyl (N-maleimidomethyl) cyclohexane-1-carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4- dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" facilitating release of the cytotoxic agent or growth inhibitory agent in the cell. For example, an acid-labile linker, a peptidase-sensitive linker, an esterase labile linker, a photolabile linker or a disulfide-containing linker (See e.g. U.S. Patent No. 5,208,020) may be used. The linker may be also a "non-cleavable linker" (for example SMCC linker) that might led to better tolerance in some cases.

Alternatively, a fusion protein comprising the bispecific antigen binding protein of the invention and a cytotoxic or growth inhibitory polypeptide may be made, by recombinant techniques or peptide synthesis. The length of DNA may comprise respective regions encoding the two portions of the conjugate either adjacent one another or separated by a region encoding a linker peptide which does not destroy the desired properties of the conjugate.

The antigen binding proteins of the present invention may also be used in Dependent Enzyme Mediated Prodrug Therapy by conjugating the polypeptide to a prodrug-activating enzyme which converts a prodrug (e.g. a peptidyl chemotherapeutic agent, see WO 81/01145) to an active anti-cancer drug (See, for example, WO 88/07378 and U.S. Patent No. 4,975,278).

In one embodiment the antigen binding protein of the invention further comprises one or more of the following: an enzyme, a cytokine (such as the human IL-2, IL-7 or IL-15), a nanocarrier, or a nucleic acid.

Different bispecific formats are described in the art and are described herein above in the section "Definition" under "formats". Techniques to produce proteins of the different formats are also disclosed in the art cited in the corresponding section and the skilled in the art can thus easily use the variable domains as defined in the context of the present invention in different formats, in particular in the herein disclosed formats. The production of antigen binding proteins, in particular soluble bispecific binding proteins, such as the TCER^{®} are also herein disclosed in the Example section. It will be understood by the skilled in the art that, in case the antigen binding protein comprises two polypeptides, the light and heavy chain variable domains may be, for example, in a parallel orientation and the alpha and beta variable domains may be in a parallel orientation as it is the case in the DVD format, or that the light and heavy chain variable domains may be, for example, in a cross orientation and the alpha and beta variable domains may be in a cross orientation as it is the case for example in the CODV format.

Accordingly, the invention further refers for reference to bispecific antigen binding protein comprising two polypeptide chains that form two antigen binding sites (A and B), wherein a first polypeptide chain has a structure represented by the formula:

V₃-L₁-V₄-L₂-C_{L} [I]

wherein V₃ is a third variable domain; V₄ is a fourth variable domain; L₁ and L₂ are linkers; L₂ may be present or absent; C_{L} is a light chain constant domain or a portion thereof and present or absent;
and wherein a second polypeptide chain has a structure represented by the formula:

   V₅-L₃-V₆-L₄-C_{H1} [II]
wherein V₅ is a fifth variable domain; V₆ is a sixth variable domain; L₃ and L₄ are linkers; L₄ may be present or absent; C_{H1} is a heavy chain constant domain 1 or a portion thereof and is present or absent; and wherein
V₃ is the V_{α} or V_{γ} variable domain and V₅ is the V_{β} or V_{δ} variable domain as defined herein above, and V₄ is a light chain variable domain and V₆ is a heavy chain variable domain or V₄ is a heavy chain variable domain and V₆ is a light chain variable domain, or,
V₃ is the V_{β} or V_{δ} variable domain and V₅ is the V_{α} or V_{γ} variable domain as defined herein above, and V₄ is a light chain variable domain and V₆ is a heavy chain variable domain or V₄ is a heavy chain variable domain and V₆ is a light chain variable domain, or,
V₃ is the V_{α} or V_{γ} variable domain and V₆ is the V_{β} or V_{δ} variable domain as defined herein above, and V₄ is a light chain variable domain and V₅ is a heavy chain variable domain or V₄ is a heavy chain variable domain and V₅ is a light chain variable domain, or
V₃ is the V_{β} or V_{δ} variable domain and V₆ is the V_{α} or V_{γ} variable domain as defined herein above, and V₄ is a light chain variable domain and V₅ is a heavy chain variable domain or V₄ is a heavy chain variable domain and V₅ is a light chain variable domain,
V₄ is the V_{α} or V_{γ} variable domain and V₅ is the V_{β} or V_{δ} variable domain as defined herein above, and V₃ is a light chain variable domain and V₆ is a heavy chain variable domain or V₃ is a heavy chain variable domain and V₆ is a light chain variable domain, or
V₄ is the V_{β} or V_{δ} variable domain and V₅ is the V_{α} or V_{γ} variable domain as defined herein above, and V₃ is a light chain variable domain and V₆ is a heavy chain variable domain or V₃ is a heavy chain variable domain and V₆ is a light chain variable domain,
and wherein the light chain variable domain and the heavy chain variable domain form together the antigen binding site A, and wherein the V_{α} and V_{β} or V_{γ} and V_{δ} variable domain form the antigen binding site B, and wherein the V_{α} or V_{γ} variable domain is preferably V_{α}, and the V_{β} or V_{δ} is preferably V_{β}. The linkers L₁, L₂, L₃, L₄ are defined herein above in the section 'Definitions.' However, in some embodiments some linker lengths might be preferable for a specific format. However, the knowledge concerning linker lengths and their amino acid sequences belongs to the general knowledge of the art, and linkers as well as linker and amino acid sequences for the different formats are part of the state of the art and are disclosed in the here above cited disclosures.

In a preferred embodiment, V₃ is V_{α} as defined herein above and V₆ is V_{β} as defined herein above, and V₄ is a light chain variable domain as defined in the context of the invention and V₅ is a heavy chain variable domain as defined in the context of the invention or

V₃ is V_{α} as defined herein above and V₆ is V_{β} as defined herein above, and V₄ is a heavy chain variable domain as defined in the context of the invention and V₅ is a light chain variable domain as defined in the context of the invention.

In one embodiment, the polypeptide of formula [I] further comprises at the C-terminus the polypeptide of formula [I] a linker (L₅) and a F_{c} domain or portion thereof and/or wherein the polypeptide of formula [II] further comprises at the C-terminus of the polypeptide of formula [II] a linker (L₆) and a F_{c} domain or a portion thereof.

The F_{c}-domain is as defined herein above in the section 'definition'.

The antigen binding protein may comprise two polypeptide chains that form two antigen-binding sites (A and B),
wherein one polypeptide chain has a structure represented by the formula [III]:

   V₃-L₁-V₄-L₂-C_{L}-L₅-F_{c1} [III]
and one polypeptide chain has a structure represented by the formula [IV]:

   V₅-L₃-V₆-L₄-C_{H1}-L₆-F_{c2} [IV]
wherein V₃, L₁, V₄, L₂, C_{L}, V₅, L₃, V₆, L₄, C_{H1}, are as defined herein above, and wherein L₅ and L₆ are linkers that are present or absent and wherein F_{c1}, and F_{c2} are F_{c}-domains and wherein F_{c1} and F_{c2} are identical or different, preferably different. The F_{c}-domain is as defined herein above in the section 'definition'.

In one embodiment, F_{c1} comprises or consists of the amino acid sequence SEQ ID NO: 132 (hole) and F_{c2} comprises or consists of the amino acid sequence SEQ ID NO: 131 (knob), or vice versa,
more preferably F_{c1} comprises or consists of the amino acid sequence SEQ ID NO: 132, when V₄ or V₃ is a heavy chain variable domain and, accordingly, F_{c2} comprises or consists of the amino acid sequence SEQ ID NO: 131, when V₅ or V₆ is a light chain variable domain, or
F_{c1} comprises or consists of the amino acid sequence SEQ ID NO: 131, when V₄ or V₃ is a light chain variable domain and, accordingly, F_{c2} comprises or consists of the amino acid sequence SEQ ID NO: 132, when V₅ or V₆ is a heavy chain variable domain.

As it can be seen from the examples, the inventor of the present invention demonstrated as a proof of principle the use of the low affinity recruiters (antigen bindings site A) in combination with maturated TCR variable domains in a TCER^{®} format (Example 2).

Accordingly, the antigen binding protein may comprise two polypeptide chains that form two antigen-binding sites (A and B), wherein one polypeptide chain has a structure represented by the formula [III]:

V₃-L₁-V₄-L₂-C_{L}-L₅-F_{c1} [III]

and one polypeptide chain has a structure represented by the formula [IV]:

   V₅-L₃-V₆-L₄-CH₁-L₆-F_{c2} [IV]
wherein L₂, C_{L}, L₅ and L₄, C_{H1}, L₆ are absent, and
wherein V₃, L₁, V₄, V₅, L₃, V₆ are as defined herein above,
preferably, V₃ is the V_{α} or V_{γ} domain and V₆ is the V_{β} or V_{δ} domain as defined in the context of the present invention, and V₄ is a light chain variable domain and V₅ is a heavy chain variable domain as defined in the context of the invention, or
preferably, V₃ is the V_{α} or V_{γ} variable domain and V₆ is the V_{β} or V_{δ} domain as defined in the context of the present invention, and V₄ is a heavy chain variable domain and V₅ is a light chain variable domain as defined in the context of the invention, and
preferably, L₁ and L₃ comprise or consist of the amino acid sequence 'GGGSGGGG' of (SEQ ID NO:118), and
preferably F_{c1} comprises or consists of the amino acid sequence SEQ ID NO: 132 and F_{c2} comprises or consists of the amino acid sequence SEQ ID NO: 131, or vice versa,
more preferably F_{c1} comprises or consists of the amino acid sequence SEQ ID NO: 132, when V₄ is a heavy chain variable domain and, accordingly, F_{c2} comprises or consists of the amino acid sequence SEQ ID NO: 131, when V₅ is a light chain variable domain, or
more preferably F_{c1} comprises or consists of the amino acid sequence SEQ ID NO: 131, when V₄ is a light chain variable domain and, accordingly, F_{c2} comprises or consists of the amino acid sequence SEQ ID NO: 132, when V₅ is a heavy chain variable domain, and the light chain variable domain and the heavy chain variable domain form together one antigen binding site A which binds to CD3,
and wherein the V_{α} and V_{β} or V_{γ} and V_{δ} variable domains form one antigen binding site B that specifically binds to the TA antigenic peptide/MHC complex, preferably TAA antigenic peptide/MHC complex, as defined in the context of the present invention.

The antigen binding protein of this embodiment may be also be referred to as TCER^{®}.

"TCER^{®}" are bispecific T cell Receptors (TCR) are soluble antigen binding proteins comprising two antigen binding domains, a heavy and light chain variable domain binding CD3 as defined in the context of the invention and a V_{α} and V_{β} or V_{γ} and V_{δ} domain as defined in the context of the invention.

Disclosed for reference is an antigen binding protein comprising a first polypeptide of formula V₃-L₁-V₄-L₂-C_{L}-L₅-F_{c1} [III] comprising or consisting of the amino acid sequence of SEQ ID NO: 165 to 167 and the second polypeptide of formula V₅-L₃-V₆-L₄-CH₁-L₆-F_{c2} [IV] comprising or consisting of the amino acid sequence of SEQ ID NO: 163 or 164.

It may be also desirable to modify the antigen binding protein of the present invention with respect to effector function, e.g. so as to enhance or reduce antigen-dependent cell-mediated cytotoxicity (ADCC) and/or complement dependent cytotoxicity (CDC) of the antigen binding protein. This may be achieved by introducing one or more amino acid substitutions in an F_{c} region of the antigen binding protein, herein also called F_{c} -variants in the context with the antigen binding proteins of the present invention. Alternatively or additionally, cysteine residue(s) may be introduced in the F_{c} region, thereby allowing inter-chain disulfide bond formation in this region. The homodimeric antigen binding protein thus generated may have improved or reduced internalization capability and/or increased complement-mediated cell killing and/or antibody-dependent cellular cytotoxicity (ADCC) (Caron PC. et al. 1992; and Shopes B. 1992).

Another type of amino acid modification of the antigen binding protein of the invention may be useful for altering the original glycosylation pattern of the antigen binding protein, i.e. by deleting one or more carbohydrate moieties found in the antigen binding protein, and/or adding one or more glycosylation sites that are not present in the antigen binding protein. The presence of either of the tripeptide sequences asparagine-X-serine, and asparagine-X-threonine, where X is any amino acid except proline, creates a potential glycosylation site. Addition or deletion of glycosylation sites to the antigen binding protein is conveniently accomplished by altering the amino acid sequence such that it contains one or more of the above-described tripeptide sequences (for N-linked glycosylation sites).

Another type of modification involves the removal of sequences identified, either in silico or experimentally, as potentially resulting in degradation products or heterogeneity of antigen binding protein preparations. As examples, deamidation of asparagine and glutamine residues can occur depending on factors such as pH and surface exposure. Asparagine residues are particularly susceptible to deamidation, primarily when present in the sequence Asn-Gly, and to a lesser extent in other dipeptide sequences such as Asn-Ala. When such a deamidation site, in particular Asn-Gly, is present in an antigen binding protein of the invention, it may therefore be desirable to remove the site, typically by conservative substitution to remove one of the implicated residues. Such substitutions in a sequence to remove one or more of the implicated residues are also intended to be encompassed by the present invention.

Another type of covalent modification involves chemically or enzymatically coupling glycosides to the antigen binding protein. These procedures are advantageous in that they do not require production of antigen binding protein in a host cell that has glycosylation capabilities for N-or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, orhydroxyproline, (e) aromatic residues such as those of phenylalanine, tyrosine, or tryptophan, or (f) the amide group of glutamine. For example, such methods are described in WO 87/05330.

Removal of any carbohydrate moieties present on the antigen binding protein may be accomplished chemically or enzymatically. Chemical deglycosylation requires exposure of the antigen binding protein to the compound trifluoromethanesulfonic acid, or an equivalent compound. This treatment results in the cleavage of most or all sugars except the linking sugar (N-acetylglucosamine or N-acetylgalactosamine), while leaving the antigen binding protein intact. Chemical deglycosylation is described by Sojahr H. et al. (1987) and by Edge, AS. et al. (1981). Enzymatic cleavage of carbohydrate moieties on antibodies can be achieved by the use of a variety of endo-and exo-glycosidases as described by Thotakura, NR. et al. (1987).

Another type of covalent modification of the antigen binding protein comprises linking the antigen binding protein to one of a variety of non-proteinaceous polymers, e.g., polyethylene glycol, polypropylene glycol, or polyoxyalkylenes, in the manner set forth in US Patent Nos. 4,640, 835; 4,496, 689; 4,301, 144; 4,670, 417; 4,791, 192 or 4,179,337.

The invention also includes particles displaying antigen binding proteins of the invention and the inclusion of said particles within a library of particles. Such particles include but are not limited to phage, yeast ribosomes, or mammalian cells. Method of producing such particles and libraries are known in the art (for example see WO2004/044004; WO01/48145, Chervin et al. (2008) J. Immuno. Methods 339.2: 175-184).

As disclosed herein, the antigen binding proteins binds with the antigen binding site A to CD3 and with the antigen binding site B to a target antigenic (TA) peptide/MHC complex. The CD3 molecule is typically present on the surface of a CD3 presenting cell, such as an effector cell, preferably a T cell. The target antigenic (TA) peptide/MHC complex is typically present on the surface of a target antigenic (TA) peptide/MHC complex presenting cell, such as a diseased cell, such as a cancer cell. The binding of the antigen binding protein to CD3 and the target antigenic (TA) peptide/MHC complex brings the effector cell and the target cell into proximity of one another and the binding of the bispecific antigen binding protein thus may elicit an immune response upon binding. Accordingly, the antigen binding protein of the present invention induces an immune response in effector cells.

Accordingly, in one embodiment, the antigen binding protein of the present invention, induces an immune response in the CD3 presenting cell, such as the effector cell, such as the T cell or the NK cell, preferably, wherein the immune response is characterized by an increase in interferon (IFN) γ levels. Accordingly, in one example the immune response might be characterized by an EC₅₀ value that is preferably determined in an IFN-gamma release assay.

In the context of the present invention, the bispecific antigen binding protein of the invention binds to CD3 with a K_{D}(A) and the antigen binding site B binds to the TA antigenic peptide C (TA-C)/MHC complex, preferably TAA antigenic peptide C (TAA-C)/MHC complex, with a K_{D}(C) and the ratio of K_{D}(A)/K_{D}(C) is more than 1, more than 4, more than 6, more than 8, more than 10, more than 15, more than 20, more than 25, more than 30, more than 40, more than 50, such as between 1 and 150, between 4 and 140, between 6 and 100, between 8 and 100, between 10 and 100, preferably between 10 and 100.

The terms "affinity" and "K_{D}" are defined herein above in the section "Definitions". Methods to measure the affinity, such as the K_{D} are known to the skilled in the art and include, for example, surface Plasmon resonance and biolayer interferometry. As it is known to the skilled in the art the experimental conditions used for those experiments, such as buffer used, concentration of the protein or temperature, may influence the results.

Accordingly, in one example, the bispecific antigen binding proteins of the invention are expressed, for instance, as soluble TCER^{®} described herein above and are analyzed for their binding affinity towards the complex HLA-A*02/MAG-003 monomers. Typically, measurements are performed, for instance, on an Octet RED384 system using, typically, settings recommended by the manufacturer. Briefly, binding kinetics were, typically, measured at 30°C and, for instance, 1000 rpm shake speed using, for example, PBS, 0.05% Tween-20, 0.1% BSA as buffer. Peptide-HLA-A:02 complexes were loaded onto biosensors, such as HIS1K, prior to analyzing the antigen binding proteins, in particular, the TCER^{®}.The same antigen binding protein is then typically further analyzed for its binding affinity towards CD3. Accordingly, the measurement was performed as described herein above for CD3 binding.

The antigen binding site A may bind to CD3 with a K_{D}(A) that is ≥ 3 nM, ≥ 5 nM, ≥8 nM, ≥10 nM, ≥12 nM, ≥14 nM, ≥16 nM, ≥18 nM, ≥20 nM, ≥25 nM, ≥30 nM, ≥35 nM, ≥40 nM, ≥45 nM, and , ≤1000 nM, ≤800 nM, ≤600 nM, ≤500 nM, ≤400 nM, such as between 3 nM and 1000 nM, 3 nM and 600 nM, between 5 nM and 600 nM, 10 nM and 600 nM, 12 nM and 600 nM, 14 nM and 600 nM, 16 nM and 600 nM, 18 nM and 600 nM, 20 nM and 600 nM, preferably 5 nM and 100 nM, preferably, as determined using Surface plasmon resonance (SPR) or Biolayer Interferometry (BLI), preferably Biolayer Interferometry (BLI).

In some embodiments the antigen binding site B binds to the TA antigenic peptide C(TA-C)/MHC complex, preferably TAA antigenic peptide C(TAA-C)/MHC complex, with a K_{D}(C) which is ≤ 100 µM, ≤ 1 µM ≤ 100 nM, ≤ 50 nM, ≤ 10 nM, for instance 0.01 nM to 150 nM, 0.05 nM to 150 nM, 0.1 nM to 150 nM, 0.1 nM to 100 nM, 0.1 nM to 50 nM, 0.1 nM to 10 nM, 0.5 nM to 10 nM, 0.5 nM to 5 nM, 0.1 nM to 5 nM preferably 0.5 nM to 5 nM, as determined using Surface plasmon resonance (SPR) or Biolayer Interferometry (BLI), preferably Biolayer Interferometry (BLI).

In one example, the antigen binding protein, binds to the MAGE-A antigenic peptide comprising or consisting of the amino acid sequence 'KVLEHVVRV' of SEQ ID NO: 10 preferably HLA-A*02, or with a K_{D} which is is ≤ 100 nM, ≤ 50 nM, ≤ 10 nM, ≤ 1nM, ≤ 1 nM, for instance 10pM to 100 nM, 10 pM to 50 nM, 10 pM to 10 nM, in particular 50pM to 100 nM, 100 pM to 50 nM, 100 pM to 10 nM, 500 pM to 10 nM, preferably 500 pM to 10 nM as determined using Surface plasmon resonance (SPR) or Biolayer Interferometry (BLI), preferably Biolayer Interferometry (BLI).

In one example, the antigen binding protein, binds to the PRAME peptide comprising or consisting of the amino acid sequence of SEQ ID NO: 9 /MHC complex, with a K_{D} which is ≤ 100 nM, ≤ 50 nM, ≤ 10 nM, ≤ 1 nM, ≤ 1 nM, for instance 10 pM to 100 nM, 10 pM to 150 nM, 10 pM to 100 nM, in particular 50 pM to 100 nM, 100 pM to 100 nM, 100 pM to 50 nM, preferably, as determined using Surface plasmon resonance (SPR) or Biolayer Interferometry (BLI), preferably Biolayer Interferometry (BLI).

In one embodiment, the bispecific antigen binding protein has an EC₅₀ for TA-C/MHC presenting cells, preferably TAA-C/MHC presenting cells, that is ≥5 fold, ≥10 fold, ≥20 fold, ≥50 fold, ≥100 fold, ≥ 500 fold, ≥ 1000 fold lower than the EC₅₀ value for normal tissue cells, wherein the EC₅₀ is preferably determined with respect to induced cytotoxicity.

The "TA-C/MHC presenting cell" or "TA presenting cell" herein refers to a cell that presents on its surface the TA antigenic peptide, such as the particular TA-C antigenic peptide, in complex with a MHC protein, wherein the copy number of said TA peptide/MHC complex that is present on the cell surface can typically be determined with methods known to the skilled in the art. In one embodiment, the TA/MHC presenting cell is a TA-C/MHC presenting cell, preferably a TAA/MHC presenting cell. In some examples, the TA antigenic peptide is a viral or bacterial peptide, in such examples, the TA/MHC presenting cell is typically a diseased cell or infected cell, wherein said diseased cell is infected with the respective virus or bacteria. In some examples, the TA antigenic peptide is a TAA antigenic peptide, and in said example, the TAA/MHC presenting cell is typically a cancer cell.

In one embodiment, the TA/MHC presenting cell, preferably the TAA/MHC presenting cell, such as a TAA/MHC presenting cancer cell, has a TA/MHC copy number, or TAA/MHC copy number, respectively, of more than 50, more than 100, more than 150, more than 200, more than 300, more than 600, more than 800, more than 1000, more than 1500, more than 2000, preferably a TAA/MHC copy number of 50 to 5000.

"Copy number" herein refers to the number of TA antigenic peptide/MHC complex that are present on the cell surface of a cell, such as a TA/MHC presenting cell, for example a TAA/MHC presenting cell, such as a cancer cell, or a normal healthy cell.

Such copy numbers depend, for example, on the specific TA and on the cell type and can be detected with methods that are known to the skilled in the art, such as FACS analysis, Mass spectrometry (MS) and RNA-Sequencing, preferably Mass spectrometry (MS) and RNA-Sequencing

"Healthy cells" may also be referred to as "normal cells" and herein refer to cells that are no cancer cells, preferably healthy cells herein refers to cells of the tissue surrounding the TA presenting cells. Preferably, when the TA is a viral or bacterial antigenic peptide, the healthy cell is preferably not diseased, i.e does not suffer from a bacterial or viral infection of the respective virus or bacteria. However, in some cases also healthy cells might express and present at their surface the TA peptide/MHC complex, for example the TAA peptide/MHC complex, such as TAA-C/MHC complex. Typically in healthy cells in the context of the present invention, as it will be understood by the skilled in the art, the TA peptide/MHC complex is present in smaller amounts (copy numbers) than in a TA presenting cell, such as a cancer cell.

Accordingly, in one embodiment, the healthy cells have a TA/MHC copy number, preferably a TAA/MHC copy number, such as a TAA-C/MHC copy number, of less than 5000, of less than 1000, of less than 500, of less than 100, of less than 50, less than 20, less than 10, preferably less than 10 TAA/MHC copy number, preferably a TAA/MHC copy number between 0 and 10, such as 0 to 5.

Healthy cells are preferably selected from the group consisting of Astrocytes, GABANeurons, Cardiomyocytes, Cardiac Microvascular Endothelial cells, Chondrocytes, Coronary Artery Endothelial cells, Dermal Microvascular Endothelial cells, Mesenchymal stem cells, Nasal Epithelial cells, Peripheral Blood Mococular cells, Pulmonary Artery Smooth Muscle cells, preferably GABANeurons, Cardiomyocytes, Cardiac Microvascular Endothelial cells, chondrocytes, Coronry Artery Endothelial cells, Nasal Epithelial cells, Peripheral Blood Mococular cells, Pulmonary Artery Smooth Muscle cells.

In one embodiment, the antigen binding protein has an EC₅₀ for TA/MHC complex presenting cell, such as TA-C/MHC complex presenting cell, for example a MAGE-A/MHC complex presenting cells, that is ≥ 1000, ≥ 15000, ≥ 9000 fold lower than the EC₅₀ value for healthy cells, preferably healthy cells selected from the group consisting of Astrocytes, GABANeurons, Cardiomyocytes, Cardiac Microvascular Endothelial cells, chondrocytes, Coronary Artery Endothelial cells, Derman Microvascular Endothelial cells, Mesenchymal stem cells, Nasal Epithelial cells, Peripheral Blood Mononuclear cells, Pulmonary Artery Smooth Muscle cells, preferably GABANeurons, Cardiomyocytes, Cardiac Microvascular Endothelial cells, chondrocytes, Coronary Artery Endothelial cells, Nasal Epithelial cells, Peripheral Blood Mononuclear cells, Pulmonary Artery Smooth Muscle cells.

The bispecific antigen binding proteins of the present invention have a high safety profile.

"Safety profile" herein refers to the capacity to distinguish tumor cells from normal healthy tissue cells or from similar peptide presenting cells. In the art, the safety profile is described using a safety window.

The "safety window" or "therapeutic window" herein refers to a factor that compares the half maximal concentration of a compound that is required for inducing 100% cytotoxicity in a tumor cell line in comparison to the half maximal concentration of a compound that is required for inducing 100% cytotoxicity normal tissue cells. If, for an antigen binding protein of interest, the EC₅₀ determined for a tumor cell line is 1 pM and the EC₅₀ value determined for, for instance, primary cells, is 1000 pM, then the safety window is 1000 since the EC₅₀ for the tumor cell line is 1000 times smaller than the EC₅₀ for the primary cells.

In one embodiment, the bispecific antigen binding protein of the invention has an EC₅₀ for TA/MHC complex presenting cells, preferably TAA/MHC complex presenting cells, such as TAA-C/MHC complex presenting cells that is ≥ 100 fold, ≥ 500 fold, ≥ 1000 fold, ≥ 2000 fold, ≥ 3000 fold, ≥ 4000 fold, ≥ 5000 fold, ≥ 6000 fold lower than the EC₅₀ value for normal tissue cells, such as an EC₅₀ for TA/MHC complex presenting cells, preferably TAA/MHC complex presenting cells, such as TAA-C/MHC complex presenting cells that is between 500 and 12000 fold, preferably 1000 and 10000 fold lower than the EC₅₀ value for normal tissue cells.

In cases when no EC₅₀ can be calculated the safety window can also be determined by calculating the ratio of the "lowest observed effect level" (LOEL) of a TCER^{®}-molecule on target cells and cells of normal tissues, respectively.

The "LOEL" is herein defined as the first TCER^{®} concentration with a response over cut-off value. The cut-off was defined as the sum of the assay background (coculture of healthy tissue cells and PBMC without added TCER^{®} molecule) and three-fold the standard deviation within all assay wells.

In one embodiment, the bispecific antigen binding protein of the invention has an LOEL for TA/MHC complex presenting cells, preferably TAA/MHC complex presenting cells, such as TAA-C/MHC complex presenting cells that is ≥ 100 fold, ≥ 500 fold, ≥ 1000 fold, ≥ 2000 fold, ≥ 3000 fold, ≥ 4000 fold, ≥ 5000 fold, ≥ 6000 fold lower than the LOEL for normal cells, such as an LOEL for TA/MHC complex presenting cells, preferably TAA/MHC complex presenting cells, such as TAA-C/MHC complex presenting cells that is between 500 and 12,000 fold, preferably 1,000 and 10,000 fold lower than the LOEL for normal cells.

In one embodiment, the bispecific antigen binding protein of the invention has an EC₅₀ for TA/MHC complex presenting cells, preferably TAA/MHC complex presenting cells, TAA-C/MHC complex presenting cells that is ≥5 fold, ≥10 fold, ≥20 fold, ≥50 fold, ≥ 100 fold, ≥ 500 fold, ≥ 1000 fold, ≥ 2,000 fold, ≥ 3,000 fold, ≥ 4,000 fold, ≥ 5000 fold, ≥ 6,000 fold lower than the EC₅₀ value for similar peptide/MHC presenting cells, wherein the EC₅₀ is preferably determined with respect to induced cytotoxicity, such as an EC₅₀ for TA/MHC complex presenting cells, preferably TAA/MHC complex presenting cells that is between 500 and 12,000, preferably 1,000 and 12000 lower than the EC₅₀ value for similar peptide/MHC presenting cells.

"Similar peptides" in the context of the present invention may also be referred to as "off-targets" and relates to peptides comprising typically 8 to 16 amino acids in length. The similar peptides in the context of the present invention are typically MHC presented. Furthermore, similar peptides in the context of the present invention comprises or consists of an amino acid sequence that is similar to the amino acid sequence of the TA antigenic peptide, in one preferred example in the context of the present invention, peptides that, in comparison to the epitope of the TA antigenic peptide, comprise an epitope wherein at least one amino acid, such as at least 1, 2 or 3, preferably 1, 2 or 3, more preferably 1, of said epitope in comparison to the epitope of the TA antigenic peptide is substituted. Due to this sequence similarity, similar peptides might be bound by a bispecific antigen binding protein of the invention, in this scenario, if, for example, the similar peptide is presented by a MHC protein and, thus bound by a bispecific antigen binding protein, the ability of a given bispecific antigen binding protein, to bind to a similar peptide will not lead to the desired effector cell response but may lead to adverse reactions. Such adverse reactions may be "off-tumor" side effects, such as cross-reactivity of a specific TCR which cross-reacted with a peptide in normal tissues as reported in Lowdell et al., Cytotherapy, published on December 4, 2018, page 7. Similar peptides in the context of the present invention may be selected from a database of, for instance, normal tissue-presented HLA-class 1 bound peptides (XPRESIDENT database), such as HLA-A*02 bound peptides in case of MAGE-A, based on, for instance, high sequence similarity (similarity BLAST search) to the TA antigenic peptide, such as MAG-003. Due to these adverse reactions the bispecific antigen binding proteins of the present invention are thus engineered to avoid cytotoxicity towards cells of normal tissues presenting similar peptides.

A "similar peptide/MHC presenting cell" herein denotes a cell that presents on its cell surface similar peptide/MHC complex.

In one embodiment, the similar peptide/MHC presenting cells have a similar peptide/MHC copy number of more than 50, more than 100, more than 150, more than 200, more than 300, more than 600, more than 800, more than 1000, more than 1500, more than 2000, preferably a TAA/MHC copy number of 50 to 5000.

In one embodiment, the similar peptide/MHC presenting cell is a MAGE-A/MHC complex presenting cell, and has a MAGE-A/MHC complex copy number of more than 50, more than 80, more than 100, more than 120, more than 150, more than 300, more than 400, more than 600, more than 800, more than 1000, more than 1500, more than 2000, preferably a MAGE-A/MHC copy number of 50 to 2000, such as 80 to 2000, such as 100 to 2000, for example 120 to 2000.

In one embodiment, the TA antigenic peptide is the Mage-A antigenic peptide and the similar peptides are selected from the list consisting of the peptide RABGAP1L-001 consisting of the amino acid sequence of SEQ ID NO: 269, AXIN1-001 consisting of the amino acid sequence of SEQ ID NO: 270, ANO5-001 consisting of the amino acid sequence of SEQ ID NO: 271, TPX2-001 consisting of the amino acid sequence of SEQ ID NO: 272, SYNE3-001 consisting of the amino acid sequence of SEQ ID NO: 273, MIA3-001 consisting of the amino acid sequence of SEQ ID NO: 274, HERC4-001 consisting of the amino acid sequence of SEQ ID NO: 275, PSME2-001 consisting of the amino acid sequence of SEQ ID NO: 276, HEATR5A-001 consisting of the amino acid sequence of SEQ ID NO: 277, CNOT1-003 consisting of the amino acid sequence of SEQ ID NO: 278, TEP1-003 consisting of the amino acid sequence of SEQ ID NO: 279, ZFC-001 consisting of the amino acid sequence of SEQ ID NO: 281, and PITPNM3-001 consisting of the amino acid sequence of SEQ ID NO: 280.

Accordingly, in one embodiment, the bispecific antigen binding protein of the invention does not bind or does not significantly bind to at least one similar peptide, such as at least 2, at least 3, at least 4, at least 5, such as 1, 2, 3, 4, 5, preferably at least 3 or 3 or all similar peptides selected from the group of peptides consisting of RABGAP1L-001, AXIN1-001, ANO5-001, TPX2-001, SYNE3-001, MIA3-001, HERC4-001, PSME2-001, HEATR5A-001, CNOT1-003, TEP1-003, PITPNM3-001, ZFC-001 preferably HEATR5A-001, HERC4-001 and ZFC-001 when said similar peptide is in a complex with a MHC protein, preferably in complex with HLA-A*02.

"Does not significantly bind" in the context of similar peptides and in the context of the bispecific antigen binding proteins herein is characterized by lower binding signals and/or higher K_{D} values. For example, in the context of the present invention the bispecific antigen binding protein has a binding response for the at least one similar peptides/MHC complex that is less than 50%, less than 45%, less than 40%, less than 30%, less than 20%, less than 20%, less than 10%, less than 5%, less than 4%, less than 3%, less than 3 % of the binding response of the same bispecific antigen binding protein to MAGE-A antigenic peptide/MHC complex in the same experimental setting and at the same bispecific antigen binding protein concentration and/or, for example, in the context of the present invention the bispecific antigen binding protein binds to the at least one similar peptide/MHC complex with an affinity that is decreased in comparison to the affinity for the specific antigen, i.e. the MAGE-A antigenic peptide/MHC complex as described herein and wherein the respective K_{D} for the respective similar peptide is increased by the factor of 5, 7, 10, 15, 20, 30, 40, 50, 100, preferably 20 to 100, more preferably 30 to 100, such as 40 to 100, typically 40 to 50. For example, when the bispecific antigen binding protein binds to the complex MAG-003/MHC with a K_{D} of 1 nM and the bispecific antigen binding protein binds to the complex of, for instance, RABGAP1L-001/MHC with a K_{D} of 100 nM then the bispecific antigen binding protein binds to RABGAP1L-001/MHC with a K_{D} that is increased by a factor of 100 and thus with an affinity that is decreased by a factor of 100. In these examples, the binding response, the dissociation constants and binding affinities are preferably measured using biolayer interferometry as described in, for example, example 4 and 5.

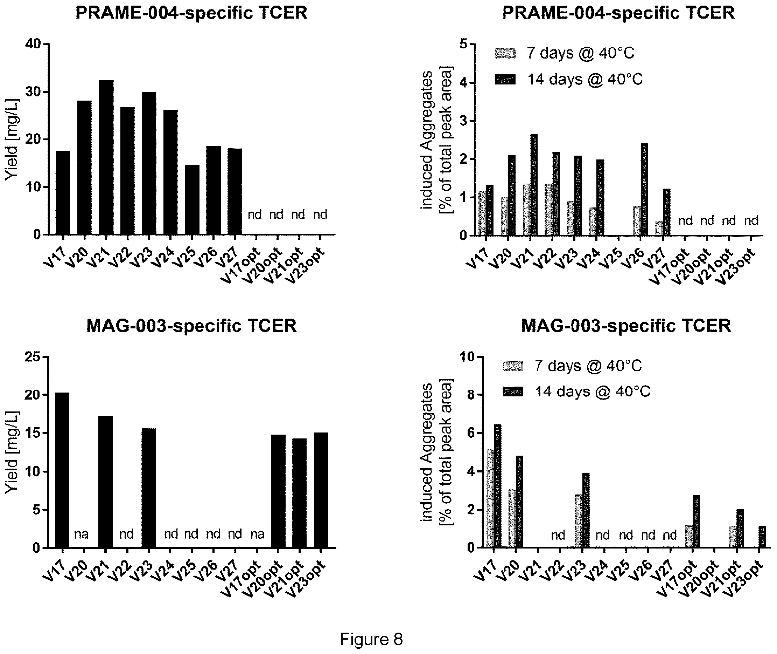

As it can be further seen from the examples, in particular example 1 and Figure 8 the described bispecific antigen binding proteins are not only soluble but can also be expressed in CHO cells in a transient expression in amounts that are > 10mg/L(cell culture),> 20mg/L or even 40mg/L. Accordingly, in one embodiment, the bispecific antigen binding protein of the present invention can be expressed in a host cell with high yields, wherein preferably the host cell is CHO cell and wherein preferably the yield is more than 10, more than 15, 18, 20, 22, 24, 26, 28, 30, 35, 40, 45 mg/L(cell culture), such as 5 to 50, 5, to 45, 5 to 40, 5 to 35, 10 to 35, 10 to 30, 10 to 25, 10 to 20 mg/L(cell culture). In one example the antigen binding protein has been expressed in transiently transfected CHO-S cells wherein the cells are cultured at T=0 in a density of 4x10⁶/mL at 37°C in a medium of GE Healthcare^{™} in a total volume of 320 mL. After one day feed solution (CellBoost 7a and b) was added and the temperature was lowered to 32°C.The cells and thus the antigen binding protein was harvested after a total culture time of 12 days and three feeds in total.

As it can be further seen from the examples, the inventors demonstrated, that the dscribed bispecific antigen binding proteins may have a comparable or even improved stability, in comparison to a reference protein, for instance, in comparison to an antigen binding protein comprising the VH domain comprising or consisting of the amino acid sequence of SEQ ID NO: 39 and the VL domain comprising or consisting of the amino acid sequence of SEQ ID NO: 38 or the VH domain comprising or consisting of the amino acid sequence of SEQ ID NO: 137 and the VL domain comprising or consisting of the amino acid sequence of SEQ ID NO: 145, preferably the VH domain comprising or consisting of the amino acid sequence of SEQ ID NO: 137 and the VL domain comprising or consisting of the amino acid sequence of SEQ ID NO: 145.

Accordingly the described antigen binding proteins may have a comparable or improved stability, optionally, in comparison to a reference protein. In the context of the present invention, a comparable or improved stability refers for example to a comparable or increased physical stability when exposed to thermal stress. The newly developed antigen binding proteins can thus comparable or better withstand stress conditions, especially thermal stress than the reference antigen binding protein, wherein said antigen binding protein is preferably in the same format.

The term "stability" in the context of the present invention refers to physical stability and can be evaluated qualitatively and/or quantitatively using various analytical techniques that are described in the art and are reviewed in for example Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993). In the context of the present invention, those methods refer in particular to the evaluation of aggregate formation (for example using size exclusion chromatography, by measuring turbidity, and/or by visual inspection. In order to measure stability a sample which comprises the antigen binding protein of the invention may be tested in a stability study, wherein a sample is exposed for a selected time period to a stress condition followed by quantitative and optionally qualitative analysis of the chemical and physical stability using an adequate analytical technique.

The described antigen binding proteins may be physical stable, for instance, when exposed to stress condition for a certain period of time, such as when exposed for, for instance, 14 days, to a temperature of 40°C.

"Physical stability" refers substantially, in the context of the present invention, to an antigen binding protein having no signs of aggregation, precipitation and/or denaturation.

Methods to access the physical stability are for example size exclusion chromatography (SEC), dynamic light scattering (DLS), light obscuration (LO) and colour and clarity.

"No signs of aggregation" means, for example, that a sample comprising the antigen binding protein, after having been exposed to a stress condition, such as, to a temperature of 40°C for 14 days in a buffer, such as PBS, has a monomer content of more than 80%, more than 86%, more than 88%, more than 90%, more than 92%, more than 94%, more than 96%, more than 97%, more than 98%, more than 99%, such as a monomer content of 94% to 99%, 95% to 99%, 96% to 99%, 97% to 99% monomer content, when measured by SEC, such as SEC- HPLC, in a buffer, such as PBS.

Accordingly, The described antigen binding proteins may have have same or a reduced aggregation, for example, in comparison to a reference protein.

For size exclusion chromatography (SEC), a difference of 1%, 2%, 3%, 4%, preferably 1 or 2%, more preferably 2%, of the monomer content is considered as significantly different in the context of the invention under the tested conditions depending on the column used, operating pressure, and velocity of the buffer.

This means, when the reference antigen binding protein has a monomer content of 96% and the antigen binding protein of the invention has a monomer content of 98%, the monomer content of the antigen binding protein of the invention is significantly different and thus significantly increased in comparison to the reference antigen binding protein, when measured in the same conditions.

### Nucleic acids, vectors and recombinant host cells

A further object of the invention relates to an isolated nucleic acid sequence comprising or consisting of a sequence encoding a bispecific antigen binding protein of the invention which is as defined herein above.

Typically, said nucleic acid is a DNA or RNA molecule, which may be included in any suitable vector, such as a plasmid, cosmid, episome, artificial chromosome, phage or a viral vector.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

So, a further object of the invention relates to a vector comprising a nucleic acid of the invention.

Such vectors may comprise regulatory elements, such as a promoter, enhancer, terminator and the like, to cause or direct expression of said polypeptide upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like. Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

The term "viral vector" refers to a nucleic acid vector construct that includes at least one element of viral origin and has the capacity to be packaged into a viral vector particle, and encodes at least an exogenous nucleic acid. The vector and/or particle can be utilized for the purpose of transferring any nucleic acids into cells either in vitro or in vivo. Numerous forms of viral vectors are known in the art. The term "virion" is used to refer to a single infective viral particle. "Viral vector", "viral vector particle" and "viral particle" also refer to a complete virus particle with its DNA or RNA core and protein coat as it exists outside the cell. For example, a viral vector may be selected from adenoviruses, poxviruses, alphaviruses, arenaviruses, flaviruses, rhabdoviruses, retroviruses, lentiviruses, herpesviruses, paramyxoviruses, or picornaviruses.

Viruses may refer to natural occurring viruses as well as artificial viruses. Viruses in accordance to some embodiments of the present invention may be either an enveloped or non-enveloped virus. Parvoviruses (such as AAVs) are examples of non-enveloped viruses. In a preferred embodiment, the viruses may be enveloped viruses. The viruses may be retroviruses and in particular lentiviruses. Viral envelope proteins that can promote viral infection of eukaryotic cells may include HIV-1 derived lentiviral vectors (LVs) pseudotyped with envelope glycoproteins (GPs) from the vesicular stomatitis virus (VSV-G), the modified feline endogenous retrovirus (RD114TR), and the modified gibbon ape leukemia virus (GALVTR). These envelope proteins can efficiently promote entry of other viruses, such as parvoviruses, including adeno-associated viruses (AAV), thereby demonstrating their broad efficiency. For example, other viral envelop proteins may be used including Moloney murine leukemia virus (MLV) 4070 env (such as described in Merten et al., J. Virol. 79:834-840, 2005;), RD114 env, chimeric envelope protein RD114pro or RDpro (which is an RD114-HIV chimera that was constructed by replacing the R peptide cleavage sequence of RD114 with the HIV-1 matrix/capsid (MA/CA) cleavage sequence, such as described in Bell et al. Experimental Biology and Medicine 2010; 235: 1269-1276;), baculovirus GP64 env (such as described in Wang et al. J. Virol. 81:10869-10878, 2007), or GALV env (such as described in Merten et al., J. Virol. 79:834-840, 2005;), or derivatives thereof.

A further object of the present invention relates to a host cell which has been transformed, transduced or transfected with a nucleic acid and/or a vector according to the invention.

The term "transformation" originally refers to a naturally occurring process of gene transfer into a host cell which involves absorption of the genetic material, such as nucleic acids, for instance, DNA or RNA, by a cell through cell membrane, so that the host cell will express the introduced gene or sequence to produce a desired substance, typically a protein or enzyme coded by the introduced gene or sequence. There are two types called as natural transformation and artificial or induced transformation. The artificial or induced method of transformation is done under laboratory condition.

A host cell that receives and expresses foreign nucleic acids, such as DNA or RNA, by the process of a transformation has been "transformed".

The term "Transfection" refers to a mode of gene transfer involving creation of pores on the cell membrane of the host cell enabling the host cell to receive the foreign genetic material. Typically transfection refers to a transformation of eukaryotic cells, such as insect or mammalian cells. Chemical mediated transfection involves use of, for instance, calcium phosphate or cationic polymers or liposomes. Non -chemical mediated transfection methods are typically electroporation, sonoporation, impalefection, optical transfection or hydro dynamic delivery. Particle based transfection uses gene gun technique where a nanoparticle is used to transfer the nucleic acid to host cell or by another method called as magnetofection. Nucleofection and use of heat shock are the other evolved methods for successful transfection. A host cell that receives foreign nucleic acids via a transfection method has been "transfected".

The term "Transduction" is generally understood to relate to the transfer of foreign nucleic acids, such as DNA or RNA, into a cell by a virus or viral vector. A host cell that receives and expresses foreign nucleic acids, such as DNA or RNA, by a virus or viral vector has been

### "transduced".

In some embodiments, cells may be transduced using the methods described in US20190216852.

The nucleic acids of the invention may be used to produce a recombinant antigen binding protein of the invention in a suitable expression system.

The term "expression system" means a host cell and compatible vector under suitable conditions, e.g. for the expression of a protein coded for by foreign DNA carried by the vector and introduced to the host cell.

Common expression systems include *E. coli* host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E. *coli, Kluyveromyces* or *Saccharomyces* yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like. In some embodiments, the YB2/0 cell may be preferred, since ADCC activity of chimeric or humanized antibodies is enhanced when expressed in this cell.

The invention also pertains to a host cell comprising a bispecific antigen recognizing construct in accordance with the invention. Specifically, the host cell of the invention comprises a nucleic acid, or a vector as described herein above. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing a bispecific antigen binding protein, such as a bispecific TCR, polypeptide, or protein, the host cell is preferably a mammalian cell.

In one particular embodiment, the host cell is a stem cell, preferably a mesenchymal stem cell.

According to the above, in one embodiment, the invention refers to a host cell comprising the bispecific antigen binding protein of the invention which is defined herein above, or the nucleic acid, or the vector of the invention, wherein said host cell preferably is a) mesenchymal stem cell or b) a cell for recombinant expression, such as a Chinese Hamster Ovary (CHO) cell.

In particular, for expression of some of the bispecific antigen binding proteins of the invention the expression vector may be either of a type in which a gene encoding the first polypeptide, such as an antibody heavy chain or an alpha chain, and a gene encoding a second polypeptide, such as an antibody light chain or a beta chain, exists on separate vectors or of a type in which both genes exist on the same vector (tandem type). In respect of easiness of construction of bispecific antigen binding protein expression vector, easiness of introduction into animal cells, and balance between the expression levels of antibody H and L chains in animal cells, humanized antibody expression vector of the tandem type is preferred (Shitara K et al. J Immunol Methods. 1994 Jan. 3;167(1-2):271-8). Examples of tandem type humanized antibody expression vector include pKANTEX93 (WO 97/10354), pEE18 and the like.

In one embodiment such recombinant host cells can be used for the production of at least one antigen binding protein of the invention

Methods of producing bispecific antigen binding proteins of the invention The present invention also relates to a method of producing the antigen binding protein as defined herein above, comprising
a. providing a suitable host cell,
b. providing a genetic construct comprising a coding sequence encoding the bispecific antigen binding protein of the invention,
c. introducing, preferably *in vitro* or *ex vivo,* said genetic construct into said suitable host cell, and
d. expressing said genetic construct by said suitable host cell, and optionally e. selecting the cells which express and/or secrete said antibody.

The bispecific antigen binding protein of the invention is as defined herein above in the corresponding section.

"Genetic construct" herein denotes nucleic acids that allow expression of the coding region in a host, and thus refers to nucleic acids, such as vectors, described herein above or RNA.

In one specific embodiment, the method may further comprise a step of cell surface presentation of said bispecific antigen recognizing construct on said suitable host cell.

In other preferred embodiments, the genetic construct in b) comprises a nucleic acid encoding the bispecific antigen binding proteins of the invention. Such nucleic acids are as defined herein above in the section 'nucleic acid, vectors and recombinant host cells'.

In a related embodiment, the genetic construct is an expression construct comprising a promoter sequence operably linked to said coding sequence.

In a related embodiment, the genetic constructs are introduced into the suitable host using transformation, transduction or transfection. Transformation, transduction or transfection are as defined in the section herein above.

Desirably, the transduction system for introducing the genetic construct into said suitable host cell is a retroviral or lentiviral vector system as described herein above in the section nucleic acid, vectors and recombinant host cells. Such systems are well known to the skilled artisan.

In one embodiment, the method further comprises the isolation and purification of the bispecific antigen binding protein from the host cell and, optionally, reconstitution of the bispecific antigen binding protein in a T cell.

A bispecific antigen binding protein of the invention may be produced by any technique known in the art, such as, without limitation, any chemical, biological, genetic or enzymatic technique, either alone or in combination.

Standard techniques for production of polypeptides, such as antibodies or fragments thereof and TCRs or fragments thereof, are known in the art and these techniques can be used by the skilled in the art to produce the bispecific antigen binding proteins of the present invention. For instance, they can be synthesized using well-known solid phase method, in particular using a commercially available peptide synthesis apparatus (such as that made by Applied Biosystems, Foster City, California) and following the manufacturer's instructions. Alternatively, bispecific antigen binding proteins of the invention, such as antibodies or fragment thereof and TCRs or fragments thereof, can be synthesized by recombinant DNA techniques as is well-known in the art. For example, fragments can be obtained as DNA expression products after incorporation of DNA sequences encoding the desired (poly)peptide into expression vectors and introduction of such vectors into suitable eukaryotic or prokaryotic hosts that will express the desired polypeptide, from which they can be later isolated using well-known techniques.

In one example, i.e. in case of TCER^{®} bispecific molecules, DNA-sequences coding for various combinations of V_{H} and V_{L} and variable alpha (Valpha) and variable beta (Vbeta), as well as sequences coding for linkers may be obtained by, for instance, gene synthesis. Resulting DNA-sequences may be cloned in frame into expression vectors coding for hinge region, C_{H2} and C_{H3} domain derived from, for example, human IgG4 [Accession#: K01316] and IgG1 [Accession#: P01857], respectively and may be further engineered. Engineering may be performed to incorporate knob-into-hole mutations into C_{H3}-domains with and without additional interchain disulfide bond stabilization; to remove an N-glycosylation site in C_{H2} (e.g. N297Q mutation); to introduce F_{c}-silencing mutations or to introduce additional disulfide bond stabilization into V_{L} and V_{H}, respectively, according to the methods described by Reiter et al. (Stabilization of the Fv Fragments in Recombinant Immunotoxins by Disulfide Bonds Engineered into Conserved Framework Regions. Biochemistry, 1994, 33, 5451 - 5459).

Bispecific antigen binding proteins of the invention are suitably separated from the culture medium by immunoglobulin purification procedures such as, for example, protein A-Sepharose, hydroxylapatite chromatography, gel electrophoresis, dialysis, or affinity chromatography.

In one embodiment, recovering the expressed bispecific antigen binding proteins or polypeptides herein refers to performing a protein A chromatography, a Kappa select chromatography, and/or a size exclusion chromatography, preferably a protein A chromatography and/or a size exclusion chromatography, more preferably a protein A chromatography and a size exclusion chromatography.

Methods for producing bispecific antigen binding proteins of the invention involve recombinant DNA and gene transfection techniques are well known in the art (See Morrison SL. et al. (1984) and patent documents US5,202,238; and US5,204, 244).

Furthermore, methods for producing humanized antibodies based on conventional recombinant DNA and gene transfection techniques are well known in the art (See, e.g., Riechmann L. et al. 1988; Neuberger MS. et al. 1985) and can be easily applied to the production of bispecific antigen binding proteins of the invention.

In one example, vectors for the expression of the recombinant antigen binding proteins of the invention were designed as mono-cistronic, for instance, controlled by HCMV-derived promoter elements, pUC19-derivatives. Plasmid DNA was amplified, for example, in E.coli according to standard culture methods and subsequently purified using commercial-available kits (Macherey & Nagel). Purified plasmid DNA was used for transient transfection of, for example, CHO-S cells according to instructions of the manufacturer (ExpiCHO^{™} system; Thermo Fisher Scientific) or utilizing an electroporation systems (MaxCyte STX). Transfected CHO-cells were cultured, for instance, for 6-14 days at, for example, 32°C to 37°C and received one to two feeds of ExpiCHO^{™} Feed or Cellboost 7a and 7b (GE Healthcare^{™}) solution. Conditioned cell supernatant was cleared by, for example, filtration (0.22 µm) utilizing, for instance, Sartoclear Dynamics^{®} Lab Filter Aid (Sartorius). Bispecific antigen binding proteins were purified using, for example, an Äkta Pure 25 L FPLC system (GE Lifesciences) equipped to perform affinity and size-exclusion chromatography in line. Affinity chromatography was performed on, for example, protein A or L columns (GE Lifesciences) following standard affinity chromatographic protocols. For instance, size exclusion chromatography was performed directly after elution (pH 2.8) from the affinity column to obtain highly pure monomeric protein using, for example, Superdex 200 pg 16/600 columns (GE Lifesciences) following standard protocols. Protein concentrations were determined on, for example, a NanoDrop system (Thermo Scientific) using calculated extinction coefficients according to predicted protein sequences. Concentration was adjusted, if needed, by using Vivaspin devices (Sartorius). Finally, purified molecules were stored in, for example, phosphate-buffered saline at concentrations of about 1 mg/mL at temperatures of 2-8°C.

Quality of purified bispecific antigen binding proteins was determined by, for example, HPLC-SEC on MabPac SEC-1 columns (5 µm, 4x300 mm) running in, for example, 50 mM sodium-phosphate pH 6.8 containing 300 mM NaCl within a Vanquish uHPLC-System.

### Pharmaceutical compositions

The invention further refers to a pharmaceutical composition comprising the bispecific antigen binding protein of the invention, the nucleic acids of the invention, the vector of the invention, or the host cell of the invention and a pharmaceutically acceptable carrier.

The invention also relates to a bispecific antigen binding protein according to the invention, for use as a medicament. The invention also relates to a pharmaceutical composition of the invention for use as a medicament.

The invention also relates to the use of a bispecific antigen binding protein according to the invention and/or to a pharmaceutical composition of the invention, in the manufacture of a medicament.

The terms "pharmaceutical composition" or "therapeutic composition" as used herein refer to a compound or composition capable of inducing a desired therapeutic effect when properly administered to a subject.

In some embodiments, the subject may also be referred to as patient.

Such therapeutic or pharmaceutical compositions may comprise a therapeutically effective amount of a bispecific antigen binding protein of the invention or further comprising a therapeutic agent, in admixture with a pharmaceutically or physiologically acceptable formulation agent selected for suitability with the mode of administration.

The bispecific antigen binding protein of the present invention will usually be supplied as part of a sterile, pharmaceutical composition which will normally include a pharmaceutically acceptable carrier.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

A "pharmaceutically-acceptable carrier" may also be referred to as "pharmaceutically acceptable diluent" or "pharmaceutically acceptable vehicles" and may include solvents, bulking agents, stabilizing agents, dispersion media, coatings, antibacterial and antifungal agents, isotonic and absorption delaying agents, and the like which are physiologically compatible. Accordingly, in one embodiment the carrier is an aqueous carrier.

In another aspect, the aqueous carrier is capable of imparting improved properties when combined with a bispecific antigen binding protein described herein, for example, improved solubility, efficacy, and/or improved immunotherapy.

The form of the pharmaceutical compositions, the route of administration, the dosage and the regimen naturally depend upon the condition to be treated, the severity of the illness, the age, weight, and gender of the patient, the desired duration of the treatment etc. This pharmaceutical composition may be in any suitable form, (depending upon the desired method of administering it to a patient). It may be provided in unit dosage form, will generally be provided in a sealed container and may be provided as part of a kit. Such a kit would normally (although not necessarily) include instructions for use. It may include a plurality of said unit dosage forms.

Empirical considerations, such as the biological half-life, generally will contribute to the determination of the dosage. Frequency of administration may be determined and adjusted over the course of therapy and is based on reducing the number of cancer cells, maintaining the reduction of cancer cells, reducing the proliferation of cancer cells, or killing the cancer cells. Alternatively, sustained continuous release formulations of the bispecific antigen binding protein may be appropriate. Various formulations and devices for achieving sustained release are known in the art.

In one embodiment, dosages for the antigen binding proteins may be determined empirically in individuals who have been given one or more administration(s). Individuals are given incremental dosages of the antigen binding protein. To assess efficacy of the antigen binding protein, a marker of the cancer cell state can be followed. These include direct measurements of cancer cell proliferation and cell death by FACS, other imaging techniques; an improvement in health as assessed by such measurements, or an increase in quality of life as measured by accepted tests or prolongation of survival. It will be apparent to one of skill in the art that the dosage will vary depending on the individual, the stage of the disease, and the past and concurrent treatments being used.

In particular, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

To prepare pharmaceutical compositions, an effective amount of the bispecific antigen binding protein of the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An antigen-binding protein of the invention can be formulated into a composition in a neutral or salt form using pharmaceutically acceptable salts.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

### Therapeutic methods and uses

Any references herein to methods of treatment by therapy or surgery or in vivo diagnosis methods are to be interpreted as references to compounds, pharmaceutical compositions, or medicaments of the present invention for use in those methods.

The inventors have shown in Example 2 of the experimental section *in vitro* for MAG-003 targeting antigen binding proteins combined with either BMA031 (V36) or UCHT1(V17) as recruiter, in particular in the TCER^{®} format, the cytotoxic activity of those molecules for different MAG-003 positive cancer cell lines. The inventors have furthermore demonstrated that said cytotoxic activity is highly specific and limited to the TAA positive cells, such as MAG-003-positive cells, since only marginal lysis was induced by the bispecific antigen binding proteins in cell lines expressing HLA-A*02 but not presenting the TAA peptide, such as MAG-003. The examples therefore demonstrate the technical advantage of combining the low affinity binding domain directed against CD3 which is disclosed in the context of the present invention with a high affinity TCR variable domains. In the examples, such a TCR variable domain specifically binds to the TAA MAGE-A, however, it will be understood by the skilled in the art, that the advantages observed for bispecific antigen binding proteins exemplified in the context of a TAA as target, are also transferable to bispecific antigen binding proteins targeting another TA, such as a viral antigenic peptide or a bacterial antigenic peptide, instead of a TAA. It will be understood by the skilled in the art, that in several embodiments the antigen binding protein, once administered to the subject binds to the target cell, such as a TA/MHC complex presenting cell and recruits endogenous effector cells, binds them via CD3, activates them and thus localizes those effector cells in the proximity of targeted cell, in particular the targeted cancer cells to achieve a killing of said target cell, in particular an anti-cancer activity.

In an aspect, binding of the antigen binding protein to a target cell and to CD3 of an effector cell elicits an immune response, such a response may refer to the proliferation and activation of effector functions, in vitro or in vivo. For MHC class I restricted cytotoxic T cells, for example, effector functions may be lysis of peptide-pulsed, peptide-precursor pulsed or naturally peptide-presenting target cells, secretion of cytokines, preferably Interferon-gamma, TNF-alpha, or IL-2 induced by peptide, secretion of effector molecules, for example, granzymes or perforins induced by peptide, or degranulation.

Accordingly, the bispecific antigen binding proteins of the present invention, in particular TCER^{®} molecules, may be used to treat a wide variety of conditions including, for example, various forms of cancer and/or infections conditions. The bispecific antigen binding proteins of the present invention may be used for therapeutic purposes in humans and/or non-human mammalian animals, in particular in humans.

In one embodiment, the bispecific antigen binding proteins of the present invention can bind to diseased cells and reduce the growth of and/or kill the diseased cells presenting the TA peptide/MHC complex on their cell surface. In one preferred embodiment, the bispecific antigen binding proteins of the present invention can bind to tumor cells and reduce the growth of and/or kill the tumor cells presenting the TAA peptide/MHC complex on their cell surface. It is understood that the bispecific antigen binding protein is administered at a concentration that promotes binding at physiological (e.g., in vivo) conditions.

Accordingly, in one embodiment, the bispecific antigen binding proteins of the invention can be used for immunotherapy directed against tumor cells of different tissues such as colon, lung, breast, prostate, ovary, pancreas, kidney etc. In another embodiment, the antigen binding proteins of the invention can bind to and reduce the growth of and/or kill tumor cells.

Disclosed for reference is a method of treating or preventing a proliferative disease or disorder comprising administering to a subject in need thereof a therapeutically effective amount of the bispecific antigen binding protein, the nucleic acid or vector, the host cell or the pharmaceutical composition as defined herein above in the section "Bispecific antigen binding protein" "Nucleic acids" or "Pharmaceutical compositions".

Disclosed for reference is a method of treating a subject who has a disease comprising administering to said subject the bispecific antigen binding protein of the invention.

Disclosed for reference is a method of eliciting an immune response in a subject, who has a disease, comprising administering to said subject a composition comprising the antigen recognizing construct of the invention optionally expressed in a host cell.

Disclosed for reference is the use of the bispecific antigen binding protein, the nucleic acid or vector, the host cell or the pharmaceutical composition according to the invention for treating or preventing a disease in a subject.

The immune response referred to in said method may be a cytotoxic T cell response. Said cytotoxic T cell response is induced by the binding of the antigen binding protein to CD3 present on an effector cell and by binding to the TA antigenic peptide/MHC complex and thus, by bringing the effector cell and the target cell into proximity.

The invention further refers to bispecific antigen binding proteins of the invention, the nucleic acid of the invention or the vector of the invention, the host cell of the invention or the pharmaceutical composition of the invention for use in the diagnosis (for reference), prevention, and/or treatment of a disease.

Disclosed for reference is the use of the bispecific antigen binding proteins of the invention, the nucleic acid of the invention or the vector of the invention, the host cell of the invention or the use of the pharmaceutical composition of the invention for the manufacture of a medicament for the diagnosis, prevention, and/or the treatment of a disease.

The term "subject" or "individual" are used interchangeably and may be, for example, a human or a non-human mammal, preferably, a human.

In the context of the invention, the term "treating" or "treatment", refers to a therapeutic use (i.e. on a subject having a given disease) and means reversing, alleviating, inhibiting the progress of one or more symptoms of such disorder or condition. Therefore, treatment does not only refer to a treatment that leads to a complete cure of the disease, but also to treatments that slow down the progression of the disease and/or prolong the survival of the subject.

By "preventing" is meant a prophylactic use (i.e. on a subject susceptible of developing a given disease).

The term "in need of treatment" refers to a subject having already the disorder as well as those in which the disorder is to be prevented. Accordingly, in one embodiment, the subject is a patient.

In one embodiment, a "disease" or "disorder" is any condition that would benefit from treatment with the antigen binding protein of the invention. In one embodiment, this includes chronic and acute disorders or diseases including those pathological conditions which predisposes the subject to the disorder in question. In particular, the disease referred to in the context of the present invention may be a proliferative disease or a disease caused by a virus or a bacteria. A disease caused by a virus or bacteria may also be referred to as a viral or bacterial infection. In the context of the present invention, the virus causing the disease may be selected from the group constituted of for example, human immunodeficiency viruses (HIV), Humane Cytomegalovirus (HCMV), cytomegalovirus (CMV), human papillomavirus (HPV), Hepatitis B virus (HBV), Hepatitis C virus (HCV), Epstein-Barr virus (EBV), Influenza virus, preferably human immunodeficiency viruses (HIV). In the context of the present invention, the bacteria causing the disease, such as Mycobacterium tuberculosis. It will be understood by the skilled in the art, that when the bispecific antigen binding protein targets a viral antigenic peptide, for instance, of HIV, the bispecfic antigen binding protein is for use in the treatment of HIV. Accordingly, a bispecific antigen binding protein targeting the viral or bacterial antigenic peptide TA-C is thus for use in the treatment of virus or bacteria from which said antigenic viral or bacterial antigenic peptide, was derived.

"Proliferative diseases", such as cancer, involve the unregulated and/or inappropriate proliferation of cells.

In one embodiment, the proliferative disorder or disease is, for example, a tumor disease characterized by the expression of the TA, more particular of the TAA, in a cancer or tumor cell of said tumor disease.

Accordingly, a particularly preferred cancer is a TA positive cancer, in particular a TAA positive cancer.

In a further embodiment, the proliferative disorder or disease is, for example, a tumor disease characterized by the expression of MAGEA4 and/or MAGEA8, in a cancer or tumor cell of said tumor disease.

Accordingly, a particularly preferred cancer is a MAGEA4 and/or MAGEA8 positive cancer.

In a further embodiment, the proliferative disorder or disease is, for example, a tumor disease characterized by the expression of PRAME, in a cancer or tumor cell of said tumor disease.

Accordingly, a particularly preferred cancer is a PRAME positive cancer.

In the context of the present invention, a cancer is considered to be '"TAA positive", such as "MAGEA4 and/or MAGEA8 positive" or "PRAME positive", if the related TAA peptide, such as one of TAA peptides as defined herein above in the section "Definitions", for example the MAG-003 peptide or PRAME-004, is presented in >98% of all cancers according to the guidelines by the NCI. In all other indications named here a biopsy can to be performed as it is standard in the treatment of these cancers and the peptide can be identified according to the XPRESIDENT^{®} and related methods (according to WO 03/100432; WO 2005/076009; WO 2011/128448; WO 2016/107740, US 7,811,828, US 9,791,444, and US 2016/0187351). In one embodiment, the cancer is readily assayed (i.e. diagnosed) for instance by using a bispecific antigen binding protein of the invention. Methods to identify an antigen expressing cancer using an antigen-binding protein are known to the skilled in the art.

In one embodiment, the cancer is selected from the list consisting of lung cancer, such as non-small cell lung cancer, small cell lung cancer, liver cancer, head and neck cancer, skin cancer, renal cell cancer, brain cancer, gastric cancer, colorectal cancer, hepatocellular cancer, pancreatic cancer, prostate cancer, leukemia, breast cancer, Merkel cell carcinoma, melanoma, ovarian cancer, urinary bladder cancer, uterine cancer, gallbladder and bile duct cancer, osteosarcoma, and esophageal cancer.

Among the texts providing guidance for cancer therapy is Cancer, Principles and Practice of Oncology, 4th Edition, DeVita et al, Eds. J. B. Lippincott Co., Philadelphia, Pa. (1993). An appropriate therapeutic approach is chosen according to the particular type of cancer, and other factors such as the general condition of the patient, as is recognized in the pertinent field. The bispecific antigen binding proteins of the present invention can be used by itself or can be added to a therapy regimen using other anti-cancer agents typically used in treating a cancer patient.

Accordingly, in some embodiments, the bispecific antigen binding protein of the invention can be administered concurrently with, before, or after a variety of drugs and treatments widely employed in cancer treatment such as, for example, chemotherapeutic agents, non-chemotherapeutic, anti-neoplastic agents, and/or radiation, preferably chemotherapeutic agents.

In further embodiments, the bispecific antigen binding proteins can also be used to treat infectious disease, such as infectious viral or bacterial diseases, wherein the viral disease is, for example, selected from the group consisting of human immunodeficiency viruses (HIV), Humane Cytomegalievirus (HCMV), cytomegalovirus (CMV), human papillomavirus (HPV), Hepatitis B virus (HBV), Hepatitis C virus (HCV), human papillomavirus infection (HPV), Epstein-Barr virus (EBV), Influenza virus, preferably HIV, HBV, Influenza and HCMV among many others and wherein the bacterial disease is for example, tuberculosis.

An antigen binding protein of the invention or the pharmaceutical composition thereof can be administered by itself or can be administered concurrently with, before, or after administration of other therapeutics used to treat such infectious diseases.

"Diagnosis" herein refers to a Medical diagnosis and refers to determining which disease or condition explains a person's symptoms and signs.

By a "therapeutically effective amount" of the bispecific antigen binding protein or pharmaceutical composition thereof is meant a sufficient amount of the bispecific antigen binding protein to treat said proliferative disease, at a reasonable benefit/risk ratio applicable to any medical treatment. It will be understood, however, that the total daily or monthly usage of the antigen binding proteins, the nucleic acid or vector, the host cell or the pharmaceutical composition of the present invention will be decided by the attending physician within the scope of sound medical judgment. The specific therapeutically effective dose level for any particular patient will depend upon a variety of factors including the disorder or disease being treated and the severity of the disorder; activity of the specific bispecific antigen binding protein employed; the specific composition employed, the age, body weight, general health, sex and diet of the patient; the time of administration, route of administration, and rate of excretion of the specific polypeptide employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved.

In one embodiment, efficacy of the treatment with a bispecific antigen binding protein of the invention is assayed *in vivo,* for instance in a mouse model of cancer and by measuring, for example, changes in tumor volume between treated and control groups.

Pharmaceutical compositions, vectors, nucleic acids and cells of the invention may be provided in substantially pure form, for example, wherein at least 80%, at least 85%, at least 90%, at least 91%, at least 92%, at least 93%, at least 94%, at least 95%, at least 96%, at least 97%, at least 98%, at least 99% or 100% by weight of the bispecific antigen binding protein of the same type are present.

The bispecific antigen binding protein of the invention, the nucleic acid of the invention or the vector of the invention, the host cell of the invention or the pharmaceutical composition of the invention can be administered by any feasible method.

Disclosed for reference is a method of killing target cells in a patient the method comprising administering to the patient an antigen binding protein. In the context of this method the antigen binding protein, once administered to the subject binds to the target cell and a CD3 expressing effector cell and preferably elicits an immune response.

In one specific approach, the host cell may be a stem cell, such as a mesenchymal stem cell and is engineered to express the bispecific antigen binding protein of the invention. In this example, the bispecific antigen binding protein is a TCER^{®} as herein described.

Accordingly, the host cell of the present invention, preferably a stem cell as defined above, may be used as active ingredients of a therapeutic composition. Thus, disclosed for reference is a method of killing target cells in a patient the method comprising administering to the patient an effective number of host cells as defined above, preferably mesenchymal stem cell.

For purposes of the inventive methods, wherein host cells or populations of cells are administered to the subject, the host cells can be cells that are allogeneic (from another subject) or autologous to the subject. Preferably, the cells are autologous to the subject.

In case the host cells are allogeneic and thus from another subject, said other subject is healthy.

By "healthy" it is meant that the subject is generally in good health, preferably has a competent immune system and, more preferably, is not suffering from any disease that can be readily tested for and detected.

Accordingly, the host cell has been transformed, transduced or transfected with a nucleic acid and/or a vector according to the invention, as described herein above in the section 'nucleic acids, vectors and recombinant host cells'.

When the host cell is transformed, transduced or transfected to express or the bispecific antigen binding protein of the invention, preferably the cell comprises an expression vector capable of expressing the antigen binding protein. Once the host cell expresses the bispecific antigen binding protein of the invention, the host cell may then be referred to as activated host cell.

In an aspect, the TCR-elicited immune response or T cell response may refer to the proliferation and activation of effector functions induced by the binding of the bispecific antigen binding to the T cell and to the TA antigenic peptide/MHC complex, such as the TA-C/MHC complex, in vitro or in vivo. For MHC class I restricted cytotoxic T cells, for example, effector functions may be lysis of peptide-pulsed, peptide-precursor pulsed or naturally peptide-presenting target cells, secretion of cytokines, preferably Interferon-gamma, TNF-alpha, or IL-2 induced by peptide, secretion of effector molecules, for example, granzymes or perforins induced by peptide, or degranulation.

Thus, a further aspect of the invention provides activated host cells obtainable by the foregoing methods of the invention.

Activated host cells, which are produced by the above method, may selectively recognize a target cell.

A "target cell" herein refers to TA-C/MHC presenting cell or a TA presenting cell as defined herein above in the section "bispecific antigen binding proteins".

In one preferred embodiment, i.e. when the TA/MHC complex is a TAA/MHC complex, the target cell is a cancer cell, wherein the cancer is as defined herein above.

*In vivo,* the target cells for the CD3 positive effector cells according to the present invention can be cells of the tumor (which sometimes express MHC class II) and/or stromal cells surrounding the tumor (tumor cells) (which sometimes also express MHC class II; (Dengjel, J. et al., Clin Cancer Res 12 (2006): 4163-4170).

### Kits

Finally, disclosed for reference are kits comprising at least one bispecific antigen binding protein of the invention.

The kit may comprise
a) at least one bispecific antigen binding protein of the invention as defined herein above in the section "bispecific antigen binding proteins",
b) optionally packaging material, and
c) optionally a label or packaging insert contained within said packaging material indicting that said bispecific antigen binding protein is effective for treating a disease, preferably cancer or for use for the treatment of a disease, preferably cancer.

The at least one antigen binding proteins of the invention may be contained in a single and/or multi-chambered pre-filled syringe/s (e.g., liquid syringes and lyosyringes).

Disclosed for reference are kits for producing a single-dose administration unit.

Accordingly, the at least one bispecific antigen binding protein of the invention as mentioned in a) of the kit may be a dried bispecific antigen binding protein of the invention contained in a first container. The kit then further contains a second container having an aqueous formulation.

Accordingly, the kit may comprise
a) a first container comprising at least one dried bispecific antigen binding protein of the invention as defined herein above in the section "Antigen binding proteins",
b) a second container comprising an aqueous formulation;
c) optionally packaging material, and
d) optionally a label or packaging insert contained within said packaging material indicting that said bispecific antigen binding protein is effective for treating a disease, preferably cancer, or for use for the treatment of a disease, preferably cancer.

The aqueous formulation is typically an aqueous solution comprising pharmaceutically-acceptable carriers as defined herein above in the section "pharmaceutical compositions".

The "first container" and the "second" container may refer to the chambers of a multi-chambered pre-filled syringes (e.g., lyosyringes).

The cancer is as defined above.

### THE INVENTION

As per the appended claims, the object of the invention is a bispecific antigen binding protein comprising at least two antigen binding sites (D and B), wherein the antigen binding site D binds to TCRα/β and wherein the antigen binding site B binds to a target antigenic (TA) peptide/MHC complex, and wherein the antigen binding site D comprises a heavy chain variable domain (V_{H}) and a light chain variable domain (V_{L}), and wherein said V_{L} comprises or consists of the amino acid sequence of SEQ ID NO: 42, and wherein said V_{H} comprises or consists of the amino acid sequence of SEQ ID NO: 43.

The antigen binding site D binds to TCRα/β with a K_{D}(D) and the antigen binding site B binds to the target antigenic peptide C (TA-C)/MHC complex with a K_{D}(C) **.** The ratio of K_{D}(D)/K_{D}(C) can be more than 1, more than 4, more than 6, more than 8, more than 10, more than 15, more than 20, more than 25, more than 30, more than 40, more than 50, such as between 1 and 150, between 4 and 140, between 6 and 100, between 8 and 100, between 10 and 100, preferably between 10 and 100.

In one embodiment, the antigen binding site B binds to the target antigenic peptide C (TA-C)/MHC complex with a K_{D}(C) which is ≤ 100 µM, ≤ 1 µM ≤ 100 nM, ≤ 50 nM, ≤ 10 nM, for instance 0.01 nM to 150 nM, 0.05 nM to 150 nM, 0.1 nM to 150 nM, 0.1 nM to 100 nM, 0.1 nM to 50 nM, 0.1 nM to 10 nM, 0.5 nM to 10 nM, 0.5 nM to 5 nM, preferably 0.5 nM to 5 nM as determined using Surface plasmon resonance (SPR) or Biolayer Interferometry (BLI), preferably Biolayer Interferometry (BLI).

The antigen binding protein may have an EC₅₀ for TA-C/MHC presenting cells that is ≥100, ≥ 500, ≥ 1000 lower than the EC₅₀ value for cells of normal tissues.

In one embodiment, the TA antigenic peptide C is a viral peptide, a bacterial peptide or a tumour associated antigen (TAA) peptide, preferably a tumour associated antigen (TAA) peptide.

In some cases, said antigen binding protein can have an EC₅₀ for TA-C/MHC complex presenting cells that is ≥5 fold, ≥10 fold, ≥20 fold, ≥50 fold, ≥ 100 fold, ≥ 500 fold, ≥ 1000 fold lower than the EC₅₀ value for cells of normal tissues.

In one embodiment, the TA antigenic peptide C is a tumour associated antigen (TAA) peptide C, and said TAA-C is selected from the group of TAA antigenic peptides comprising or consisting of amino acid sequences of SEQ ID NO: 162 to 317, SEQ ID NO: 9 and 10, such as the PRAME antigenic peptide comprising or consisting of the amino acid sequence 'SLLQHLIGL' of SEQ ID NO: 9 or the MAGE-A antigenic peptide comprising or consisting of the amino acid sequence 'KVLEHVVRV' of SEQ ID NO: 10, wherein the MHC is preferably a HLA-A*02.

For example the TA antigenic peptide C can be the MAGE-A antigenic peptide comprising or consisting of the amino acid sequence 'KVLEHVVRV' of SEQ ID NO: 10 and wherein the similar peptide is selected from the list consisting of RABGAP1L-001, AXIN1-001, ANO5-001, TPX2-001, SYNE3-001, MIA3-001, HERC4-001, PSME2-001, HEATR5A-001, CNOT1-003, TEP1-003, PITPNM3-001, ZFC-001 preferably HEATR5A-001, HERC4-001 and CNOT1-003.

In one aspect, the antigen binding site B comprises an antibody or a fragment thereof or an alpha chain variable domain (v_{α}) and a beta chain variable domain (v_{β}) , or a gamma chain variable domain (v_{γ}) , or a delta chain variable domain (v_{δ}), preferably an alpha chain variable domain (v_{α}) and a beta chain variable domain (v_{β}) or a gamma chain variable domain (v_{γ}) and a delta chain variable domain (v_{δ}), preferably a v_{α} and v_{β}.

In one embodiment thereof, the v_{α} comprises or consists of the amino acid sequence selected from the group consisting of 'EDVEQSLFLSVREGDSVVINCTYTDSSSTYLYWYKQEPGKGLQLLTYIYSSQDSKQDQRLT VLLNKKDKHLSLRIADTQTGDSAIYFCAEMTSESKIIFGSGTRLSIRP' SEQ ID NO: 20, 'EDVEQSLFLSVREGDSVVINCTYTDSSSTYLYWYKQEPGKGLQLLTYIYSSQDQKQDQRLT VLLNKKDKHLSLRIADTQTGDSAIYFCAEMTSESKIIFGSGTRLSIRP' 'SEQ ID NO: 21, 'EDVEQSLFLSVREGDSVVINCTYTESSSTYLYWYKQEPGKGLQLLTYIYSSQDQKQDQRLT VLLNKKDKHLSLRIADTQTGDSAIYFCAEMTSESKIIFGSGTRLSIRP' SEQ ID NO: 22 or an amino acid sequence at least 85% identical to the amino acid sequence selected from the group consisting of SEQ ID NO: 20, 21 and 22 and wherein preferably the amino acid sequence at least 85% identical to the amino acid sequence of SEQ ID NO: 20 preferably comprises the amino acid sequence of CDRa1 of SEQ ID NO: 23, CDRa2 of SEQ ID NO: 24 and CDRa3 of SEQ ID NO: 25, and wherein preferably the amino acid sequence at least 85% identical to the amino acid sequence of SEQ ID NO: 21 preferably comprises the amino acid sequence of CDRa1 of SEQ ID NO: 23, CDRa2 of SEQ ID NO: 26 and CDRa3 of SEQ ID NO: 25, and wherein preferably the amino acid sequence at least 85% identical to the amino acid sequence of SEQ ID NO: 22 preferably comprises the amino acid sequence of CDRa1 of SEQ ID NO: 27, CDRa2 of SEQ ID NO: 26 and CDRa3 of SEQ ID NO: 25, and wherein the amino acid of said first variable domain preferably comprises the amino acids 19V and/or 48K, and the v_{β} comprises or consists of the amino acid sequence 'DAGVIQSPRHEVTEMGQEVTLRCKPIPGHDYLFWYRQTMMRGLELLFYFCYGTPCDDSGM PEDRFSAKMPNASFSTLKIQPSEPRDSAVYFCASRADTGELFFGEGSRLTVL' SEQ ID NO: 30 or an amino acid sequence at least 85% identical to the amino acid sequence consisting of SEQ ID NO 30, and wherein preferably the amino acid sequence at least 85% identical to the amino acid sequence of SEQ ID NO: 30 preferably comprises the amino acid sequence of CDRb1 of SEQ ID NO: 31, CDRb2 of SEQ ID NO: 34 and CDRb3 of SEQ ID NO: 35, respectively, and optionally comprises the amino acid 54F and/or 66C.

In another embodiment thereof, the v_{α} or v_{γ} comprises or consists of the amino acid sequence SEQ ID NO: 48 or an amino acid sequence at least 85% identical to the amino acid sequence of SEQ ID NO: 48, wherein preferably an amino acid sequence at least 85% identical to the amino acid sequence of SEQ ID NO: 48 comprises the amino acid sequence of CDRa1 of SEQ ID NO: 49, CDRa2 of SEQ ID NO: 50 and CDRa3 of SEQ ID NO: 51 and the v_{β} or v_{δ} comprises or consists of the amino acid sequence of SEQ ID NO: 44 or an amino acid sequence at least 85% identical to SEQ ID NO: 44 wherein preferably said amino acid sequence at least 85% identical to the amino acid sequence of SEQ ID NO: 44 comprises the amino acid sequence of CDRb1 of SEQ ID NO: 45, CDRb2 of SEQ ID NO: 46 and CDRb3 of SEQ ID NO: 47.

For example, the antigen binding protein may comprise a first polypeptide of formula V₃-L₁-V₄-L₂-C_{L}-L₅-F_{c1} [III] comprising or consisting of the amino acid sequence of SEQ ID NO: 282 or 284 and the second polypeptide of formula V₅-L₃-V₆-L₄-CH₁-L₆-F_{c2} [IV] comprising or consisting of the amino acid sequence of SEQ ID NO: 283.

The bispecific antigen binding protein may further comprise one or more of the following:
(i) a diagnostic agent;
(ii) a therapeutic agent; or
(iii) a pharmacokinetics (PK) modifying moiety.

A further object of the invention is an isolated nucleic acid comprising a sequence encoding for a bispecific antigen binding protein as defined above, or a nucleic acid vector comprising said nucleic acid.

A further object of the invention is a recombinant host cell comprising , a bispecific antigen binding protein as defined in the above mentioned aspects and items, or a nucleic acid or a vector as defined above wherein said host cell preferably is a) a stem cell, preferably a mesenchymal stem cell or b) a cell for recombinant expression, such as a Chinese Hamster Ovary (CHO) cell.

A further object of the invention is a pharmaceutical composition comprising the aforementioned bispecific binding protein, or the aforementioned nucleic acid or vector, and a pharmaceutically acceptable carrier, diluent, stabilizer and/or excipient.

A further object of the inventino is a method of producing the aforementioned bispecific antigen binding protein comprising
a. providing a suitable host cell,
b. providing a genetic construct comprising a coding sequence encoding the bispecific antigen binding protein as defined in the above mentioned aspects and items,
c. introducing said genetic construct into said suitable host cell, and
d. expressing said genetic construct by said suitable host cell.

The method may further be followed by the isolation and purification of the bispecific antigen binding protein from the suitable host cell.

In a further aspec of the invention, the aforementioned bispecific antigen binding protein, or nucleic acid or vector or pharmaceutical composition, are for use in medicine. for use in medicine.

In particular, they can be for use in a method of treating a proliferative disease, like cancer, and more particularly a TAA/MHC positive cancer.

The following examples and accompanying figures illustrate the invention but should not be taken to limit the scope of the claims.

### DESCRIPTION OF THE FIGURES

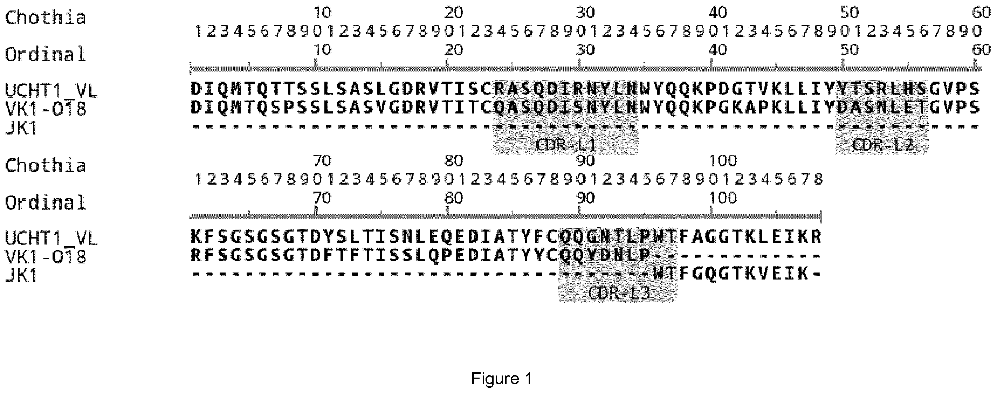

**Figure 1** shows the alignment of UCHT1 VL domain to the identified human acceptor framework. CDRs as identified in the figure are identified according to Cothia definitions.

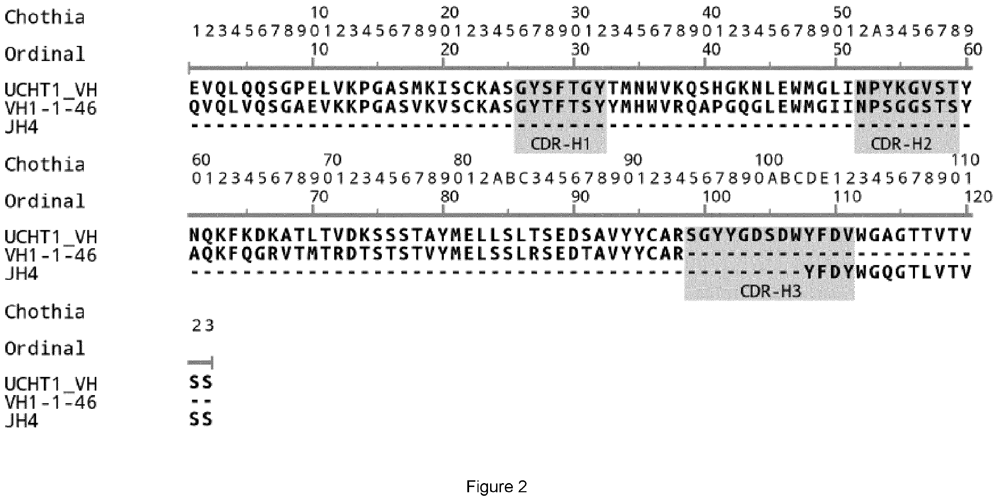

**Figure 2** shows the alignment of UCHT1 VH domain to the identified human acceptor framework. CDRs as identified in the figure are identified according to Cothia definitions.
**Figure 3** shows concentration-dependent binding of PRAME-004-specific TCERO-molecules to Jurkat cells as measured by flow cytometry.

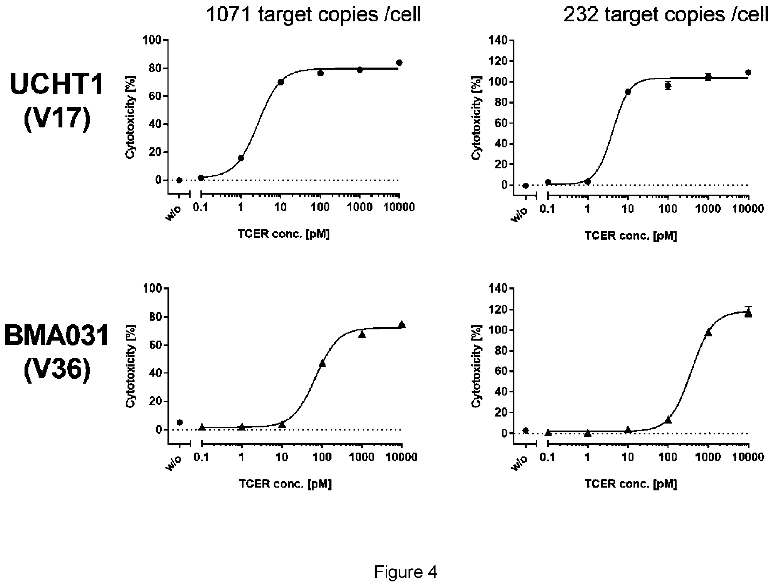

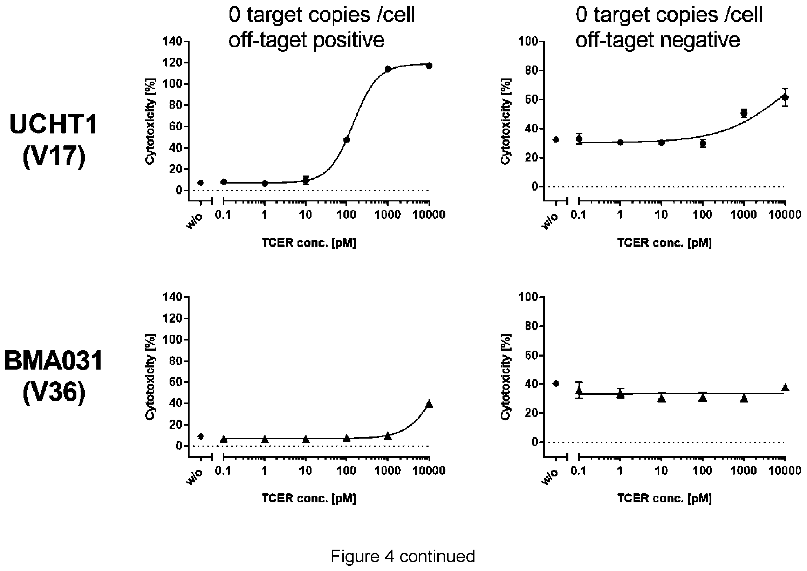

**Figure 4** shows the results of a representative LDH-release assays using PBMC of a healthy HLA-A*02-positive donor. MAG-003-specific TCERO-molecules employing UCHT1(V17) or BMA031(V36) were tested on MAG-003-positive and MAG-003-negative as well as Off-target-positive tumor cell lines. Tested cell lines (from left to right): H695T, A375, T98G, BV173. Error bars depict standard deviations within triplicates.
**Figure 5** shows the results of LDH-release assays on healthy cells incubated with MAG-003-specific TCER^{®} based on UCHT1(V17). Each cytotoxicity plot shows LDH-release of a primary healthy cell type (empty circles) in comparison to the control tumor cell line Hs695T (filled circles) in the same medium combination after co-incubation with PBMCs and increasing concentrations of the TCER molecule. Calculated safety windows based on EC₅₀-values are also depicted.
**Figure 6** shows the results of LDH-release assays on healthy cells incubated with MAG-003-specific TCER^{®} based on BMA031(V36). Each cytotoxicity plot shows LDH-release of a primary healthy cell type (empty circles) in comparison to the control tumor cell line Hs695T (filled circles) in the same medium combination after co-incubation with PBMCs and increasing concentrations of the TCER molecule. Calculated safety windows based on either EC₅₀-values or based on LOEL are also depicted.

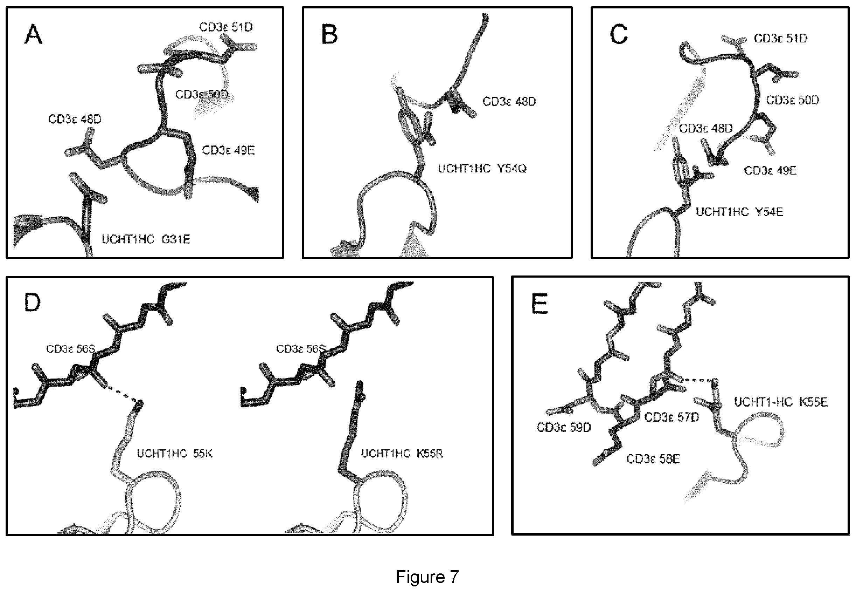

**Figure 7** shows the visualization of the introduced point mutations. Pane A shows the newly introduced Asp side chain in position 31 of the UCHT1 heavy chain and the negatively charged side chains of CD3ε located in its spatial proximity. Pane B shows both, the wt Tyr and the replacing GIn in position 54 of the UCHT1 heavy chain. Replacement of the aromatic side chain with a smaller, polar amino acid removes the hydrophobic interaction with the apolar stem of the Asp in CD3ε's position 48. Pane C shows replacement of the same Tyr in position 54 of the heavy chain of UCHT1 by Glu and the negatively charged side chains on CD3ε (48D, 49E, 50D, 51D) located in spatial proximity that are likely to cause electrostatic repulsion of the newly introduced Glu on UCHT1. Pane D shows the wt Lys in UCHT1 heavy chain position 55 forming a hydrogen bond (dashed line) with the backbone of a Ser in CD3ε`s position 56 (left). Replacement of said Lys by Arg removes the polar interaction and introduces further bulk, forcing the Arg side chain into a strained rotamer (right). Pane E shows the same Lys in position 55 of UCHT1's heavy chain and its replacement by Glu; this mutation also removes the H-bond formed between Lys and the Ser in position 56 of CD3ε. Additionally, it introduces a negatively charged side chain in spatial proximity of a negatively charged patch on the surface of CD3ε that is formed by Asp 48, Glu 49, Asp 50, and Asp 51.
**Figure** 8 shows a summary of production yields and stability characteristics of TCER^{®}-molecules based on humanized UCHT1-variants. (na) not applicable, (nd) not done.
**Figure 9A** shows binding curves of TCER^{®} antigen binding proteins comprising different UCHT1 variants for MAG-003 in complex with HLA-A*02 as measured by biolayer interferometry. Increasing concentrations of TCER^{®} molecules in solution were applied and are given in nM.
**Figure 9B** shows binding curves of TCER^{®} antigen binding proteins comprising different UCHT1 variants for CD3δε-Fc as measured by biolayer interferometry. Increasing concentrations of TCER^{®} molecules in solution were applied and are given in nM.
**Figure** 10 shows the results of two independent LDH-release assays using PBMC of two healthy HLA-A*02-positive donors (HBC-982 and HBC-720). MAG-003-specific TCER^{®}-molecules employing UCHT1-variants with various affinities were tested on MAG-003-positive tumor cell lines. Error bars depict standard deviations within triplicates.

### EXAMPLES

### Example 1: Humanization of mouse monoclonal Ab UCHT1

Humanization of the mouse monoclonal antibody UCHT1 was performed by CDR-grafting according to published methods. Therefore, CDRs of VH and VL were identified according to the Cothia definitions. Sequence alignments comparing UCHT1 variable domains to the human germlines were generated. Based on overall sequence identity, matching interface positions and similarly classed CDR canonical positions, a germline was identified for each of the light and heavy chains as the most promising Acceptor frameworks, VK1-O18 for the light chain and VH-1-46 for the heavy chain. The J-segment genes were compared to the Parental sequence over FR4 and J-segments JK1 and JH4 were selected for the light and heavy chain respectively.

A list of all the positions with differing residues between the Parental and Acceptors framework was generated. All positions were analysed and considered both in isolation and in the context of other potential substitutions. Each position was ranked as Neutral, Critical or Contributing and a suggestion about which residues to substitute and evaluate in humanised variants was made. Potential humanised variant sequences were screened using Epibase^{™} (Lonza). Each epitope or cluster of epitopes was analysed for substitutions that would either remove the epitope or further reduce the predicted immunogenicity. Further on potential sites of post-translational modifications within the CDRs were identified and respective remediations were proposed. In total this resulted in the generation of four different VH and five different VL-domains. Therefore, 17 humanization variants of UCHT1 were generated. All variants were expressed in CHO-cells as Fab-molecules. Expressed proteins were purified and ranked according to expression titer, aggregate levels and the EC₅₀-values of binding to Jurkat cells. Based on these results the humanized UCHT1(V17), defined by SEQ ID No: 137 and SEQ ID No: 145, was selected for establishment of the inventors TCER^{®}-molecules.

PRAME-004 (SEQ ID No: 9) targeting TCER^{®}-molecules were constructed utilizing either the recruiting domains of humanized UCHT1(V17) resulting in molecules containing SEQ ID No: 171 and SEQ ID No. 170 or humanized BMA031(V10) resulting in SEQ ID No: 168 and SEQ ID No: 169, respectively. Vectors for the expression of recombinant proteins were designed as mono-cistronic, controlled by HCMV-derived promoter elements, pUC19-derivatives. Plasmid DNA was amplified in E.coli according to standard culture methods and subsequently purified using commercial-available kits (Macherey & Nagel). Purified plasmid DNA was used for transient transfection of CHO-S cells according to instructions of the manufacturer (ExpiCHO^{™} system; Thermo Fisher Scientific). Transfected CHO-cells were cultured for 6-14 days at 32°C to 37°C and received one to two feeds of ExpiCHO^{™} Feed solution.

Conditioned cell supernatant was harvested by centrifugation (4000 x *g;* 30 minutes) and cleared by filtration (0.22 µm). Bispecific molecules were purified using an Äkta Pure 25 L FPLC system (GE Lifesciences) equipped to perform affinity and size-exclusion chromatography in line. Affinity chromatography was performed on protein A columns (GE Lifesciences) following standard affinity chromatographic protocols. Size exclusion chromatography was performed directly after elution (pH 2.8) from the affinity column to obtain highly pure monomeric protein using Superdex 200 pg 16/600 columns (GE Lifesciences) following standard protocols. Protein concentrations were determined on a NanoDrop system (Thermo Scientific) using calculated extinction coefficients according to predicted protein sequences. Concentration, if needed, and buffer exchange was performed using Vivaspin devices (Sartorius). Finally, purified molecules were stored in phosphate-buffered saline at concentrations of about 1 mg/mL at temperatures of 2-8°C.

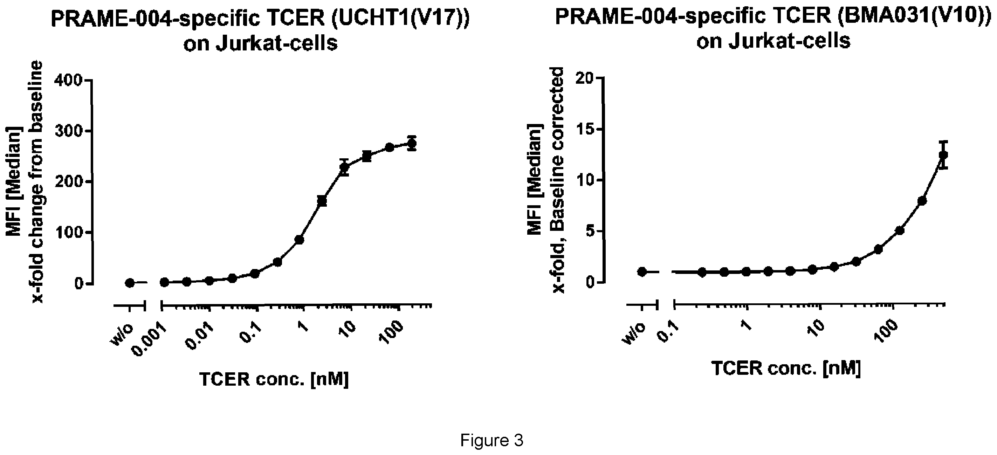

Binding affinity of these TCERO-molecules towards effector cells was assessed by flow cytometry. Therefore, Jurkat cells (CD3+ and TCRab+) were incubated with raising concentrations of TCER^{®}.After washing the cells bound TCER^{®}-molecules were stained using a PE-labeled secondary reagent (#709-116-098, Jackson ImmunoResearch). Cells were finally analyzed on an Intellicyt^{®} iQue Cell Screener. Results of one of four independent experiments are shown in Figure 3 demonstrating the concentration-dependent binding of PRAME-004-specific TCERO-molecules to Jurkat cells. It is obvious that the UCHT1-based TCERO-molecule shows an EC₅₀ of binding of around 2-3 nM, whereas BMA031-based TCER^{®} molecules exhibit a at least 50-100-fold weaker binding towards the Jurkat cells.

### Example 2: Proof of principle Cytotoxicity using recruiters having a different affinity

Antigen-binding proteins targeting the peptide MAG-003 (SEQ ID No: 10) were generated by the combination of engineered variable domains of a T Cell Receptor (SEQ ID No: 20 and SEQ ID No: 30) with the variable domains of either UCHT1(V17) (SEQ ID No: 137 and SEQ ID No: 145) or BMA031(V36) (SEQ ID No: 42 and SEQ ID No. 43), respectively, within the TCER^{®} construct.

Vectors for the expression of the respective TCERO-molecuels were designed as mono-cistronic, controlled by HCMV-derived promoter elements, pUC19-derivatives. Plasmid DNA was amplified in E.coli according to standard culture methods and subsequently purified using commercial-available kits (Macherey & Nagel). Purified plasmid DNA was used for transient transfection of CHO-S cells utilizing an electroporation systems (MaxCyte STX). Transfected CHO-cells were cultured 10-12 days at 32°C to 37°C and received one to three feeds of Cellboost 7a and 7b (GE Healthcare^{™}) solution.

Conditioned cell supernatant was cleared by filtration (0.22 µm) utilizing Sartoclear Dynamics^{®} Lab Filter Aid (Sartorius). Bispecific antigen binding proteins were purified using an Äkta Pure 25 L FPLC system (GE Lifesciences) equipped to perform affinity and size-exclusion chromatography in line. Affinity chromatography was performed on MAbSelect SuRE or protein L columns (GE Lifesciences) following standard affinity chromatographic protocols. Size exclusion chromatography was performed directly after elution (pH 2.8) from the affinity column to obtain highly pure monomeric protein using, Superdex 200 pg 26/600 columns (GE Lifesciences) following standard protocols. Protein concentrations were determined on a NanoDrop system (Thermo Scientific) using calculated extinction coefficients according to predicted protein sequences. Concentration was adjusted, if needed, by using Vivaspin devices (Sartorius). Finally, purified molecules were stored in phosphate-buffered saline at concentrations of about 1 mg/mL at temperatures of 2-8°C.

The cytotoxic activity of the bispecific molecules against MAG-positive and MAG-negative tumor cell lines, respectively was analyzed by LDH-release assay. Therefore, tumor cell lines presenting variable amounts of HLA-A*02/MAG-003 on the cell surface were co-incubated with PBMC isolated from healthy donors (HLA-A*02+) in presence of increasing concentrations of TCER^{®} molecules. After 48 hours, lysis of target cell lines was measured utilizing CytoTox 96 Non-Radioactive Cytotoxicity Assay Kits (PROMEGA).

Exemplary results of such assays are shown in Figure (Example 2). The resulting EC₅₀-values are summarized in Table 5.

**Table 5: Summary of EC₅₀-values of killing assays comparing different recruiting antibodies.**

| **Recruiter** | **EC₅₀ on Target High** | **EC₅₀ on Target low** | **EC₅₀ on Off-target positive** | **EC₅₀ on Negative** |
|---|---|---|---|---|
| UCHT1 (V17) | 3 pM | 4 pM | 146 pM | 11559 pM |
| BMA031 (V36) | 69 pM | 383 pM | 21555 pM | n/a |

These results reveal the reduced potency of the BMA031(V36)-based TCER^{®}-molecule compared to the UCHT1(V17)-based molecule (23 -fold on target high expressing cell line and 96-fold on target low expressing cell lines, respectively). Based on the EC₅₀-values the safety window can be calculated as described above in the definitions-section. Briefly, safety windows are defined as ratios of EC₅₀ of killing of Off-target expressing cells and EC50 of killing of target (TAA)-expressing cells. This implies that the UCHT1(V17)-based TCER^{®} exhibits a safety window of approx. 49-fold, whereas the BMA031(V36)-based TCER^{®} shows an increased safety window of approx. 312-fold (comparison of off-target expressing tumor cell line to target (TAA) high expressing tumor cell line).

These findings suggested that in general the utilization of low-affinity recruiting domains may improve discrimination between target and off-target thereby increasing the safety window. For further verification of this hypothesis the cytotoxic activity of the MAG-003-specific TCER^{®}-molecules towards primary healthy tissue cells (HLA-A*02+) was assessed. To this end LDH was determined in co-cultures of eleven different primary healthy tissue cells (HLA-A*02+) with PBMC effector cells from healthy HLA-A*02+ donors at an E:T ratio of 10:1 and increasing TCER^{®} concentrations. Cells were co-incubated in a 50% mixture of primary tissue cell-specific medium and optimal T cell medium. To determine a safety window, the TCER^{®} molecules were co-incubated in an identical setup with the MAG-003-positive tumor cell line Hs695T in the respective medium combination of the primary cells as well as 100% optimal T cell medium to exclude a bias caused by the different media. After 48h of co-culture, supernatants were harvested and cell lysis was analyzed by measuring LDH-release using the LDH-Glo^{™} Kit (Promega).

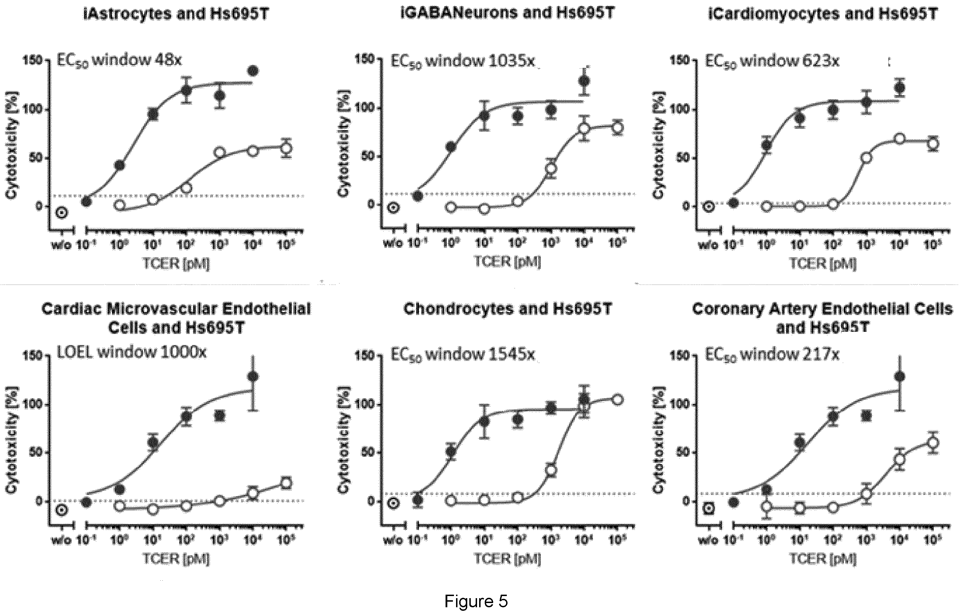

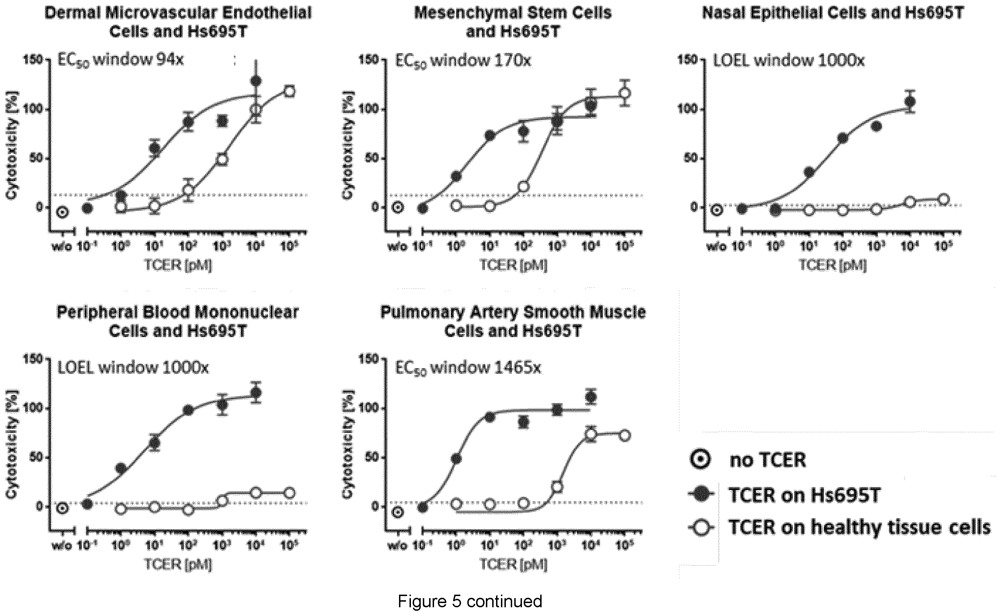

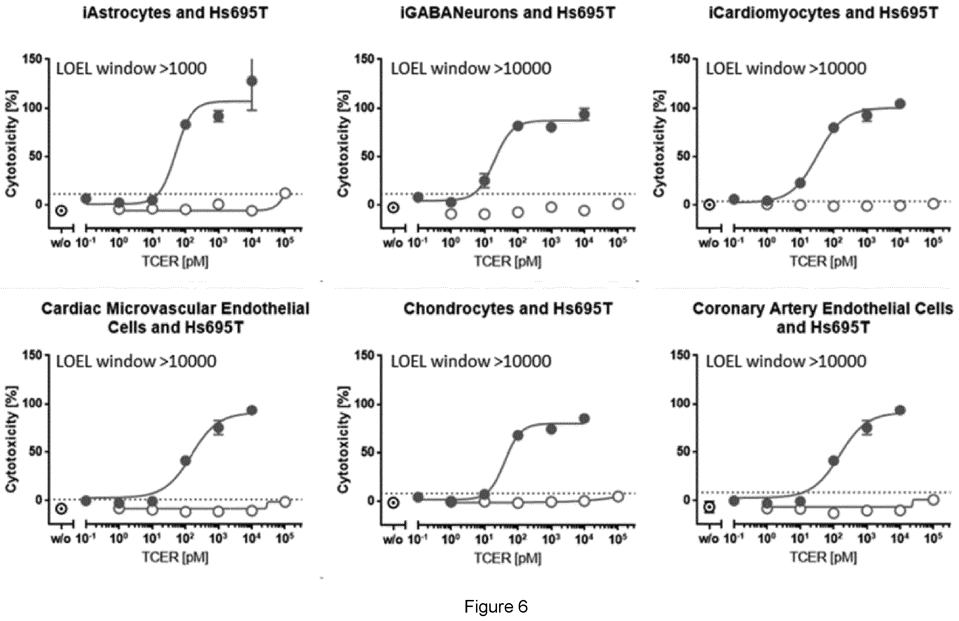

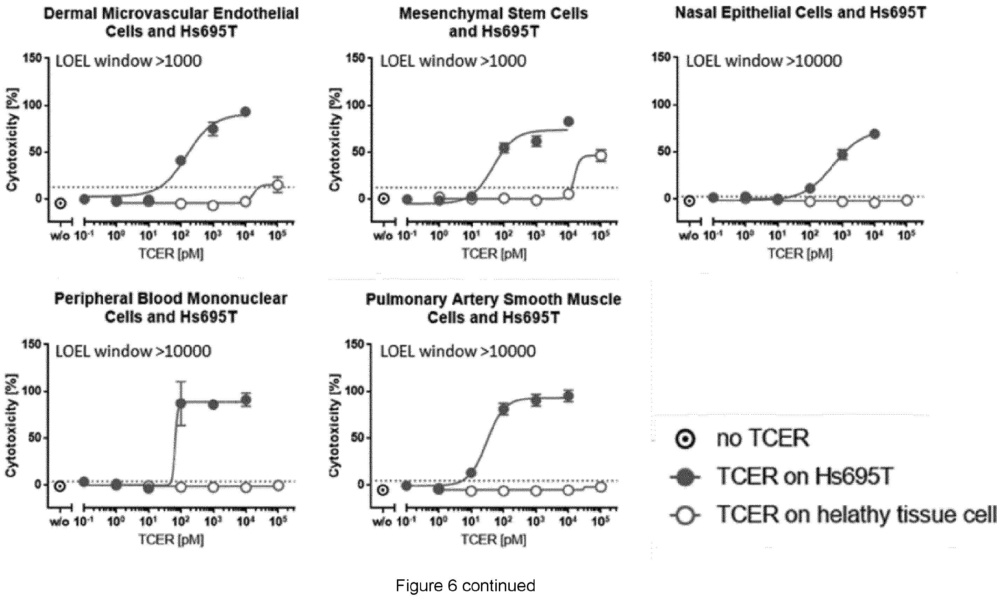

In Figure 5 and Figure 6, each cytotoxicity plot shows LDH-release of a primary healthy cell type (empty circles) in comparison to the control tumor cell line Hs695T (filled circles) in the same medium combination after co-incubation with PBMCs and increasing concentrations of the TCER molecule. Figure 5 summarizes the results of the UCHT1(V17)-based MAG-003-specific TCER^{®}-molecule. For all tested cell types, with exception of nasal epithelial cells and PBMC, strong reactivities were detectable and respective EC₅₀-values could be determined. Based on EC₅₀-values safety windows were calculated as described above within the definitions section. Safety windows in x-fold are noted within the figure. For the UCHT1(V17)-based TCER^{®}. The most critical safety windows were determined for iAstrocytes (48-fold), dermal microvascular endothelial cells (94-fold) and mesenchymal stem cells (170-fold).

In case of the BMA031(V36)-based TCER^{®} molecule all responses against healthy primary cells were too low to calculate an EC₅₀. Instead, we defined the safety window based on the lowest observed effect level (LOEL) determined as the first TCER^{®} concentration with a response over cut-off value. The cut-off was defined as $\left(\left[standard deviation from all triplicates\times 3\right]+\left[w/o TCER control\right]\right)$ (w/o TCER-control is indicated as dotted line in each cytotoxicity plot) was used as threshold to determine the LOEL and safety window between healthy tissue cells and tumor control cell line. All determined safety windows of the BMA031(V36)-based TCER^{®} were greater than 1000-fold.

It is obvious from comparison of Figure 5 and Figure 6 that the utilization of the lower-affinity recruiter BMA031(V36) within the context of the MAG-003-specific TCERO-molecule results in significant expanded safety windows.

### Example 3: Creation of Affinity reduced humanized UCHT1 variants

To obtain lower-affinity variants of the CD3-specific humanized antibody UCHT1(V17) structure-guided design of variants was performed. Based on the solved structure of UCHT1 in complex with its target CD3δ/ε (PDB ID: 1xiw), point mutations were introduced on the antibody that were assumed to lower the affinity without destabilizing the protein itself.

In order to achieve this goal, positions were selected primarily within the CDRs; as can be inferred from the solved structure, the interface between the two proteins is formed primarily between CD3 ε and the antibody heavy chain, therefore only positions within the heavy chain are considered for mutations.

For clarification, positions on CD3ε are numbered sequentially as in the PDB entry with ID 1xiw, chain ID: A.

G31E introduces a negative charge on the surface of the antibody that faces a negatively charged surface patch formed by CD3ε48D, CD3ε49E, CD3ε50D, and CD3ε51D, presumably causing electrostatic repulsion and thus lowering affinity.

Y54Q alters shape complementarity of the binding surfaces and removes the hydrophobic interaction between Y54's aromatic ring and the apolar stem of CD3ε48D.

Y54E alters shape complementarity of the binding surfaces and removes the hydrophobic interaction between Y54's aromatic ring and the apolar stem of CD3ε48D and additionally introducing a negative charge facing the negatively charged patch formed by CD3ε48D, CD3ε49E, CD3ε50D, and CD3ε51D.

K55R introduces a bulkier side chain with similar physicochemical properties, removing the H-bond formed between 55K's Nζ and the backbone of CD3ε56S. The increased size of the side chain might also cause a slight change in binding geometry.

K55E replaces a positive charge with a negative charge, removing the H-bond formed between 55K's Nζ and the backbone of CD3ε36S. Additionally, introduction of the negative charge causes electrostatic repulsion from the negatively charged patch formed by CD3ε57D, CD3ε58E, and CD3ε59D.

Based on these findings sequences coding for UCHT1(V20) to UCHT1(V27) were generated as summarized in Table 6.

In a further attempt to optimize the humanized UCHT1-sequences a potential post-translational modification site (Asp-Isomerization, 106D107S) within CDR-H3 was removed by introduction of 106E. This modification was introduced in UCHT1(V17), UCHT1(20), UCHT1(V21) and UCHT1(V23) resulting in the variants UCHT1(V17opt), UCHT1(V20opt), UCHT1(V21opt) and UCHT1(V23opt), respectively.

**Table 6: Combination of sequences resulting in humanized UCHT1-variants.**

| **UCHT-variant** | **SEQ ID No. [VH]** | **SEQ ID No. [VL]** |
|---|---|---|
| V20 | 158 | 145 |
| V21 | 149 | 145 |
| V22 | 150 | 145 |
| V23 | 151 | 145 |
| V24 | 152 | 145 |
| V25 | 153 | 145 |
| V26 | 154 | 145 |
| V27 | 155 | 145 |
| V17opt | 156 | 145 |
| V20opt | 159 | 145 |
| V21opt | 157 | 145 |
| V23opt | 160 | 145 |

Using the humanized UCHT1-variants described in Table 6 PRAME-004-specific (Vα: SEQ ID No: 48, Vβ: SEQ ID No: 44) and MAG-003-specific (Vα: SEQ ID No: 21, Vβ: SEQ ID No: 30), respectively, TCER^{®} molecules were generated, produced and purified as described above.

### Example 4: Affinity-determination of designed UCHT1-variants

For affinity determination using biolayer-interferometry the molecule CD3δε-Fc was generated. Therefore, the extracellular domains of human CD3δ and CD3ε were fused to the N-terminus of Fc-domains as utilized within the TCER^{®}-constructs (containing Knob-into-hole mutations and an additional C-terminal His-Tag) resulting in SEQ ID No: 161 and SEQ ID No. 162, respectively.

CD3δε-Fc-molecules were expressed in ExpiCHO cells and purified using Protein A affinity chromatography followed by size exclusion chromatography as described above.

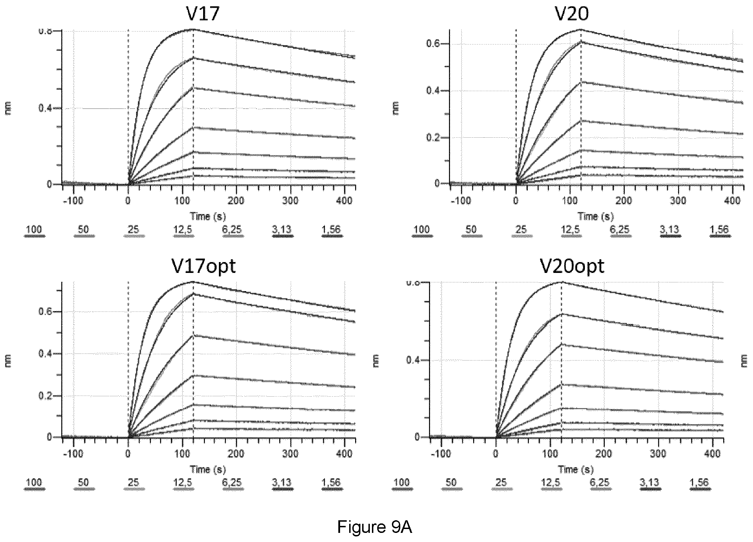

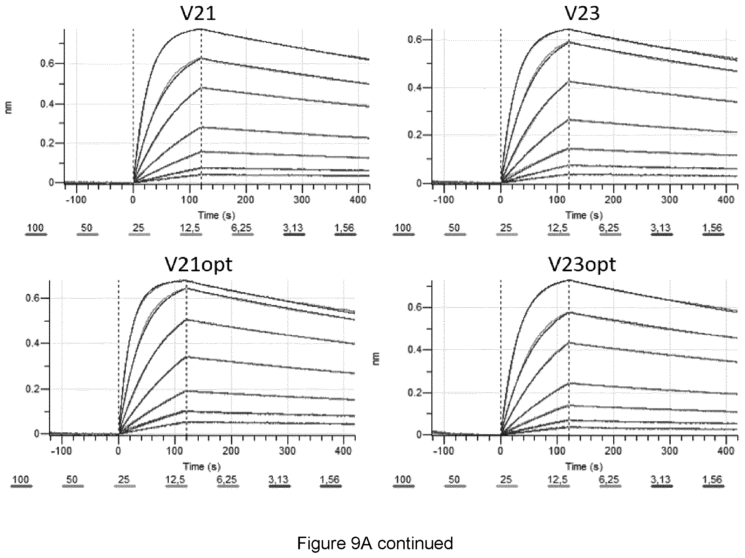

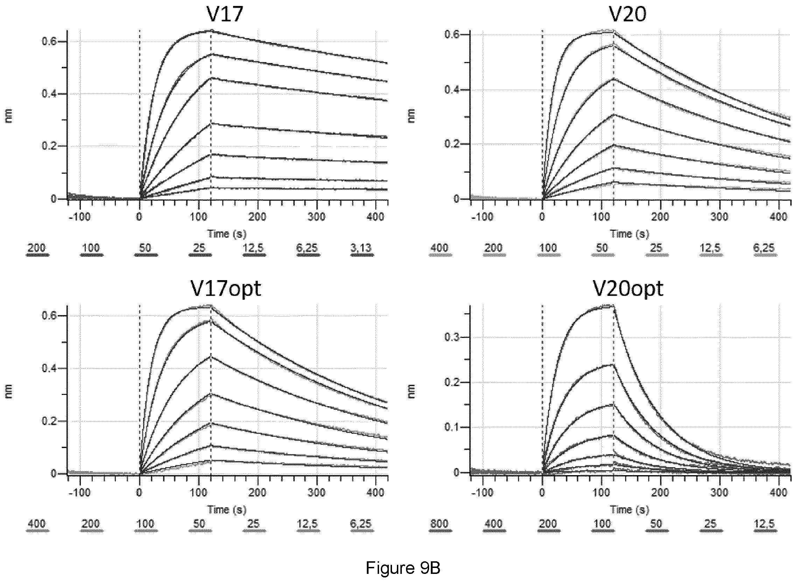

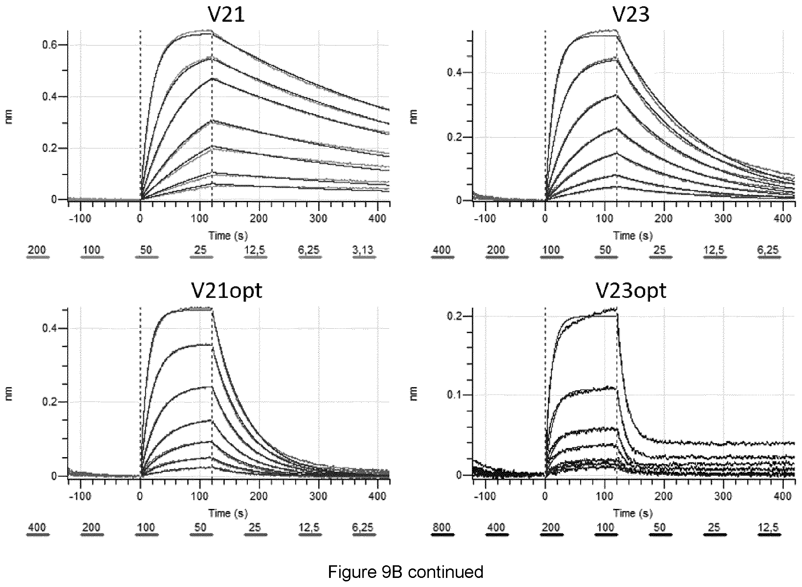

Using biolayer interferometry, bispecific TCER^{®} antigen binding proteins comprising different UCHT1 variants (as shown in Table 6) were characterized for their binding affinity towards the MAGE-A antigenic peptide (SEQ ID NO: 10) in complex with HLA-A*02 (Figure 9A, Table 7) and CD3δε-Fc (Figure 9B, Table 7). Measurements were performed on an Octet RED384 system using settings recommended by the manufacturer. Briefly, binding kinetics were measured at 30°C and 1000 rpm shake speed using PBS, 0.05% Tween-20, 0.1% BSA as buffer. Peptide-HLA-A*02 complex or CD3δε-Fc were loaded onto biosensors (HIS1K) prior to analyzing serial dilutions of the bispecific TCER^{®} molecules. All TCER^{®} molecules showed similar binding to HLA-A*02/MAG-003 with K_{D} values of ~2 nM. CD3δε affinities covered a more than 200-fold window with K_{D} values ranging from 3 to 750 nM.

**Table 7: Affinity analysis of TCER^{®} molecules comprising different UCHT1 variants according to Table 6. K_{D} values were measured by biolayer interferometry**

| **UCHT-variant** | K_{D} HLA-A*02/MAG-003 [nM] | K_{D} CD3δε-Fc [nM] |
|---|---|---|
| V17 | 1.9 | 3.4 |
| V20 | 2.0 | 17.8 |
| V21 | 2.3 | 7.9 |
| V23 | 2.1 | 46.1 |
| V17opt | 2.0 | 22.6 |
| V20opt | 1.7 | 373.0 |
| V21opt | 2.1 | 106.4 |
| V23opt | 2.4 | 747.1 |

### Example 5: Reduced potency of low affinity humanized UCHT1 variants

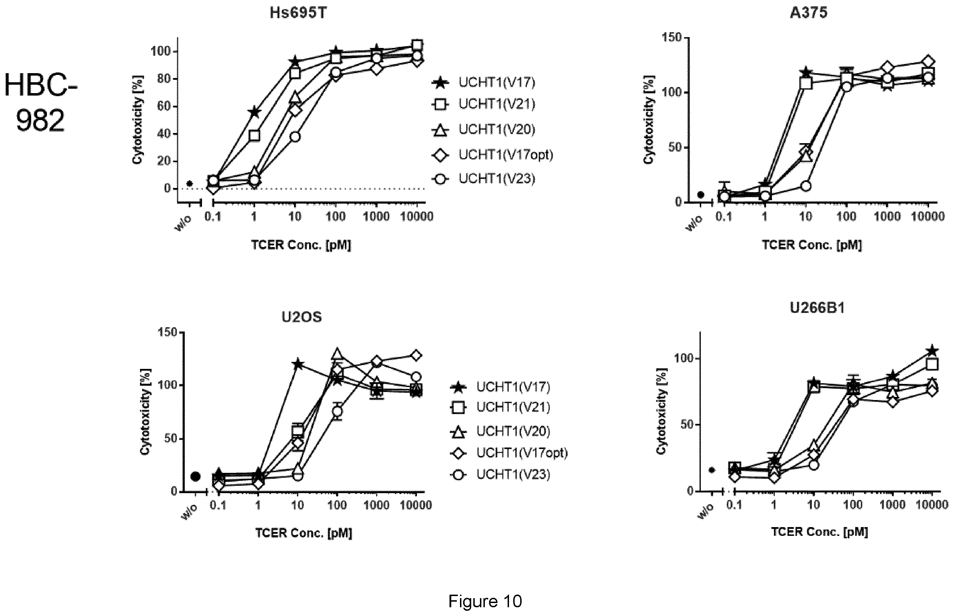

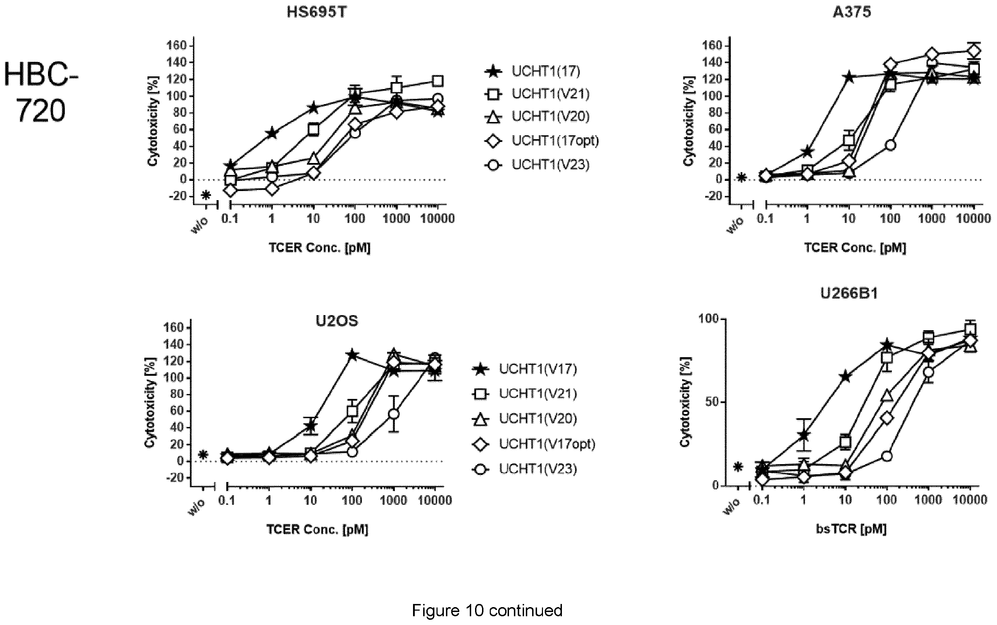

Potency with respect to cytotoxicity of the MAG-003-specific TCER^{®}-molecules was assessed in LDH-release assays as described above within example 2. Results of representative assays are shown in Figure 10. As expected, the newly designed variants (UCHT1(V17opt), UCHT1(V20), UCHT1(V21), UCHT1(V23)) showed reduced potency in comparison to the TCER^{®} containing high affinity recruiting domains of UCHT1(V17). Ranking the TCER^{®}-molecules according to their potencies also closely reflects the affinities of the respective recruiter variants (highest potency: UCHT1(V17) < UCHT1(V21) < UCHT1(V20) < UCHT1(V17opt) < UCHT1V23). These effects on potencies could solely be attributed to the recruiting domains as the affinities of the TCR-domains towards MAG-003 in complex with HLA-A*02 are comparable (Figure 9).

## Claims

1. A bispecific antigen binding protein comprising at least two antigen binding sites B and D, wherein the antigen binding site D binds to T cell receptor (TCR) alpha/beta and wherein the antigen binding site B binds to a target antigenic (TA) peptide/MHC complex, wherein the antigen binding site D comprises a heavy chain variable domain (V_{H}) and a light chain variable domain (V_{L}), and wherein
(i) said V_{L} comprises or consists of the amino acid sequence of SEQ ID NO: 42, and
(ii) said V_{H} comprises or consists of the amino acid sequence of SEQ ID NO: 43.

2. The bispecific antigen binding protein according to claim 1, wherein the TA peptide is a TA peptide C (TA-C), and wherein the antigen binding site B binds to the TA-C/MHC complex with a K_{D}(C) which is ≤ 100µM, ≤ 1µM ≤ 100nM, ≤ 50nM, ≤ 10nM, for instance 0.01nM to 150nM, 0.05nM to 150nM, 0.1nM to 150nM, 0.1nM to 100nM, 0.1nM to 50nM, 0.1nM to 10nM, 0.5nM to 10nM, 0.5nM to 5nM as determined using Surface Plasmon Resonance (SPR) or Biolayer Interferometry (BLI).

3. The bispecific antigen binding protein according to claim 1 or 2, wherein the TA peptide is a TA-C, and wherein said TA-C is a viral peptide, a bacterial peptide or a tumour associated antigen (TAA) peptide.

4. The bispecific antigen binding protein according to any of claims 1 to 3, wherein the TA peptide is a TA-C, wherein said TA-C is a tumour associated antigen (TAA) peptide C (TAA-C), and wherein said TAA-C is selected from the group of TAA antigenic peptides comprising or consisting of amino acid sequences of SEQ ID NO: 162 to 317, SEQ ID NO: 9 and 10, wherein the MHC is preferably a HLA-A*02.

5. The bispecific antigen binding protein according to any one of claims 1 to 4, wherein the antigen binding site B comprises an antibody or (a) fragment(s) thereof; or a TCR alpha chain variable domain (vα) and a TCR beta chain variable domain (vβ); or a TCR gamma chain variable domain (vγ) or a TCR delta chain variable domain (vδ).

6. The bispecific antigen binding protein according to any of claims 1 to 5, further comprising one or more of the following:
(i) a diagnostic agent;
(ii) a therapeutic agent; or
(iii) a pharmacokinetics (PK) modifying moiety.

7. An isolated nucleic acid comprising a sequence encoding an antigen binding protein according to any one of claims 1 to 6 or a nucleic acid vector comprising said nucleic acid.

8. A recombinant host cell comprising the antigen binding protein according to any one of claims 1 to 6, the nucleic acid or the nucleic acid vector according to claim 7.

9. A pharmaceutical composition comprising the antigen binding protein according to any one of claims 1 to 6, the nucleic acid or the nucleic acid vector according to claim 7, and a pharmaceutically acceptable carrier, diluent, stabilizer and/or excipient.

10. The pharmaceutical composition according to claim 9, wherein the antigen binding protein is dissolved or dispersed in a pharmaceutically acceptable carrier or aqueous medium.

11. A method of producing the antigen binding protein according to any one of claims 1 to 6, comprising
a) providing a suitable host cell,
b) providing a genetic construct comprising a coding sequence encoding the antigen binding protein according to any of claims 1 to 6,
c) introducing said genetic construct into said suitable host cell, and
d) expressing said genetic construct by said suitable host cell.

12. The antigen binding protein according to any one of claims 1 to 6, the nucleic acid or the nucleic acid vector according to claim 7or the pharmaceutical composition according to claim 9 or 10 for use in medicine.

13. The antigen binding protein according to any one of claims 1 to 6, the nucleic acid or the nucleic acid vector according to claim 7or the pharmaceutical composition according to claim 9 or 10 for use in the treatment of a proliferative disease.

14. The antigen binding protein, the nucleic acid or the nucleic acid vector or the pharmaceutical composition for use according to claim 13, wherein the proliferative disease is cancer.

## Patentansprüche

1. Bispezifisches antigenbindendes Protein, das mindestens zwei Antigenbindungsstellen B und D umfasst, wobei die Antigenbindungsstelle D an den T-Zell-Rezeptor (TCR) alpha/beta bindet und wobei die Antigenbindungsstelle B an einen Peptid/MHC-Komplex eines Zielantigens (TA) bindet, wobei die Antigenbindungsstelle D eine variable Domäne der schweren Kette (V_{H}) und eine variable Domäne der leichten Kette (V_{L}) umfasst, und wobei
(i) die V_{L} die Aminosäuresequenz von SEQ ID NO: 42 umfasst oder daraus besteht, und
(ii) die V_{H} die Aminosäuresequenz von SEQ ID NO: 43 umfasst oder daraus besteht.

2. Bispezifisches antigenbindendes Protein nach Anspruch 1, wobei das TA-Peptid ein TA-Peptid C (TA-C) ist und wobei die Antigenbindungsstelle B an den TA-C/MHC-Komplex mit einer K_{D}(C) bindet, die ≤ 100 µM, ≤ 1 µM, ≤ 100 nM, ≤ 50 nM, ≤ 10 nM ist, beispielsweise 0,01 nM bis 150 nM, 0,05 nM bis 150 nM, 0,1 nM bis 150 nM, 0,1 nM bis 100 nM, 0,1 nM bis 50 nM, 0,1 nM bis 10 nM, 0,5 nM bis 10 nM, 0,5 nM bis 5 nM, bestimmt mittels Oberflächenplasmonenresonanz (SPR) oder Biolayer-Interferometrie (BLI).

3. Bispezifisches antigenbindendes Protein nach Anspruch 1 oder 2, wobei das TA-Peptid ein TA-C ist und wobei das TA-C ein virales Peptid, ein bakterielles Peptid oder ein Peptid eines tumorassoziierten Antigens (TAA) ist.

4. Bispezifisches antigenbindendes Protein nach einem der Ansprüche 1 bis 3, wobei das TA-Peptid ein TA-C ist, wobei das TA-C ein Peptid C (TAA-C) eines tumorassoziierten Antigens (TAA) ist und wobei das TAA-C ausgewählt ist aus der Gruppe von TAA-Antigen-Peptiden, die Aminosäuresequenzen von SEQ ID NO: 162 bis 317, SEQ ID NO: 9 und 10 umfassen oder daraus bestehen, wobei es sich bei dem MHC vorzugsweise um einen HLA-A*02 handelt.

5. Bispezifisches antigenbindendes Protein nach einem der Ansprüche 1 bis 4, wobei die Antigenbindungsstelle B einen Antikörper oder (ein) Fragment(e) davon umfasst; oder eine variable Domäne der TCR-alpha-Kette (vα) und eine variable Domäne der TCR-beta-Kette (vβ);
oder eine variable Domäne der TCR-gamma-Kette (vγ) oder eine variable Domäne der TCRdelta-Kette (vδ).

6. Bispezifisches antigenbindendes Protein nach einem der Ansprüche 1 bis 5, weiter umfassend eines oder mehrere der folgenden:
(i) ein Diagnostikum;
(ii) ein Therapeutikum; oder
(iii) eine die Pharmakokinetik (PK) modifizierende Einheit.

7. Isolierte Nukleinsäure, umfassend eine Sequenz, die für ein antigenbindendes Protein nach einem der Ansprüche 1 bis 6 kodiert, oder Nukleinsäurevektor, der die Nukleinsäure umfasst.

8. Rekombinante Wirtszelle, umfassend das antigenbindende Protein nach einem der Ansprüche 1 bis 6, die Nukleinsäure oder den Nukleinsäurevektor nach Anspruch 7.

9. Pharmazeutische Zusammensetzung, umfassend das antigenbindende Protein nach einem der Ansprüche 1 bis 6, die Nukleinsäure oder den Nukleinsäurevektor nach Anspruch 7 und einen pharmazeutisch unbedenklichen Träger, Verdünner, Stabilisator und/oder Hilfsstoff.

10. Pharmazeutische Zusammensetzung nach Anspruch 9, wobei das antigenbindende Protein in einem pharmazeutisch unbedenklichen Träger oder wässrigen Medium gelöst oder dispergiert ist.

11. Verfahren zur Herstellung des antigenbindenden Proteins nach einem der Ansprüche 1 bis 6, umfassend
a) Bereitstellen einer geeigneten Wirtszelle,
b) Bereitstellen eines genetischen Konstrukts, das eine kodierende Sequenz umfasst, die für das antigenbindende Protein nach einem der Ansprüche 1 bis 6 kodiert,
c) Einbringen des genetischen Konstrukts in die geeignete Wirtszelle, und
d) Exprimieren des genetischen Konstrukts durch die geeignete Wirtszelle.

12. Antigenbindendes Protein nach einem der Ansprüche 1 bis 6, Nukleinsäure oder Nukleinsäurevektor nach Anspruch 7 oder pharmazeutische Zusammensetzung nach Anspruch 9 oder 10 zur Verwendung in der Medizin.

13. Antigenbindendes Protein nach einem der Ansprüche 1 bis 6, Nukleinsäure oder Nukleinsäurevektor nach Anspruch 7 oder pharmazeutische Zusammensetzung nach Anspruch 9 oder 10 zur Verwendung bei der Behandlung einer proliferativen Krankheit.

14. Antigenbindendes Protein, Nukleinsäure oder Nukleinsäurevektor oder pharmazeutische Zusammensetzung zur Verwendung nach Anspruch 13, wobei es sich bei der proliferativen Krankheit um Krebs handelt.

## Revendications

1. Protéine bispécifique de liaison à un antigène, comprenant au moins deux sites, B et D, de liaison à un antigène, dans laquelle le site D de liaison à un antigène se lie au récepteur des cellules T (TCR) alpha/bêta, et dans laquelle le site B de liaison à un antigène se lie à un complexe peptide antigénique cible (TA)/CMH, dans laquelle le site D de liaison à un antigène comprend un domaine variable de chaîne lourde (V_{H}) et un domaine variable de chaîne légère (V_{L}), et dans laquelle
(i) ledit V_{L} comprend ou consiste en la séquence d'acides aminés de SEQ ID N° : 42, et
(ii) ledit V_{H} comprend ou consiste en la séquence d'acides aminés de SEQ ID N° : 43.

2. Protéine bispécifique de liaison à un antigène selon la revendication 1, dans laquelle le peptide TA est un peptide TA C (TA-C), et dans laquelle le site B de liaison à un antigène se lie au complexe TA-C/CMH avec une constante K_{D}(C) qui est ≤ 100 µM, ≤ 1 µM, ≤ 100 nM, ≤ 50 nM, ≤ 10 nM, par exemple comprise entre 0,01 nM et 150 nM, 0,05 nM et 150 nM, 0,1 nM et 150 nM, 0,1 nM et 100 nM, 0,1 nM et 50 nM, 0,1 nM et 10 nM, 0,5 nM et 10 nM, 0,5 nM et 5 nM, telle que déterminée par résonance plasmonique de surface (SPR) ou par interférométrie de couche biologique (BLI).

3. Protéine bispécifique de liaison à un antigène selon la revendication 1 ou 2, dans laquelle le peptide TA est un TA-C, et dans laquelle ledit TA-C est un peptide viral, un peptide bactérien ou un peptide d'antigène tumoral (TAA).

4. Protéine bispécifique de liaison à un antigène selon l'une quelconque des revendications 1 à 3, dans laquelle le peptide TA est un TA-C, dans laquelle ledit TA-C est un peptide C d'antigène tumoral (TAA-C), et dans laquelle ledit TAA-C est choisi dans le groupe des peptides antigéniques TAA comprenant ou consistant en des séquences d'acides aminés de SEQ ID N° : 162 à 317, SEQ ID N° : 9 et 10, dans laquelle le CMH est de préférence un HLA-A*02.

5. Protéine bispécifique de liaison à un antigène selon l'une quelconque des revendications 1 à 4, dans laquelle le site B de liaison à un antigène comprend un anticorps ou un ou plusieurs fragments de celui-ci ; ou un domaine variable de chaîne alpha du TCR (vα) et un domaine variable de chaîne bêta du TCR (vβ) ; ou un domaine variable de chaîne gamma du TCR (vγ) ou un domaine variable de chaîne delta du TCR (vδ).

6. Protéine bispécifique de liaison à un antigène selon l'une quelconque des revendications 1 à 5, comprenant en outre un ou plusieurs des éléments suivants :
(i) un agent diagnostique ;
(ii) un agent thérapeutique ; ou
(iii) un groupement fonctionnel modifiant la pharmacocinétique (PK).

7. Acide nucléique isolé comprenant une séquence codant pour une protéine de liaison à un antigène selon l'une quelconque des revendications 1 à 6, ou vecteur d'acide nucléique comprenant ledit acide nucléique.

8. Cellule hôte recombinante comprenant la protéine de liaison à un antigène selon l'une quelconque des revendications 1 à 6, l'acide nucléique ou le vecteur d'acide nucléique selon la revendication 7.

9. Composition pharmaceutique comprenant la protéine de liaison à un antigène selon l'une quelconque des revendications 1 à 6, l'acide nucléique ou le vecteur d'acide nucléique selon la revendication 7, et un support, un diluant, un stabilisant et/ou un excipient pharmaceutiquement acceptable.

10. Composition pharmaceutique selon la revendication 9, dans laquelle la protéine de liaison à un antigène est dissoute ou dispersée dans un support pharmaceutiquement acceptable ou dans un milieu aqueux.

11. Procédé de production de la protéine de liaison à un antigène selon l'une quelconque des revendications 1 à 6, comprenant
a) la fourniture d'une cellule hôte appropriée,
b) la fourniture d'une construction génétique comprenant une séquence codante codant pour la protéine de liaison à un antigène selon l'une quelconque des revendications 1 à 6,
c) l'introduction de ladite construction génétique dans ladite cellule hôte appropriée, et
d) l'expression de ladite construction génétique par ladite cellule hôte appropriée.

12. Protéine de liaison à un antigène selon l'une quelconque des revendications 1 à 6, acide nucléique ou vecteur d'acide nucléique selon la revendication 7, ou composition pharmaceutique selon la revendication 9 ou 10, destinés à être utilisés en médecine.

13. Protéine de liaison à un antigène selon l'une quelconque des revendications 1 à 6, acide nucléique ou vecteur d'acide nucléique selon la revendication 7, ou composition pharmaceutique selon la revendication 9 ou 10, destinés à être utilisés dans le traitement d'une maladie proliférative.

14. Protéine de liaison à un antigène, acide nucléique, vecteur d'acide nucléique ou composition pharmaceutique destinés à être utilisés selon la revendication 13, dans lesquels la maladie proliférative est un cancer.
